# EUROPEAN PATENT SPECIFICATION

(11) **EP 1 836 223 B1**
(45) Date of publication and mention of the grant of the patent: **23.11.2011**
(21) Application number: 06700410.1
(22) Date of filing: 02.01.2006
(51) Int. Cl.: C07K 14/47, C12N 9/64, C12N 15/10, C12N 15/63, G01N 33/68

(54) **Method for identifying a nucleic acid encoding a hemopexin-like structure which specifically binds a predetermined target molecule.**
Methode zur Identifizierung einer Nukeinsäure kodierend für eine Hemopexin-ähnliche Struktur mit spezifischer Bindung zu einem vorgegebenen Molekul
Méthode d'identification un acide nucléique codant pour un support similaire de hemopexin liant spécifique à un molecule predeterminé.

(30) Priority: 03.01.2005 EP 05000013
(43) Date of publication of application: 26.09.2007
(60) Divisional application: 10176618.6
(73) Proprietor: F. Hoffmann-La Roche AG, 4070 Basel (CH)
(72) Inventor: LANZENDOERFER, Martin, 82327 Tutzing (DE); SCHRAEML, Michael, 82377 Penzberg (DE)
(74) Representative: Schreiner, Siegfried
(86) International application number: PCT/EP2006/000004
(87) International publication number: WO 2006/072563

(56) References cited:
- WO-A-95/11998
- WO-A-02/088171
- WO-A-03/014325
- WO-A-03/014325
- WO-A-2004/113521
- US-B1- 6 500 924
- CHA HYUNJU ET AL: "Structural basis of the adaptive molecular recognition by MMP9." JOURNAL OF MOLECULAR BIOLOGY. 26 JUL 2002, vol. 320, no. 5, 26 July 2002 (2002-07-26), pages 1065-1079, XP002374553 ISSN: 0022-2836
- NYGREN P-A ET AL: "Binding proteins from alternative scaffolds" JOURNAL OF IMMUNOLOGICAL METHODS, ELSEVIER SCIENCE PUBLISHERS B.V.,AMSTERDAM, NL, vol. 290, no. 1-2, 1 July 2004 (2004-07-01), pages 3-28, XP004521978 ISSN: 0022-1759
- BINZ H KASPAR ET AL: "High-affinity binders selected from designed ankyrin repeat protein libraries." NATURE BIOTECHNOLOGY. MAY 2004, vol. 22, no. 5, May 2004 (2004-05), pages 575-582, XP002343919 ISSN: 1087-0156
- CHA HYUNJU ET AL: "Structural basis of the adaptive molecular recognition by MMP9." JOURNAL OF MOLECULAR BIOLOGY. 26 JUL 2002, vol. 320, no. 5, 26 July 2002 (2002-07-26), pages 1065-1079, XP002374553 ISSN: 0022-2836
- FORRER PATRIK ET AL: "Consensus design of repeat proteins." CHEMBIOCHEM : A EUROPEAN JOURNAL OF CHEMICAL BIOLOGY. 6 FEB 2004, vol. 5, no. 2, 6 February 2004 (2004-02-06), pages 183-189, XP002382372 ISSN: 1439-4227 cited in the application
- BINZ H K ET AL: "Designing Repeat Proteins: Well-expressed, Soluble and Stable Proteins from Combinatorial Libraries of Consensus Ankyrin Repeat Proteins" JOURNAL OF MOLECULAR BIOLOGY, LONDON, GB, vol. 332, no. 2, 12 September 2003 (2003-09-12), pages 489-503, XP004450112 ISSN: 0022-2836 cited in the application
- FORRER P ET AL: "A novel strategy to design binding molecules harnessing the modular nature of repeat proteins" FEBS LETTERS, ELSEVIER, AMSTERDAM, NL, vol. 539, no. 1-3, 27 March 2003 (2003-03-27), pages 2-6, XP004415434 ISSN: 0014-5793
- STUMPP M T ET AL: "Designing Repeat Proteins: Modular Leucine-rich Repeat Protein Libraries Based on the Mammalian Ribonuclease Inhibitor Family" JOURNAL OF MOLECULAR BIOLOGY, LONDON, GB, vol. 332, no. 2, 12 September 2003 (2003-09-12), pages 471-487, XP004450111 ISSN: 0022-2836

### SEQUENCE LISTING

<110> F. Hoffmann-La Roche AG
<120> Hemopexin-like structure as new polypeptide-scaffold
<130> 22953
<150> EP 05000013.2
   <151> 2005-01-03
<160> 88
<170> PatentIn version 3.2
<210> 1
   <211> 195
   <212> PRT
   <213> Artificial
<220>
   <223> consensus sequence
<400> 1
   Pro Glu Ile Cys Lys Gln Asp Ile Val Phe Asp Gly Ile Ala Gln Ile
   1               5                   10                  15
   Arg Gly Glu Ile Phe Phe Phe Lys Asp Arg Phe Ile Trp Arg Thr Val
               20                  25                  30
   Thr Pro Arg Asp Lys Pro Met Gly Pro Leu Leu Val Ala Thr Phe Trp
           35                  40                  45
   Pro Glu Leu Pro Glu Lys Ile Asp Ala Val Tyr Glu Ala Pro Gln Glu
       50                  55                  60
   Glu Lys Ala Val Phe Phe Ala Gly Asn Glu Tyr Trp Ile Tyr Ser Ala
   65                  70                  75                  80
   Ser Thr Leu Glu Arg Gly Tyr Pro Lys Pro Leu Thr Ser Leu Gly Leu
                   85                  90                  95
   Pro Pro Asp Val Gln Arg Val Asp Ala Ala Phe Asn Trp Ser Lys Asn
               100                 105                 110
   Lys Lys Thr Tyr Ile Phe Ala Gly Asp Lys Phe Trp Arg Tyr Asn Glu
           115                 120                 125
   Val Lys Lys Lys Met Asp Pro Gly Phe Pro Lys Leu Ile Ala Asp Ala
       130                 135                 140
   Trp Asn Ala Ile Pro Asp Asn Leu Asp Ala Val Val Asp Leu Gln Gly
   145                 150                 155                 160
   Gly Gly His Ser Tyr Phe Phe Lys Gly Ala Tyr Tyr Leu Lys Leu Glu
                   165                 170                 175
   Asn Gln Ser Leu Lys Ser Val Lys Phe Gly Ser Ile Lys Ser Asp Trp
               180                 185                 190
   Leu Gly Cys
           195
<210> 2
   <211> 195
   <212> PRT
   <213> Gallus gallus
<400> 2
   Pro Glu Leu Cys Lys His Asp Ile Val Phe Asp Gly Val Ala Gln Ile
   1               5                   10                  15
   Arg Gly Glu Ile Phe Phe Phe Lys Asp Arg Phe Met Trp Arg Thr Val
               20                  25                  30
   Asn Pro Arg Gly Lys Pro Thr Gly Pro Leu Leu Val Ala Thr Phe Trp
           35                  40                  45
   Pro Asp Leu Pro Glu Lys Ile Asp Ala Val Tyr Glu Ser Pro Gln Asp
       50                  55                  60
   Glu Lys Ala Val Phe Phe Ala Gly Asn Glu Tyr Trp Val Tyr Thr Ala
   65                  70                  75                  80
   Ser Asn Leu Asp Arg Gly Tyr Pro Lys Lys Leu Thr Ser Leu Gly Leu
                   85                  90                  95
   Pro Pro Asp Val Gln Arg Ile Asp Ala Ala Phe Asn Trp Gly Arg Asn
               100                 105                 110
   Lys Lys Thr Tyr Ile Phe Ser Gly Asp Arg Tyr Trp Lys Tyr Asn Glu
           115                 120                 125
   Glu Lys Lys Lys Met Glu Leu Ala Thr Pro Lys Phe Ile Ala Asp Ser
       130                 135                 140
   Trp Asn Gly Val Pro Asp Asn Leu Asp Ala Val Leu Gly Leu Thr Asp
   145                 150                 155                 160
   Ser Gly Tyr Thr Tyr Phe Phe Lys Asp Gln Tyr Tyr Leu Gln Met Glu
                   165                 170                 175
   Asp Lys Ser Leu Lys Ile Val Lys Ile Gly Lys Ile Ser Ser Asp Trp
               180                 185                 190
   Leu Gly Cys
           195
<210> 3
   <211> 305
   <212> PRT
   <213> Mus musculus
<400> 3
   Met Ala Ala Arg Glu Glu Ser Thr Gln Ser Ala Asn Arg Val Leu Arg
   1               5                   10                  15
   Ile Ser Gln Leu Asp Ala Leu Glu Leu Asn Lys Ala Leu Glu Gln Leu
               20                  25                  30
   Val Trp Ser Gln Phe Thr Gln Cys Phe His Gly Phe Lys Pro Gly Leu
           35                  40                  45
   Leu Ala Arg Phe Glu Pro Glu Val Lys Ala Phe Leu Trp Leu Phe Leu
       50                  55                  60
   Trp Arg Phe Thr Ile Tyr Ser Lys Asn Ala Thr Val Gly Gln Ser Val
   65                  70                  75                  80
   Leu Asn Ile Gln His Lys Asn Asp Ser Ser Pro Asn Pro Val Tyr Gln
                   85                  90                  95
   Pro Pro Ser Lys Asn Gln Lys Leu Leu Tyr Ala Val Cys Thr Ile Gly
               100                 105                 110
   Gly Arg Trp Leu Glu Glu Arg Cys Tyr Asp Leu Phe Arg Asn Arg His
           115                 120                 125
   Leu Ala Ser Phe Gly Lys Ala Lys Gln Cys Met Asn Phe Val Val Gly
       130                 135                 140
   Leu Leu Lys Leu Gly Glu Leu Met Asn Phe Leu Ile Phe Leu Gln Lys
   145                 150                 155                 160
   Gly Lys Phe Ala Thr Leu Thr Glu Arg Leu Leu Gly Ile His Ser Val
                   165                 170                 175
   Phe Cys Lys Pro Gln Asn Met Arg Glu Val Gly Phe Glu Tyr Met Asn
               180                 185                 190
   Arg Glu Leu Leu Trp His Gly Phe Ala Glu Phe Leu Ile Phe Leu Leu
           195                 200                 205
   Pro Leu Ile Asn Ile Gln Lys Leu Lys Ala Lys Leu Ser Ser Trp Cys
       210                 215                 220
   Thr Leu Cys Thr Gly Ala Ala Gly His Asp Ser Thr Leu Gly Ser Ser
   225                 230                 235                 240
   Gly Lys Glu Cys Ala Leu Cys Gly Glu Trp Pro Thr Met Pro His Thr
                   245                 250                 255
   Ile Gly Cys Glu His Val Phe Cys Tyr Tyr Cys Val Lys Ser Ser Phe
               260                 265                 270
   Leu Phe Asp Ile Tyr Phe Thr Cys Pro Lys Cys Gly Thr Glu Val His
           275                 280                 285
   Ser Val Gln Pro Leu Lys Ala Gly Ile Gln Met Ser Glu Val Asn Ala
       290                 295                 300
   Leu
   305
<210> 4
   <211> 305
   <212> PRT
   <213> Rattus norvegicus
<400> 4
   Met Ala Ala Arg Glu Glu Ser Thr Gln Ser Ala Asn Arg Val Leu Arg
   1               5                   10                  15
   Ile Ser Gln Leu Asp Ala Leu Glu Leu Asn Lys Ala Leu Glu Gln Leu
               20                  25                  30
   Val Trp Ser Gln Phe Thr Gln Cys Phe His Gly Phe Lys Pro Gly Leu
           35                  40                  45
   Leu Ala Arg Phe Glu Pro Glu Val Lys Ala Phe Leu Trp Leu Phe Leu
       50                  55                  60
   Trp Arg Phe Thr Ile Tyr Ser Lys Asn Ala Thr Val Gly Gln Ser Val
   65                  70                  75                  80
   Leu Asn Ile Gln Tyr Lys Asn Asp Ser Ser Pro Asn Pro Val Tyr Gln
                   85                  90                  95
   Pro Pro Ser Lys Asn Gln Lys Leu Leu Tyr Ala Val Cys Thr Ile Gly
               100                 105                 110
   Gly Arg Trp Leu Glu Glu Arg Cys Tyr Asp Leu Phe Arg Asn Arg His
           115                 120                 125
   Leu Ala Ser Phe Gly Lys Ala Lys Gln Cys Met Asn Phe Val Val Gly
       130                 135                 140
   Leu Leu Lys Leu Gly Glu Leu Met Asn Phe Leu Ile Phe Leu Gln Lys
   145                 150                 155                 160
   Gly Lys Phe Ala Thr Leu Thr Glu Arg Leu Leu Gly Ile His Ser Val
                   165                 170                 175
   Phe Cys Lys Pro Gln Ser Met Arg Glu Val Gly Phe Glu Tyr Met Asn
               180                 185                 190
   Arg Glu Leu Leu Trp His Gly Phe Ala Glu Phe Leu Val Phe Leu Leu
           195                 200                 205
   Pro Leu Ile Asn Ile Gln Lys Leu Lys Ala Lys Leu Ser Ser Trp Cys
       210                 215                 220
   Ile Pro Leu Thr Ser Thr Ala Gly Ser Asp Ser Thr Leu Gly Ser Ser
   225                 230                 235                 240
   Gly Lys Glu Cys Ala Leu Cys Gly Glu Trp Pro Thr Met Pro His Thr
                   245                 250                 255
   Ile Gly Cys Glu His Val Phe Cys Tyr Tyr Cys Val Lys Ser Ser Phe
               260                 265                 270
   Leu Phe Asp Met Tyr Phe Thr Cys Pro Lys Cys Gly Thr Glu Val His
           275                 280                 285
   Ser Val Gln Pro Leu Lys Ser Gly Ile Glu Met Ser Glu Val Asn Ala
       290                 295                 300
   Leu
   305
<210> 5
   <211> 195
   <212> PRT
   <213> Oryctolagus cuniculus
<400> 5
   Pro Glu Ile Cys Thr Gln Asp Ile Val Phe Asp Gly Ile Ala Gln Ile
   1               5                   10                  15
   Arg Gly Glu Ile Phe Phe Phe Lys Asp Arg Phe Ile Trp Arg Thr Val
               20                  25                  30
   Thr Pro Gly Asp Lys Pro Met Gly Pro Leu Leu Val Ala Thr Phe Trp
           35                  40                  45
   Pro Glu Leu Pro Glu Lys Ile Asp Ala Val Tyr Glu Ala Pro Gln Glu
       50                  55                  60
   Glu Lys Ala Val Phe Phe Ala Gly Asn Glu Tyr Trp Val Tyr Ser Ala
   65                  70                  75                  80
   Ser Thr Leu Glu Arg Gly Tyr Pro Lys Pro Leu Thr Ser Leu Gly Leu
                   85                  90                  95
   Pro Pro Asp Val Gln Arg Val Asp Ala Ala Phe Asn Trp Ser Lys Asn
               100                 105                 110
   Lys Lys Thr Tyr Ile Phe Ala Gly Asp Lys Phe Trp Arg Tyr Asn Glu
               115                 120                 125
   Val Lys Lys Lys Met Asp Pro Gly Phe Pro Arg Leu Ile Ala Asp Ala
       130                 135                 140
   Trp Asn Ala Ile Pro Asp His Leu Asp Ala Val Val Asp Leu Gln Gly
   145                 150                 155                 160
   Ser Gly His Ser Tyr Phe Phe Lys Gly Thr Tyr Tyr Leu Lys Leu Glu
                   165                 170                 175
   Asn Gln Ser Leu Lys Ser Val Lys Val Gly Ser Ile Lys Thr Asp Trp
               180                 185                 190
   Leu Gly Cys
           195
<210> 6
   <211> 195
   <212> PRT
   <213> Bos taurus
<400> 6
   Pro Glu Val Cys Asp Ser Lys Leu Thr Phe Asp Ala Ile Thr Thr Ile
   1               5                   10                  15
   Arg Gly Glu Val Met Phe Phe Lys Asp Arg Phe Tyr Met Arg Thr Asn
               20                  25                  30
   Pro Leu Tyr Pro Glu Val Glu Leu Asn Phe Ile Ser Val Phe Trp Pro
           35                  40                  45
   Gln Leu Pro Asn Gly Leu Gln Ala Ala Tyr Glu Val Ala Asp Arg Asp
       50                  55                  60
   Glu Val Arg Phe Phe Lys Gly Asn Lys Tyr Trp Ala Val Lys Gly Gln
   65                  70                  75                  80
   Asp Val Leu Arg Gly Tyr Pro Arg Asp Ile Tyr Arg Ser Phe Gly Phe
                   85                  90                  95
   Pro Arg Thr Val Lys Ser Ile Asp Ala Ala Val Ser Glu Glu Asp Thr
               100                 105                 110
   Gly Lys Thr Tyr Phe Phe Val Ala Asn Lys Cys Trp Arg Tyr Asp Glu
   115 120 125
   Tyr Lys Gln Ser Met Asp Ala Gly Tyr Pro Lys Met Ile Ala Glu Asp
   130 135 140
   Phe Pro Gly Ile Gly Asn Lys Val Asp Ala Val Phe Gln Lys Gly Gly
   145 150 155 160
   Phe Phe Tyr Phe Phe His Gly Arg Arg Gln Tyr Lys Phe Asp Pro Gln
   165 170 175
   Thr Lys Arg Ile Leu Thr Leu Leu Lys Ala Asn Ser Trp Phe Asn Cys
   180 185 190
   Arg Lys Asn
   195
<210> 7
   <211> 195
   <212> PRT
   <213> Equus caballus
<400> 7
   Pro Glu Val Cys Asp Ser Lys Leu Thr Phe Asp Ala Ile Thr Thr Ile
   1 5 10 15
   Arg Gly Glu Val Met Phe Phe Lys Asp Arg Phe Tyr Met Arg Ile Asn
   20 25 30
   Pro Tyr Tyr Pro Glu Ala Glu Leu Asn Phe Ile Ser Ile Phe Trp Pro
   35 40 45
   Gln Leu Pro Asn Gly Leu Gln Ala Ala Tyr Glu Val Ser His Arg Asp
   50 55 60
   Glu Val Arg Phe Phe Lys Gly Asn Lys Tyr Trp Ala Val Lys Gly Gln
   65 70 75 80
   Asp Val Leu Tyr Gly Tyr Pro Lys Asp Ile His Arg Ser Phe Gly Phe
   85 90 95
   Pro Ser Thr Val Lys Asn Ile Asp Ala Ala Val Ser Glu Glu Asp Thr
   100 105 110
   Gly Lys Thr Tyr Phe Phe Val Ala Asp Lys Tyr Trp Arg Tyr Asp Glu
           115                     120                     125
   Tyr Lys Arg Ser Met Asp Ala Gly Tyr Pro Lys Met Ile Ala Asp Asp
       130                     135                     140
   Phe Pro Gly Ile Gly Asp Lys Val Asp Ala Val Phe Gln Lys Asp Gly
   145                 150                     155                 160
   Phe Phe Tyr Phe Phe His Gly Thr Arg Gln Tyr Lys Phe Asp Pro Lys
                   165                     170                     175
   Thr Lys Arg Ile Leu Thr Leu Gln Lys Ala Asn Ser Trp Phe Asn Cys
               180                     185                     190
   Arg Lys Asn
           195
<210> 8
   <211> 195
   <212> PRT
   <213> Homo sapiens
<400> 8
   Pro Lys Ala Cys Asp Ser Lys Leu Thr Phe Asp Ala Ile Thr Thr Ile
   1               5                   10                  15
   Arg Gly Glu Val Met Phe Phe Lys Asp Arg Phe Tyr Met Arg Thr Asn
               20                  25                  30
   Pro Phe Tyr Pro Glu Val Glu Leu Asn Phe Ile Ser Val Phe Trp Pro
           35                  40                  45
   Gln Leu Pro Asn Gly Leu Glu Ala Ala Tyr Glu Phe Ala Asp Arg Asp
       50                  55                  60
   Glu Val Arg Phe Phe Lys Gly Asn Lys Tyr Trp Ala Val Gln Gly Gln
   65                  70                  75                  80
   Asn Val Leu His Gly Tyr Pro Lys Asp Ile Tyr Ser Ser Phe Gly Phe
                   85                  90                  95
   Pro Arg Thr Val Lys His Ile Asp Ala Ala Leu Ser Glu Glu Asn Thr
               100                 105                 110
   Gly Lys Thr Tyr Phe Phe Val Ala Asn Lys Tyr Trp Arg Tyr Asp Glu
           115                 120                 125
   Tyr Lys Arg Ser Met Asp Pro Gly Tyr Pro Lys Met Ile Ala His Asp
       130                 135                 140
   Phe Pro Gly Ile Gly His Lys Val Asp Ala Val Phe Met Lys Asp Gly
   145                 150                 155                 160
   Phe Phe Tyr Phe Phe His Gly Thr Arg Gln Tyr Lys Phe Asp Pro Lys
                   165                 170                 175
   Thr Lys Arg Ile Leu Thr Leu Gln Lys Ala Asn Ser Trp Phe Asn Cys
               180                 185                 190
   Arg Lys Asn
           195
<210> 9
   <211> 195
   <212> PRT
   <213> Sus scrofa
<400> 9
   Pro Gln Val Cys Asp Ser Lys Leu Thr Phe Asp Ala Ile Thr Thr Leu
   1               5                   10                  15
   Arg Gly Glu Leu Met Phe Phe Lys Asp Arg Phe Tyr Met Arg Thr Asn
               20                  25                  30
   Ser Phe Tyr Pro Glu Val Glu Leu Asn Phe Ile Ser Val Phe Trp Pro
           35                  40                  45
   Gln Val Pro Asn Gly Leu Gln Ala Ala Tyr Glu Ile Ala Asp Arg Asp
       50                  55                  60
   Glu Val Arg Phe Phe Lys Gly Asn Lys Tyr Trp Ala Val Arg Gly Gln
   65                  70                  75                  80
   Asp Val Leu Tyr Gly Tyr Pro Lys Asp Ile His Arg Ser Phe Gly Phe
                   85                  90                  95
   Pro Ser Thr Val Lys Asn Ile Asp Ala Ala Val Phe Glu Glu Asp Thr
   100 105 110
   Gly Lys Thr Tyr Phe Phe Val Ala His Glu Cys Trp Arg Tyr Asp Glu
   115 120 125
   Tyr Lys Gln Ser Met Asp Thr Gly Tyr Pro Lys Met Ile Ala Glu Glu
   130 135 140
   Phe Pro Gly Ile Gly Asn Lys Val Asp Ala Val Phe Gln Lys Asp Gly
   145 150 155 160
   Phe Leu Tyr Phe Phe His Gly Thr Arg Gln Tyr Gln Phe Asp Phe Lys
   165 170 175
   Thr Lys Arg Ile Leu Thr Leu Gln Lys Ala Asn Ser Trp Phe Asn Cys
   180 185 190
   Arg Lys Asn
   195
<210> 10
   <211> 195
   <212> PRT
   <213> Homo sapiens
<400> 10
   Pro Glu Ile Cys Lys Gln Asp Ile Val Phe Asp Gly Ile Ala Gln Ile
   1 5 10 15
   Arg Gly Glu Ile Phe Phe Phe Lys Asp Arg Phe Ile Trp Arg Thr Val
   20 25 30
   Thr Pro Arg Asp Lys Pro Met Gly Pro Leu Leu Val Ala Thr Phe Trp
   35 40 45
   Pro Glu Leu Pro Glu Lys Ile Asp Ala Val Tyr Glu Ala Pro Gln Glu
   50 55 60
   Glu Lys Ala Val Phe Phe Ala Gly Asn Glu Tyr Trp Ile Tyr Ser Ala
   65 70 75 80
   Ser Thr Leu Glu Arg Gly Tyr Pro Lys Pro Leu Thr Ser Leu Gly Leu
   85 90 95
   Pro Pro Asp Val Gln Arg Val Asp Ala Ala Phe Asn Trp Ser Lys Asn
   100 105 110
   Lys Lys Thr Tyr Ile Phe Ala Gly Asp Lys Phe Trp Arg Tyr Asn Glu
   115 120 125
   Val Lys Lys Lys Met Asp Pro Gly Phe Pro Lys Leu Ile Ala Asp Ala
   130 135 140
   Trp Asn Ala Ile Pro Asp Asn Leu Asp Ala Val Val Asp Leu Gln Gly
   145 150 155 160
   Gly Gly His Ser Tyr Phe Phe Lys Gly Ala Tyr Tyr Leu Lys Leu Glu
   165 170 175
   Asn Gln Ser Leu Lys Ser Val Lys Phe Gly Ser Ile Lys Ser Asp Trp
   180 185 190
   Leu Gly Cys
   195
<210> 11
   <211> 191
   <212> PRT
   <213> Homo sapiens
<400> 11
   Pro Ala Asn Cys Asp Pro Ala Leu Ser Phe Asp Ala Val Ser Thr Leu
   1 5 10 15
   Arg Gly Glu Ile Leu Ile Phe Lys Asp Arg His Phe Trp Arg Lys Ser
   20 25 30
   Leu Arg Lys Leu Glu Pro Glu Leu His Leu Ile Ser Ser Phe Trp Pro
   35 40 45
   Ser Leu Pro Ser Gly Val Asp Ala Ala Tyr Glu Val Thr Ser Lys Asp
   50 55 60
   Leu Val Phe Ile Phe Lys Gly Asn Gln Phe Trp Ala Ile Arg Gly Asn
   65 70 75 80
   Glu Val Arg Ala Gly Tyr Pro Arg Gly Ile His Thr Leu Gly Phe Pro
   85 90 95
   Pro Thr Val Arg Lys Ile Asp Ala Ala Ile Ser Asp Lys Glu Lys Asn
   100 105 110
   Lys Thr Tyr Phe Phe Val Glu Asp Lys Tyr Trp Arg Phe Asp Glu Lys
   115 120 125
   Arg Asn Ser Met Glu Pro Gly Phe Pro Lys Gln Ile Ala Glu Asp Phe
   130 135 140
   Pro Gly Ile Asp Ser Lys Ile Asp Ala Val Phe Glu Glu Phe Gly Phe
   145 150 155 160
   Phe Tyr Phe Phe Thr Gly Ser Ser Gln Leu Glu Phe Asp Pro Asn Ala
   165 170 175
   Lys Lys Val Thr His Thr Leu Lys Ser Asn Ser Trp Leu Asn Cys
   180 185 190
<210> 12
   <211> 191
   <212> PRT
   <213> Mus musculus
<400> 12
   Ser Pro Met Cys Ser Ser Thr Leu Phe Phe Asp Ala Val Ser Thr Leu
   1 5 10 15
   Arg Gly Glu Val Leu Phe Phe Lys Asp Arg His Phe Trp Arg Lys Ser
   20 25 30
   Leu Arg Thr Pro Glu Pro Glu Phe Tyr Leu Ile Ser Ser Phe Trp Pro
   35 40 45
   Ser Leu Pro Ser Asn Met Asp Ala Ala Tyr Glu Val Thr Asn Arg Asp
   50 55 60
   Thr Val Phe Ile Phe Lys Gly Asn Gln Phe Trp Ala Ile Arg Gly His
   65 70 75 80
   Glu Glu Leu Ala Gly Tyr Pro Lys Ser Ile His Thr Leu Gly Leu Pro
   85 90 95
   Ala Thr Val Lys Lys Ile Asp Ala Ala Ile Ser Asn Lys Glu Lys Arg
   100 105 110
   Lys Thr Tyr Phe Phe Val Glu Asp Lys Tyr Trp Arg Phe Asp Glu Lys
   115 120 125
   Lys Gln Ser Met Glu Pro Gly Phe Pro Arg Lys Ile Ala Glu Asp Phe
   130 135 140
   Pro Gly Val Asp Ser Arg Val Asp Ala Val Phe Glu Ala Phe Gly Phe
   145 150 155 160
   Leu Tyr Phe Phe Ser Gly Ser Ser Gln Leu Glu Phe Asp Pro Asn Ala
   165 170 175
   Lys Lys Val Thr His Ile Leu Lys Ser Asn Ser Trp Phe Asn Cys
   180 185 190
<210> 13
   <211> 191
   <212> PRT
   <213> Oryctolagus cuniculus
<400> 13
   Pro Val Met Cys Asp Pro Asp Leu Ser Phe Asp Ala Ile Ser Thr Leu
   1 5 10 15
   Arg Gly Glu Ile Leu Phe Phe Lys Asp Arg Tyr Phe Trp Arg Lys Ser
   20 25 30
   Leu Arg Ile Leu Glu Pro Glu Phe His Leu Ile Ser Ser Phe Trp Pro
   35 40 45
   Ser Leu Pro Ser Ala Val Asp Ala Ala Tyr Glu Val Ile Ser Arg Asp
   50 55 60
   Thr Val Phe Ile Phe Lys Gly Thr Gln Phe Trp Ala Ile Arg Gly Asn
   65 70 75 80
   Glu Val Gln Ala Gly Tyr Pro Arg Ser Ile His Thr Leu Gly Phe Pro
   85 90 95
   Ser Thr Ile Arg Lys Ile Asp Ala Ala Ile Ser Asp Lys Glu Arg Lys
   100 105 110
   Lys Thr Tyr Phe Phe Val Glu Asp Lys Tyr Trp Arg Phe Asp Glu Lys
   115 120 125
   Arg Gln Ser Leu Glu Pro Gly Phe Pro Arg His Ile Ala Glu Asp Phe
   130 135 140
   Pro Gly Ile Asn Pro Lys Ile Asp Ala Val Phe Glu Ala Phe Gly Phe
   145 150 155 160
   Phe Tyr Phe Phe Ser Gly Ser Ser Gln Ser Glu Phe Asp Pro Asn Ala
   165 170 175
   Lys Lys Val Thr His Val Leu Lys Ser Asn Ser Trp Phe Gln Cys
   180 185 190
<210> 14
   <211> 191
   <212> PRT
   <213> Rattus norvegicus
<400> 14
   Leu Pro Met Cys Ser Ser Ala Leu Ser Phe Asp Ala Val Ser Thr Leu
   1 5 10 15
   Arg Gly Glu Val Leu Phe Phe Lys Asp Arg His Phe Trp Arg Lys Ser
   20 25 30
   Leu Arg Thr Pro Glu Pro Gly Phe Tyr Leu Ile Ser Ser Phe Trp Pro
   35 40 45
   Ser Leu Pro Ser Asn Met Asp Ala Ala Tyr Glu Val Thr Asn Arg Asp
   50 55 60
   Thr Val Phe Ile Leu Lys Gly Asn Gln Ile Trp Ala Ile Arg Gly His
   65 70 75 80
   Glu Glu Leu Ala Gly Tyr Pro Lys Ser Ile His Thr Leu Gly Leu Pro
   85 90 95
   Glu Thr Val Gln Lys Ile Asp Ala Ala Ile Ser Leu Lys Asp Gln Lys
   100 105 110
   Lys Thr Tyr Phe Phe Val Glu Asp Lys Phe Trp Arg Phe Asp Glu Lys
   115 120 125
   Lys Gln Ser Met Asp Pro Glu Phe Pro Arg Lys Ile Ala Glu Asn Phe
       130                 135                 140
   Pro Gly Ile Gly Thr Lys Val Asp Ala Val Phe Glu Ala Phe Gly Phe
   145                 150                 155                 160
   Leu Tyr Phe Phe Ser Gly Ser Ser Gln Leu Glu Phe Asp Pro Asn Ala
                   165                 170                 175
   Gly Lys Val Thr His Ile Leu Lys Ser Asn Ser Trp Phe Asn Cys
               180                 185                 190
<210> 15
   <211> 192
   <212> PRT
   <213> Homo sapiens
<400> 15
   Pro Lys Pro Cys Asp Pro Ser Leu Thr Phe Asp Ala Ile Thr Thr Leu
   1               5                   10                  15
   Arg Gly Glu Ile Leu Phe Phe Lys Asp Arg Tyr Phe Trp Arg Arg His
               20                  25                  30
   Pro Gln Leu Gln Arg Val Glu Met Asn Phe Ile Ser Leu Phe Trp Pro
           35                  40                  45
   Ser Leu Pro Thr Gly Ile Gln Ala Ala Tyr Glu Asp Phe Asp Arg Asp
       50                  55                  60
   Leu Ile Phe Leu Phe Lys Gly Asn Gln Tyr Trp Ala Leu Ser Gly Tyr
   65                  70                  75                  80
   Asp Ile Leu Gln Gly Tyr Pro Lys Asp Ile Ser Asn Tyr Gly Phe Pro
                   85                  90                  95
   Ser Ser Val Gln Ala Ile Asp Ala Ala Val Phe Tyr Arg Ser Lys Thr
               100                 105                 110
   Tyr Phe Phe Val Asn Asp Gln Phe Trp Arg Tyr Asp Asn Gln Arg Gln
           115                 120                 125
   Phe Met Glu Pro Gly Tyr Pro Lys Ser Ile Ser Gly Ala Phe Pro Gly
       130                 135                 140
   Ile Glu Ser Lys Val Asp Ala Val Phe Gln Gln Glu His Phe Phe His
   145                 150                 155                 160
   Val Phe Ser Gly Pro Arg Tyr Tyr Ala Phe Asp Leu Ile Ala Gln Arg
                   165                 170                 175
   Val Thr Arg Val Ala Arg Gly Asn Lys Trp Leu Asn Cys Arg Tyr Gly
               180                 185                 190
<210> 16
   <211> 190
   <212> PRT
   <213> Mus musculus
<400> 16
   Pro Lys Ala Cys Asp Pro His Leu Arg Phe Asp Ala Thr Thr Thr Leu
   1               5                   10                  15
   Arg Gly Glu Ile Tyr Phe Phe Lys Glu Lys Tyr Phe Trp Arg Arg His
               20                  25                  30
   Pro Gln Leu Arg Thr Val Asp Leu Asn Phe Ile Ser Leu Phe Trp Pro
           35                  40                  45
   Gly Leu Pro Asn Gly Leu Gln Ala Ala Tyr Glu Asp Phe Asp Arg Asp
       50                  55                  60
   Leu Val Phe Leu Phe Lys Gly Arg Gln Tyr Trp Ala Leu Ser Gly Tyr
   65                  70                  75                  80
   Asp Leu Gln Gln Gly Tyr Pro Arg Asp Ile Ser Asn Tyr Gly Phe Pro
                   85                  90                  95
   Arg Ser Val Gln Ala Ile Asp Ala Ala Val Ser Tyr Asn Gly Lys Thr
               100                 105                 110
   Tyr Phe Phe Ile Asn Asn Gln Cys Trp Arg Tyr Asp Asn Glu Arg Arg
           115                 120                 125
   Ser Met Asp Pro Gly Tyr Pro Lys Ser Ile Pro Ser Met Phe Pro Gly
       130                 135                 140
   Val Asn Cys Arg Val Asp Ala Val Phe Leu Gln Asp Ser Phe Phe Leu
   145                 150                 155                 160
   Phe Phe Ser Gly Pro Gln Tyr Phe Ala Phe Asn Phe Val Ser His Arg
   165 170 175
   Val Thr Arg Val Ala Arg Ser Asn Leu Trp Leu Asn Cys Ser
   180 185 190
<210> 17
   <211> 190
   <212> PRT
   <213> Rattus norvegicus
<400> 17
   Pro Thr Ala Cys Asp Pro His Leu Arg Phe Asp Ala Ala Thr Thr Leu
   1 5 10 15
   Arg Gly Glu Ile Tyr Phe Phe Lys Asp Lys Tyr Phe Trp Arg Arg His
   20 25 30
   Pro Gln Leu Arg Thr Val Asp Leu Asn Phe Ile Ser Leu Phe Trp Pro
   35 40 45
   Phe Leu Pro Asn Gly Leu Gln Ala Ala Tyr Glu Asp Phe Asp Arg Asp
   50 55 60
   Leu Val Phe Leu Phe Lys Gly Arg Gln Tyr Trp Ala Leu Ser Ala Tyr
   65 70 75 80
   Asp Leu Gln Gln Gly Tyr Pro Arg Asp Ile Ser Asn Tyr Gly Phe Pro
   85 90 95
   Arg Ser Val Gln Ala Ile Asp Ala Ala Val Ser Tyr Asn Gly Lys Thr
   100 105 110
   Tyr Phe Phe Val Asn Asn Gln Cys Trp Arg Tyr Asp Asn Gln Arg Arg
   115 120 125
   Ser Met Asp Pro Gly Tyr Pro Thr Ser Ile Ala Ser Val Phe Pro Gly
   130 135 140
   Ile Asn Cys Arg Ile Asp Ala Val Phe Gln Gln Asp Ser Phe Phe Leu
   145 150 155 160
   Phe Phe Ser Gly Pro Gln Tyr Phe Ala Phe Asn Leu Val Ser Arg Arg
   165 170 175
   Val Thr Arg Val Ala Arg Ser Asn Leu Trp Leu Asn Cys Pro
   180 185 190
<210> 18
   <211> 195
   <212> PRT
   <213> Bos taurus
<400> 18
   Glu Asp Val Cys Asn Val Asp Ile Phe Asp Ala Ile Ala Glu Ile Arg
   1 5 10 15
   Asn Arg Leu His Phe Phe Lys Ala Gly Lys Tyr Trp Arg Leu Ser Glu
   20 25 30
   Gly Gly Gly Arg Arg Val Gln Gly Pro Phe Leu Val Lys Ser Lys Trp
   35 40 45
   Pro Ala Leu Pro Arg Lys Leu Asp Ser Ala Phe Glu Asp Pro Leu Thr
   50 55 60
   Lys Lys Ile Phe Phe Phe Ser Gly Arg Gln Val Trp Val Tyr Thr Gly
   65 70 75 80
   Ala Ser Leu Leu Gly Pro Arg Arg Leu Asp Lys Leu Gly Leu Gly Pro
   85 90 95
   Glu Val Ala Gln Val Thr Gly Ala Leu Pro Arg Pro Glu Gly Lys Val
   100 105 110
   Leu Leu Phe Ser Gly Gln Ser Phe Trp Arg Phe Asp Val Lys Thr Gln
   115 120 125
   Lys Val Asp Pro Gln Ser Val Thr Pro Val Asp Gln Met Phe Pro Gly
   130 135 140
   Val Pro Ile Ser Thr His Asp Ile Phe Gln Tyr Gln Glu Lys Ala Tyr
   145 150 155 160
   Phe Cys Gln Asp His Phe Tyr Trp Arg Val Ser Ser Gln Asn Glu Val
   165 170 175
   Asn Gln Val Asp Tyr Val Gly Tyr Val Thr Phe Asp Leu Leu Lys Cys
               180                 185                 190
   Pro Glu Asp
           195
<210> 19
   <211> 195
   <212> PRT
   <213> Canis familiaris
<400> 19
   Glu Asp Ile Cys Lys Val Asn Ile Phe Asp Ala Ile Ala Glu Ile Arg
   1               5                   10                  15
   Asn Tyr Leu His Phe Phe Lys Glu Gly Lys Tyr Trp Arg Phe Ser Lys
               20                  25                  30
   Gly Lys Gly Arg Arg Val Gln Gly Pro Phe Leu Ser Pro Ser Thr Trp
           35                  40                  45
   Pro Ala Leu Pro Arg Lys Leu Asp Ser Ala Phe Glu Asp Gly Leu Thr
       50                  55                  60
   Lys Lys Thr Phe Phe Phe Ser Gly Arg Gln Val Trp Val Tyr Thr Gly
   65                  70                  75                  80
   Thr Ser Val Val Gly Pro Arg Arg Leu Asp Lys Leu Gly Leu Gly Pro
                   85                  90                  95
   Glu Val Thr Gln Val Thr Gly Ala Leu Pro Gln Gly Gly Gly Lys Val
               100                 105                 110
   Leu Leu Phe Ser Arg Gln Arg Phe Trp Ser Phe Asp Val Lys Thr Gln
           115                 120                 125
   Thr Val Asp Pro Arg Ser Ala Gly Ser Val Glu Gln Met Tyr Pro Gly
       130                 135                 140
   Val Pro Leu Asn Thr His Asp Ile Phe Gln Tyr Gln Glu Lys Ala Tyr
   145                 150                 155                 160
   Phe Cys Gln Asp Arg Phe Tyr Trp Arg Val Asn Ser Arg Asn Glu Val
                   165                 170                 175
   Asn Gln Val Asp Glu Val Gly Tyr Val Thr Phe Asp Ile Leu Gln Cys
   180 185 190
   Pro Glu Asp
   195
<210> 20
   <211> 195
   <212> PRT
   <213> Homo sapiens
<400> 20
   Asp Asp Ala Cys Asn Val Asn Ile Phe Asp Ala Ile Ala Glu Ile Gly
   1 5 10 15
   Asn Gln Leu Tyr Leu Phe Lys Asp Gly Lys Tyr Trp Arg Phe Ser Glu
   20 25 30
   Gly Arg Gly Ser Arg Pro Gln Gly Pro Phe Leu Ile Ala Asp Lys Trp
   35 40 45
   Pro Ala Leu Pro Arg Lys Leu Asp Ser Val Phe Glu Glu Pro Leu Ser
   50 55 60
   Lys Lys Leu Phe Phe Phe Ser Gly Arg Gln Val Trp Val Tyr Thr Gly
   65 70 75 80
   Ala Ser Val Leu Gly Pro Arg Arg Leu Asp Lys Leu Gly Leu Gly Ala
   85 90 95
   Asp Val Ala Gln Val Thr Gly Ala Leu Arg Ser Gly Arg Gly Lys Met
   100 105 110
   Leu Leu Phe Ser Gly Arg Arg Leu Trp Arg Phe Asp Val Lys Ala Gln
   115 120 125
   Met Val Asp Pro Arg Ser Ala Ser Glu Val Asp Arg Met Phe Pro Gly
   130 135 140
   Val Pro Leu Asp Thr His Asp Val Phe Gln Tyr Arg Glu Lys Ala Tyr
   145 150 155 160
   Phe Cys Gln Asp Arg Phe Tyr Trp Arg Val Ser Ser Arg Ser Glu Leu
   165 170 175
   Asn Gln Val Asp Gln Val Gly Tyr Val Thr Tyr Asp Ile Leu Gln Cys
   180 185 190
   Pro Glu Asp
   195
<210> 21
   <211> 200
   <212> PRT
   <213> Mus musculus
<400> 21
   Asp Asn Pro Cys Asn Val Asp Val Phe Asp Ala Ile Ala Glu Ile Gln
   1 5 10 15
   Gly Ala Leu His Phe Phe Lys Asp Gly Trp Tyr Trp Lys Phe Leu Asn
   20 25 30
   His Arg Gly Ser Pro Leu Gln Gly Pro Phe Leu Thr Ala Arg Thr Trp
   35 40 45
   Pro Ala Leu Pro Ala Thr Leu Asp Ser Ala Phe Glu Asp Pro Gln Thr
   50 55 60
   Lys Arg Val Phe Phe Phe Ser Gly Arg Gln Met Trp Val Tyr Thr Gly
   65 70 75 80
   Lys Thr Val Leu Gly Pro Arg Ser Leu Asp Lys Leu Gly Leu Gly Pro
   85 90 95
   Glu Val Thr His Val Ser Gly Leu Leu Pro Arg Arg Pro Gly Lys Ala
   100 105 110
   Leu Leu Phe Ser Lys Gly Arg Val Trp Arg Phe Asp Leu Lys Ser Gln
   115 120 125
   Lys Val Asp Pro Gln Ser Val Ile Arg Val Asp Lys Glu Phe Ser Gly
   130 135 140
   Val Pro Trp Asn Ser His Asp Ile Phe Gln Tyr Gln Asp Lys Ala Tyr
   145 150 155 160
   Phe Cys His Gly Lys Phe Phe Trp Arg Val Ser Phe Gln Asn Glu Val
   165 170 175
   Asn Lys Val Asp Pro Glu Val Asn Gln Val Asp Asp Val Gly Tyr Val
   180 185 190
   Thr Tyr Asp Leu Leu Gln Cys Pro
   195 200
<210> 22
   <211> 191
   <212> PRT
   <213> Homo sapiens
<400> 22
   Pro Ala Lys Cys Asp Pro Ala Leu Ser Phe Asp Ala Ile Ser Thr Leu
   1 5 10 15
   Arg Gly Glu Tyr Leu Phe Phe Lys Asp Arg Tyr Phe Trp Arg Arg Ser
   20 25 30
   His Trp Asn Pro Glu Pro Glu Phe His Leu Ile Ser Ala Phe Trp Pro
   35 40 45
   Ser Leu Pro Ser Tyr Leu Asp Ala Ala Tyr Glu Val Asn Ser Arg Asp
   50 55 60
   Thr Val Phe Ile Phe Lys Gly Asn Glu Phe Trp Ala Ile Arg Gly Asn
   65 70 75 80
   Glu Val Gln Ala Gly Tyr Pro Arg Gly Ile His Thr Leu Gly Phe Pro
   85 90 95
   Pro Thr Ile Arg Lys Ile Asp Ala Ala Val Ser Asp Lys Glu Lys Lys
   100 105 110
   Lys Thr Tyr Phe Phe Ala Ala Asp Lys Tyr Trp Arg Phe Asp Glu Asn
   115 120 125
   Ser Gln Ser Met Glu Gln Gly Phe Pro Arg Leu Ile Ala Asp Asp Phe
   130 135 140
   Pro Gly Val Glu Pro Lys Val Asp Ala Val Leu Gln Ala Phe Gly Phe
   145 150 155 160
   Phe Tyr Phe Phe Ser Gly Ser Ser Gln Phe Glu Phe Asp Pro Asn Ala
                   165                 170                 175
   Arg Met Val Thr His Ile Leu Lys Ser Asn Ser Trp Leu His Cys
               180                 185                 190
<210> 23
   <211> 191
   <212> PRT
   <213> Mus musculus
<400> 23
   Pro Asp Lys Cys Asp Pro Ala Leu Ser Phe Asp Ser Val Ser Thr Leu
   1               5                   10                  15
   Arg Gly Glu Val Leu Phe Phe Lys Asp Arg Tyr Phe Trp Arg Arg Ser
               20                  25                  30
   His Trp Asn Pro Glu Pro Glu Phe His Leu Ile Ser Ala Phe Trp Pro
           35                  40                  45
   Thr Leu Pro Ser Asp Leu Asp Ala Ala Tyr Glu Ala His Asn Thr Asp
       50                  55                  60
   Ser Val Leu Ile Phe Lys Gly Ser Gln Phe Trp Ala Val Arg Gly Asn
   65                  70                  75                  80
   Glu Val Gln Ala Gly Tyr Pro Lys Gly Ile His Thr Leu Gly Phe Pro
                   85                  90                  95
   Pro Thr Val Lys Lys Ile Asp Ala Ala Val Phe Glu Lys Glu Lys Lys
               100                 105                 110
   Lys Thr Tyr Phe Phe Val Gly Asp Lys Tyr Trp Arg Phe Asp Glu Thr
           115                 120                 125
   Arg His Val Met Asp Lys Gly Phe Pro Arg Gln Ile Thr Asp Asp Phe
       130                 135                 140
   Pro Gly Ile Glu Pro Gln Val Asp Ala Val Leu His Glu Phe Gly Phe
   145                 150                 155                 160
   Phe Tyr Phe Phe Arg Gly Ser Ser Gln Phe Glu Phe Asp Pro Asn Ala
                   165                 170                 175
   Arg Thr Val Thr His Ile Leu Lys Ser Asn Ser Trp Leu Leu Cys
   180 185 190
<210> 24
   <211> 198
   <212> PRT
   <213> Homo sapiens
<400> 24
   Pro Asp Ala Cys Glu Ala Ser Phe Asp Ala Val Ser Thr Ile Arg Gly
   1 5 10 15
   Glu Leu Phe Phe Phe Lys Ala Gly Phe Val Trp Arg Leu Arg Gly Gly
   20 25 30
   Gln Leu Gln Pro Gly Tyr Pro Ala Leu Ala Ser Arg His Trp Gln Gly
   35 40 45
   Leu Pro Ser Pro Val Asp Ala Ala Phe Glu Asp Ala Gln Gly His Ile
   50 55 60
   Trp Phe Phe Gln Gly Ala Gln Tyr Trp Val Tyr Asp Gly Glu Lys Pro
   65 70 75 80
   Val Leu Gly Pro Ala Pro Leu Thr Glu Leu Gly Leu Val Arg Phe Pro
   85 90 95
   Val His Ala Ala Leu Val Trp Gly Pro Glu Lys Asn Lys Ile Tyr Phe
   100 105 110
   Phe Arg Gly Arg Asp Tyr Trp Arg Phe His Pro Ser Thr Arg Arg Val
   115 120 125
   Asp Ser Pro Val Pro Arg Arg Ala Thr Asp Trp Arg Gly Val Pro Ser
   130 135 140
   Glu Ile Asp Ala Ala Phe Gln Asp Ala Asp Gly Tyr Ala Tyr Phe Leu
   145 150 155 160
   Arg Gly Arg Leu Tyr Trp Lys Phe Asp Pro Val Lys Val Lys Ala Leu
   165 170 175
   Glu Gly Phe Pro Arg Leu Val Gly Pro Asp Phe Phe Gly Cys Ala Glu
   180 185 190
   Pro Ala Asn Thr Phe Leu
   195
<210> 25
   <211> 198
   <212> PRT
   <213> Mus musculus
<400> 25
   Pro Asp Val Cys Glu Thr Ser Phe Asp Ala Val Ser Thr Ile Arg Gly
   1               5                   10                  15
   Glu Leu Phe Phe Phe Lys Ala Gly Phe Val Trp Arg Leu Arg Ser Gly
               20                  25                  30
   Arg Leu Gln Pro Gly Tyr Pro Ala Leu Ala Ser Arg His Trp Gln Gly
           35                  40                  45
   Leu Pro Ser Pro Val Asp Ala Ala Phe Glu Asp Ala Gln Gly Gln Ile
       50                  55                  60
   Trp Phe Phe Gln Gly Ala Gln Tyr Trp Val Tyr Asp Gly Glu Lys Pro
   65                  70                  75                  80
   Val Leu Gly Pro Ala Pro Leu Ser Lys Leu Gly Leu Gln Gly Ser Pro
                   85                  90                  95
   Val His Ala Ala Leu Val Trp Gly Pro Glu Lys Asn Lys Ile Tyr Phe
               100                 105                 110
   Phe Arg Gly Gly Asp Tyr Trp Arg Phe His Pro Arg Thr Gln Arg Val
           115                 120                 125
   Asp Asn Pro Val Pro Arg Arg Ser Thr Asp Trp Arg Gly Val Pro Ser
       130                 135                 140
   Glu Ile Asp Ala Ala Phe Gln Asp Ala Glu Gly Tyr Ala Tyr Phe Leu
   145                 150                 155                 160
   Arg Gly His Leu Tyr Trp Lys Phe Asp Pro Val Lys Val Lys Val Leu
                   165                 170                 175
   Glu Gly Phe Pro Arg Pro Val Gly Pro Asp Phe Phe Asp Cys Ala Glu
   180 185 190
   Pro Ala Asn Thr Phe Arg
   195
<210> 26
   <211> 192
   <212> PRT
   <213> Homo sapiens
<400> 26
   Pro Ala Leu Cys Asp Pro Asn Leu Ser Phe Asp Ala Val Thr Thr Val
   1 5 10 15
   Gly Asn Lys Ile Phe Phe Phe Lys Asp Arg Phe Phe Trp Leu Lys Val
   20 25 30
   Ser Glu Arg Pro Lys Thr Ser Val Asn Leu Ile Ser Ser Leu Trp Pro
   35 40 45
   Thr Leu Pro Ser Gly Ile Glu Ala Ala Tyr Glu Ile Glu Ala Arg Asn
   50 55 60
   Gln Val Phe Leu Phe Lys Asp Asp Lys Tyr Trp Leu Ile Ser Asn Leu
   65 70 75 80
   Arg Pro Glu Pro Asn Tyr Pro Lys Ser Ile His Ser Phe Gly Phe Pro
   85 90 95
   Asn Phe Val Lys Lys Ile Asp Ala Ala Val Phe Asn Pro Arg Phe Tyr
   100 105 110
   Arg Thr Tyr Phe Phe Val Asp Asn Gln Tyr Trp Arg Tyr Asp Glu Arg
   115 120 125
   Arg Gln Met Met Asp Pro Gly Tyr Pro Lys Leu Ile Thr Lys Asn Phe
   130 135 140
   Gln Gly Ile Gly Pro Lys Ile Asp Ala Val Phe Tyr Ser Lys Asn Lys
   145 150 155 160
   Tyr Tyr Tyr Phe Phe Gln Gly Ser Asn Gln Phe Glu Tyr Asp Phe Leu
   165 170 175
   Leu Gln Arg Ile Thr Lys Thr Leu Lys Ser Asn Ser Trp Phe Gly Cys
   180 185 190
<210> 27
   <211> 191
   <212> PRT
   <213> Mus musculus
<400> 27
   Ser Thr Phe Cys His Gln Ser Leu Ser Phe Asp Ala Val Thr Thr Val
   1 5 10 15
   Gly Glu Lys Ile Leu Phe Phe Lys Asp Trp Phe Phe Trp Trp Lys Leu
   20 25 30
   Pro Gly Ser Pro Ala Thr Asn Ile Thr Ser Ile Ser Ser Ile Trp Pro
   35 40 45
   Ser Ile Pro Ser Ala Ile Gln Ala Ala Tyr Glu Ile Glu Ser Arg Asn
   50 55 60
   Gln Leu Phe Leu Phe Lys Asp Glu Lys Tyr Trp Leu Ile Asn Asn Leu
   65 70 75 80
   Val Pro Glu Pro His Tyr Pro Arg Ser Ile Tyr Ser Leu Gly Phe Ser
   85 90 95
   Ala Ser Val Lys Lys Val Asp Ala Ala Val Phe Asp Pro Leu Arg Gln
   100 105 110
   Lys Val Tyr Phe Phe Val Asp Lys His Tyr Trp Arg Tyr Asp Val Arg
   115 120 125
   Gln Glu Leu Met Asp Pro Ala Tyr Pro Lys Leu Ile Ser Thr His Phe
   130 135 140
   Pro Gly Ile Lys Pro Lys Ile Asp Ala Val Leu Tyr Phe Lys Arg His
   145 150 155 160
   Tyr Tyr Ile Phe Gln Gly Ala Tyr Gln Leu Glu Tyr Asp Pro Leu Phe
   165 170 175
   Arg Arg Val Thr Lys Thr Leu Lys Ser Thr Ser Trp Phe Gly Cys
   180 185 190
<210> 28
   <211> 191
   <212> PRT
   <213> Oryctolagus cuniculus
<400> 28
   Pro Thr Ala Cys Asp His Asn Leu Lys Phe Asp Ala Val Thr Thr Val
   1               5                   10                  15
   Gly Asn Lys Ile Phe Phe Phe Lys Asp Ser Phe Phe Trp Trp Lys Ile
               20                  25                  30
   Pro Lys Ser Ser Thr Thr Ser Val Arg Leu Ile Ser Ser Leu Trp Pro
           35                  40                  45
   Thr Leu Pro Ser Gly Ile Glu Ala Ala Tyr Glu Ile Gly Asp Arg His
       50                  55                  60
   Gln Val Phe Leu Phe Lys Gly Asp Lys Phe Trp Leu Ile Ser His Leu
   65                  70                  75                  80
   Arg Leu Gln Pro Asn Tyr Pro Lys Ser Ile His Ser Leu Gly Phe Pro
                   85                  90                  95
   Asp Phe Val Lys Lys Ile Asp Ala Ala Val Phe Asn Pro Ser Leu Arg
               100                 105                 110
   Lys Thr Tyr Phe Phe Val Asp Asn Leu Tyr Trp Arg Tyr Asp Glu Arg
           115                 120                 125
   Arg Glu Val Met Asp Ala Gly Tyr Pro Lys Leu Ile Thr Lys His Phe
       130                 135                 140
   Pro Gly Ile Gly Pro Lys Ile Asp Ala Val Phe Tyr Phe Gln Arg Tyr
   145                 150                 155                 160
   Tyr Tyr Phe Phe Gln Gly Pro Asn Gln Leu Glu Tyr Asp Thr Phe Ser
                   165                 170                 175
   Ser Arg Val Thr Lys Lys Leu Lys Ser Asn Ser Trp Phe Asp Cys
               180                 185                 190
<210> 29
   <211> 191
   <212> PRT
   <213> Rattus norvegicus
<400> 29
   Ser Thr Val Cys His Gln Ser Leu Ser Phe Asp Ala Val Thr Thr Val
   1               5                   10                  15
   Gly Asp Lys Ile Phe Phe Phe Lys Asp Trp Phe Phe Trp Trp Arg Leu
               20                  25                  30
   Pro Gly Ser Pro Ala Thr Asn Ile Thr Ser Ile Ser Ser Met Trp Pro
           35                  40                  45
   Thr Ile Pro Ser Gly Ile Gln Ala Ala Tyr Glu Ile Gly Gly Arg Asn
       50                  55                  60
   Gln Leu Phe Leu Phe Lys Asp Glu Lys Tyr Trp Leu Ile Asn Asn Leu
   65                  70                  75                  80
   Val Pro Glu Pro His Tyr Pro Arg Ser Ile His Ser Leu Gly Phe Pro
                   85                  90                  95
   Ala Ser Val Lys Lys Ile Asp Ala Ala Val Phe Asp Pro Leu Arg Gln
               100                 105                 110
   Lys Val Tyr Phe Phe Val Asp Lys Gln Tyr Trp Arg Tyr Asp Val Arg
           115                 120                 125
   Gln Glu Leu Met Asp Ala Ala Tyr Pro Lys Leu Ile Ser Thr His Phe
       130                 135                 140
   Pro Gly Ile Arg Pro Lys Ile Asp Ala Val Leu Tyr Phe Lys Arg His
   145                 150                 155                 160
   Tyr Tyr Ile Phe Gln Gly Ala Tyr Gln Leu Glu Tyr Asp Pro Leu Leu
                   165                 170                 175
   Asp Arg Val Thr Lys Thr Leu Ser Ser Thr Ser Trp Phe Gly Cys
               180                 185                 190
<210> 30
   <211> 191
   <212> PRT
   <213> Bos taurus
<400> 30
   Pro Asp Lys Cys Asp Pro Ser Leu Ser Leu Asp Ala Ile Thr Ser Leu
   1               5                   10                  15
   Arg Gly Glu Thr Leu Ile Phe Lys Asp Arg Phe Phe Trp Arg Leu His
               20                  25                  30
   Pro Gln Gln Val Glu Ala Glu Leu Phe Leu Thr Lys Ser Phe Gly Pro
           35                  40                  45
   Glu Leu Pro Asn Arg Ile Asp Ala Ala Tyr Glu His Pro Ser His Asp
       50                  55                  60
   Leu Ile Phe Ile Phe Arg Gly Arg Lys Phe Trp Ala Leu Ser Gly Tyr
   65                  70                  75                  80
   Asp Ile Leu Glu Asp Tyr Pro Lys Lys Ile Ser Glu Leu Gly Phe Pro
                   85                  90                  95
   Lys His Val Lys Lys Ile Ser Ala Ala Leu His Phe Glu Asp Ser Gly
               100                 105                 110
   Lys Thr Leu Phe Phe Ser Glu Asn Gln Val Trp Ser Tyr Asp Asp Thr
           115                 120                 125
   Asn His Val Met Asp Lys Asp Tyr Pro Arg Leu Ile Glu Glu Val Phe
       130                 135                 140
   Pro Gly Ile Gly Asp Lys Val Asp Ala Val Tyr Gln Lys Asn Gly Tyr
   145                 150                 155                 160
   Ile Tyr Phe Phe Asn Gly Pro Ile Gln Phe Glu Tyr Ser Ile Trp Ser
                   165                 170                 175
   Asn Arg Ile Val Arg Val Met Thr Thr Asn Ser Leu Leu Trp Cys
               180                 185                 190
<210> 31
   <211> 191
   <212> PRT
   <213> Equus caballus
<400> 31
   Pro Asp Lys Cys Asp Pro Ser Leu Ser Leu Asp Ala Ile Thr Ser Leu
   1               5                   10                  15
   Arg Gly Glu Thr Met Val Phe Lys Asp Arg Phe Phe Trp Arg Leu His
               20                  25                  30
   Pro Gln Leu Val Asp Ala Glu Leu Phe Leu Thr Lys Ser Phe Trp Pro
           35                  40                  45
   Glu Leu Pro Asn Arg Ile Asp Ala Ala Tyr Glu His Pro Ser Lys Asp
       50                  55                  60
   Leu Ile Phe Ile Phe Arg Gly Arg Lys Phe Trp Ala Leu Asn Gly Tyr
   65                  70                  75                  80
   Asp Ile Leu Glu Gly Tyr Pro Gln Lys Ile Ser Glu Leu Gly Phe Pro
                   85                  90                  95
   Lys Asp Val Lys Lys Ile Ser Ala Ala Val His Phe Glu Asp Thr Gly
               100                 105                 110
   Lys Thr Leu Phe Phe Ser Gly Asn Gln Val Trp Arg Tyr Asp Asp Thr
           115                 120                 125
   Asn Arg Met Met Asp Lys Asp Tyr Pro Arg Leu Ile Glu Glu Asp Phe
       130                 135                 140
   Pro Gly Ile Gly Asp Lys Val Asp Ala Val Tyr Glu Lys Asn Gly Tyr
   145                 150                 155                 160
   Ile Tyr Phe Phe Asn Gly Pro Ile Gln Phe Glu Tyr Ser Ile Trp Ser
                   165                 170                 175
   Asn Arg Ile Val Arg Val Met Pro Thr Asn Ser Leu Leu Trp Cys
               180                 185                 190
<210> 32
   <211> 191
   <212> PRT
   <213> Homo sapiens
<400> 32
   Pro Asp Lys Cys Asp Pro Ser Leu Ser Leu Asp Ala Ile Thr Ser Leu
   1               5                   10                  15
   Arg Gly Glu Thr Met Ile Phe Lys Asp Arg Phe Phe Trp Arg Leu His
               20                  25                  30
   Pro Gln Gln Val Asp Ala Glu Leu Phe Leu Thr Lys Ser Phe Trp Pro
           35                  40                  45
   Glu Leu Pro Asn Arg Ile Asp Ala Ala Tyr Glu His Pro Ser His Asp
       50                  55                  60
   Leu Ile Phe Ile Phe Arg Gly Arg Lys Phe Trp Ala Leu Asn Gly Tyr
   65                  70                  75                  80
   Asp Ile Leu Glu Gly Tyr Pro Lys Lys Ile Ser Glu Leu Gly Leu Pro
                   85                  90                  95
   Lys Glu Val Lys Lys Ile Ser Ala Ala Val His Phe Glu Asp Thr Gly
               100                 105                 110
   Lys Thr Leu Leu Phe Ser Gly Asn Gln Val Trp Arg Tyr Asp Asp Thr
           115                 120                 125
   Asn His Ile Met Asp Lys Asp Tyr Pro Arg Leu Ile Glu Glu Asp Phe
       130                 135                 140
   Pro Gly Ile Gly Asp Lys Val Asp Ala Val Tyr Glu Lys Asn Gly Tyr
   145                 150                 155                 160
   Ile Tyr Phe Phe Asn Gly Pro Ile Gln Phe Glu Tyr Ser Ile Trp Ser
                   165                 170                 175
   Asn Arg Ile Val Arg Val Met Pro Ala Asn Ser Ile Leu Trp Cys
               180                 185                 190
<210> 33
   <211> 191
   <212> PRT
   <213> Oryctolagus cuniculus
<400> 33
   Pro Asp Lys Cys Asp Pro Ser Leu Ser Leu Asp Ala Ile Thr Ser Leu
   1               5                   10                  15
   Arg Gly Glu Thr Met Ile Phe Lys Asp Arg Phe Phe Trp Arg Leu His
               20                  25                  30
   Pro Gln Gln Val Asp Ala Glu Leu Phe Leu Thr Lys Ser Phe Trp Pro
           35                  40                  45
   Glu Leu Pro Asn Arg Ile Asp Ala Ala Tyr Glu His Pro Ala Arg Asp
       50                  55                  60
   Leu Ile Phe Ile Phe Arg Gly Lys Lys Phe Trp Ala Pro Asn Gly Tyr
   65                  70                  75                  80
   Asp Ile Leu Glu Gly Tyr Pro Gln Lys Leu Ser Glu Leu Gly Phe Pro
                   85                  90                  95
   Arg Glu Val Lys Lys Ile Ser Ala Ala Val His Phe Glu Asp Thr Gly
               100                 105                 110
   Lys Thr Leu Phe Phe Ser Gly Asn Gln Val Trp Ser Tyr Asp Asp Thr
           115                 120                 125
   Asn His Thr Met Asp Gln Asp Tyr Pro Arg Leu Ile Glu Glu Glu Phe
       130                 135                 140
   Pro Gly Ile Gly Gly Lys Val Asp Ala Val Tyr Glu Lys Asn Gly Tyr
   145                 150                 155                 160
   Ile Tyr Phe Phe Asn Gly Pro Ile Gln Phe Glu Tyr Ser Ile Trp Ser
                   165                 170                 175
   Lys Arg Ile Val Arg Val Met Pro Thr Asn Ser Leu Leu Trp Cys
               180                 185                 190
<210> 34
   <211> 196
   <212> PRT
   <213> Homo sapiens
<400> 34
   Pro Asn Ile Cys Asp Gly Asn Phe Asp Thr Val Ala Met Leu Arg Gly
   1               5                   10                  15
   Glu Met Phe Val Phe Lys Lys Arg Trp Phe Trp Arg Val Arg Asn Asn
           20                  25                  30
   Gln Val Met Asp Gly Tyr Pro Met Pro Ile Gly Gln Phe Trp Arg Gly
       35                  40                  45
   Leu Pro Ala Ser Ile Asn Thr Ala Tyr Glu Arg Lys Asp Gly Lys Phe
       50                  55                  60
   Val Phe Phe Lys Gly Asp Lys His Trp Val Phe Asp Glu Ala Ser Leu
   65                  70                  75                  80
   Glu Pro Gly Tyr Pro Lys His Ile Lys Glu Leu Gly Arg Gly Leu Pro
                   85                  90                  95
   Thr Asp Lys Ile Asp Ala Ala Leu Phe Trp Met Pro Asn Gly Lys Thr
               100                 105                 110
   Tyr Phe Phe Arg Gly Asn Lys Tyr Tyr Arg Phe Asn Glu Glu Leu Arg
           115                 120                 125
   Ala Val Asp Ser Glu Tyr Pro Lys Asn Ile Lys Val Trp Glu Gly Ile
       130                 135                 140
   Pro Glu Ser Pro Arg Gly Ser Phe Met Gly Ser Asp Glu Val Phe Thr
   145                 150                 155                 160
   Tyr Phe Tyr Lys Gly Asn Lys Tyr Trp Lys Phe Asn Asn Gln Lys Leu
                   165                 170                 175
   Lys Val Glu Pro Gly Tyr Pro Lys Ser Ala Leu Arg Asp Trp Met Gly
               180                 185                 190
   Cys Pro Ser Gly
           195
<210> 35
   <211> 196
   <212> PRT
   <213> Mus musculus
<400> 35
   Pro Asn Ile Cys Asp Gly Asn Phe Asp Thr Val Ala Met Leu Arg Gly
   1               5                   10                  15
   Glu Met Phe Val Phe Lys Glu Arg Trp Phe Trp Arg Val Arg Asn Asn
   20 25 30
   Gln Val Met Asp Gly Tyr Pro Met Pro Ile Gly Gln Phe Trp Arg Gly
   35 40 45
   Leu Pro Ala Ser Ile Asn Thr Ala Tyr Glu Arg Lys Asp Gly Lys Phe
   50 55 60
   Val Phe Phe Lys Gly Asp Lys His Trp Val Phe Asp Glu Ala Ser Leu
   65 70 75 80
   Glu Pro Gly Tyr Pro Lys His Ile Lys Glu Leu Gly Arg Gly Leu Pro
   85 90 95
   Thr Asp Lys Ile Asp Ala Ala Leu Phe Trp Met Pro Asn Gly Lys Thr
   100 105 110
   Tyr Phe Phe Arg Gly Asn Lys Tyr Tyr Arg Phe Asn Glu Glu Phe Arg
   115 120 125
   Ala Val Asp Ser Glu Tyr Pro Lys Asn Ile Lys Val Trp Glu Gly Ile
   130 135 140
   Pro Glu Ser Pro Arg Gly Ser Phe Met Gly Ser Asp Glu Val Phe Thr
   145 150 155 160
   Tyr Phe Tyr Lys Gly Asn Lys Tyr Trp Lys Phe Asn Asn Gln Lys Leu
   165 170 175
   Lys Val Glu Pro Gly Tyr Pro Lys Ser Ala Leu Arg Asp Trp Met Gly
   180 185 190
   Cys Pro Ser Gly
   195
<210> 36
   <211> 196
   <212> PRT
   <213> Sus scrofa
<400> 36
   Pro Asn Ile Cys Asp Gly Asn Phe Asp Thr Val Ala Met Leu Arg Gly
   1 5 10 15
   Glu Met Phe Val Phe Lys Glu Arg Trp Phe Trp Arg Val Arg Lys Asn
               20                  25                  30
   Gln Val Met Asp Gly Tyr Pro Met Pro Ile Gly Gln Phe Trp Arg Gly
           35                  40                  45
   Leu Pro Ala Ser Ile Asn Thr Ala Tyr Glu Arg Lys Asp Gly Lys Phe
       50                  55                  60
   Val Phe Phe Lys Gly Asp Lys His Trp Val Phe Asp Glu Ala Ser Leu
   65                  70                  75                  80
   Glu Pro Gly Tyr Pro Lys His Ile Lys Glu Leu Gly Arg Arg Leu Pro
                   85                  90                  95
   Thr Asp Lys Ile Asp Ala Ala Leu Phe Trp Met Pro Asn Gly Lys Asp
               100                 105                 110
   Tyr Phe Phe Arg Gly Asn Lys Tyr Tyr Arg Phe Asn Glu Glu Leu Arg
           115                 120                 125
   Ala Val Asp Ser Glu Tyr Pro Lys Asn Ile Lys Val Trp Glu Gly Ile
       130                 135                 140
   Pro Glu Ser Pro Arg Gly Ser Phe Met Gly Ser Asp Glu Val Phe Thr
   145                 150                 155                 160
   Tyr Phe Tyr Lys Gly Asn Lys Tyr Trp Lys Phe Asn Asn Gln Lys Leu
                   165                 170                 175
   Lys Val Glu Pro Gly Tyr Pro Lys Ser Ala Leu Arg Asp Trp Met Gly
               180                 185                 190
   Cys Pro Ser Gly
           195
<210> 37
   <211> 196
   <212> PRT
   <213> Oryctolagus cuniculus
<400> 37
   Pro Lys Ile Cys Asp Gly Asn Phe Asp Thr Val Ala Val Phe Arg Gly
   1               5                   10                  15
   Glu Met Phe Val Phe Lys Glu Arg Trp Phe Trp Arg Val Arg Asn Asn
               20                  25                  30
   Gln Val Met Asp Gly Tyr Pro Met Pro Ile Gly Gln Leu Trp Arg Gly
           35                  40                  45
   Leu Pro Ala Ser Ile Asn Thr Ala Tyr Glu Arg Lys Asp Gly Lys Phe
       50                  55                  60
   Val Phe Phe Lys Gly Asp Lys His Trp Val Phe Asp Glu Ala Ser Leu
   65                  70                  75                  80
   Glu Pro Gly Tyr Pro Lys His Ile Lys Glu Leu Gly Arg Gly Leu Pro
                   85                  90                  95
   Thr Asp Lys Ile Asp Ala Ala Leu Phe Trp Met Pro Asn Gly Lys Thr
               100                 105                 110
   Tyr Phe Phe Arg Gly Asn Lys Tyr Tyr Arg Phe Asn Glu Glu Leu Arg
           115                 120                 125
   Ala Val Asp Ser Glu Tyr Pro Lys Asn Ile Lys Val Trp Glu Gly Ile
       130                 135                 140
   Pro Glu Ser Pro Arg Gly Ser Phe Met Gly Ser Asp Glu Val Phe Thr
   145                 150                 155                 160
   Tyr Phe Tyr Lys Gly Asn Lys Tyr Trp Lys Phe Asn Asn Gln Lys Leu
                   165                 170                 175
   Lys Val Glu Pro Gly Tyr Pro Lys Ser Ala Leu Arg Asp Trp Met Gly
               180                 185                 190
   Cys Pro Ala Gly
           195
<210> 38
   <211> 196
   <212> PRT
   <213> Rattus norvegicus
<400> 38
   Pro Asn Ile Cys Asp Gly Asn Phe Asp Thr Val Ala Met Leu Arg Gly
   1               5                   10                  15
   Glu Met Phe Val Phe Lys Glu Arg Trp Phe Trp Arg Val Arg Asn Asn
               20                  25                  30
   Gln Val Met Asp Gly Tyr Pro Met Pro Ile Gly Gln Phe Trp Arg Gly
           35                  40                  45
   Leu Pro Ala Ser Ile Asn Thr Ala Tyr Glu Arg Lys Asp Gly Lys Phe
       50                  55                  60
   Val Phe Phe Lys Gly Asp Lys His Trp Val Phe Asp Glu Ala Ser Leu
   65                  70                  75                  80
   Glu Pro Gly Tyr Pro Lys His Ile Lys Glu Leu Gly Arg Gly Leu Pro
                   85                  90                  95
   Thr Asp Lys Ile Asp Ala Ala Leu Phe Trp Met Pro Asn Gly Lys Thr
               100                 105                 110
   Tyr Phe Phe Arg Gly Asn Lys Tyr Tyr Arg Phe Asn Glu Glu Phe Arg
           115                 120                 125
   Ala Val Asp Ser Glu Tyr Pro Lys Asn Ile Lys Val Trp Glu Gly Ile
       130                 135                 140
   Pro Glu Ser Pro Arg Gly Ser Phe Met Gly Ser Asp Glu Val Phe Thr
   145                 150                 155                 160
   Tyr Phe Tyr Lys Gly Asn Lys Tyr Trp Lys Phe Asn Asn Gln Lys Leu
                   165                 170                 175
   Lys Val Glu Pro Gly Tyr Pro Lys Ser Ala Leu Arg Asp Trp Met Gly
               180                 185                 190
   Cys Pro Ser Gly
           195
<210> 39
   <211> 196
   <212> PRT
   <213> Homo sapiens
<400> 39
   Pro Asn Ile Cys Asp Gly Asp Phe Asp Thr Val Ala Met Leu Arg Gly
   1               5                   10                  15
   Glu Met Phe Val Phe Lys Gly Arg Trp Phe Trp Arg Val Arg His Asn
               20                  25                  30
   Arg Val Leu Asp Asn Tyr Pro Met Pro Ile Gly His Phe Trp Arg Gly
           35                  40                  45
   Leu Pro Gly Asp Ile Ser Ala Ala Tyr Glu Arg Gln Asp Gly Arg Phe
       50                  55                  60
   Val Phe Phe Lys Gly Asp Arg Tyr Trp Leu Phe Arg Glu Ala Asn Leu
   65                  70                  75                  80
   Glu Pro Gly Tyr Pro Gln Pro Leu Thr Ser Tyr Gly Leu Gly Ile Pro
                   85                  90                  95
   Tyr Asp Arg Ile Asp Thr Ala Ile Trp Trp Glu Pro Thr Gly His Thr
               100                 105                 110
   Phe Phe Phe Gln Glu Asp Arg Tyr Trp Arg Phe Asn Glu Glu Thr Gln
           115                 120                 125
   Arg Gly Asp Pro Gly Tyr Pro Lys Pro Ile Ser Val Trp Gln Gly Ile
       130                 135                 140
   Pro Ala Ser Pro Lys Gly Ala Phe Leu Ser Asn Asp Ala Ala Tyr Thr
   145                 150                 155                 160
   Tyr Phe Tyr Lys Gly Thr Lys Tyr Trp Lys Phe Asp Asn Glu Arg Leu
                   165                 170                 175
   Arg Met Glu Pro Gly Tyr Pro Lys Ser Ile Leu Arg Asp Phe Met Gly
               180                 185                 190
   Cys Gln Glu His
           195
<210> 40
   <211> 196
   <212> PRT
   <213> Mus musculus
<400> 40
   Pro Asn Ile Cys Asp Gly Asn Phe Asp Thr Val Ala Val Leu Arg Gly
   1               5                   10                  15
   Glu Met Phe Val Phe Lys Gly Arg Trp Phe Trp Arg Val Arg His Asn
               20                  25                  30
   Arg Val Leu Asp Asn Tyr Pro Met Pro Ile Gly His Phe Trp Arg Gly
           35                  40                  45
   Leu Pro Gly Asn Ile Ser Ala Ala Tyr Glu Arg Gln Asp Gly His Phe
       50                  55                  60
   Val Phe Phe Lys Gly Asn Arg Tyr Trp Leu Phe Arg Glu Ala Asn Leu
   65                  70                  75                  80
   Glu Pro Gly Tyr Pro Gln Pro Leu Ser Ser Tyr Gly Thr Asp Ile Pro
                   85                  90                  95
   Tyr Asp Arg Ile Asp Thr Ala Ile Trp Trp Glu Pro Thr Gly His Thr
               100                 105                 110
   Phe Phe Phe Gln Ala Asp Arg Tyr Trp Arg Phe Asn Glu Glu Thr Gln
           115                 120                 125
   His Gly Asp Pro Gly Tyr Pro Lys Pro Ile Ser Val Trp Gln Gly Ile
       130                 135                 140
   Pro Thr Ser Pro Lys Gly Ala Phe Leu Ser Asn Asp Ala Ala Tyr Thr
   145                 150                 155                 160
   Tyr Phe Tyr Lys Gly Thr Lys Tyr Trp Lys Phe Asn Asn Glu Arg Leu
                   165                 170                 175
   Arg Met Glu Pro Gly His Pro Lys Ser Ile Leu Arg Asp Phe Met Gly
               180                 185                 190
   Cys Gln Glu His
           195
<210> 41
   <211> 196
   <212> PRT
   <213> Homo sapiens
<400> 41
   Pro Asn Ile Cys Asp Gly Asn Phe Asn Thr Leu Ala Ile Leu Arg Arg
   1               5                   10                  15
   Glu Met Phe Val Phe Lys Asp Gln Trp Phe Trp Arg Val Arg Asn Asn
               20                  25                  30
   Arg Val Met Asp Gly Tyr Pro Met Gln Ile Thr Tyr Phe Trp Arg Gly
           35                  40                  45
   Leu Pro Pro Ser Ile Asp Ala Val Tyr Glu Asn Ser Asp Gly Asn Phe
       50                  55                  60
   Val Phe Phe Lys Gly Asn Lys Tyr Trp Val Phe Lys Asp Thr Thr Leu
   65                  70                  75                  80
   Gln Pro Gly Tyr Pro His Asp Leu Ile Thr Leu Gly Ser Gly Ile Pro
                   85                  90                  95
   Pro His Gly Ile Asp Ser Ala Ile Trp Trp Glu Asp Val Gly Lys Thr
               100                 105                 110
   Tyr Phe Phe Lys Gly Asp Arg Tyr Trp Arg Tyr Ser Glu Glu Met Lys
           115                 120                 125
   Thr Met Asp Pro Gly Tyr Pro Lys Pro Ile Thr Val Trp Lys Gly Ile
       130                 135                 140
   Pro Glu Ser Pro Gln Gly Ala Phe Val His Lys Glu Asn Gly Phe Thr
   145                 150                 155                 160
   Tyr Phe Tyr Lys Gly Lys Glu Tyr Trp Lys Phe Asn Asn Gln Ile Leu
                   165                 170                 175
   Lys Val Glu Pro Gly Tyr Pro Arg Ser Ile Leu Lys Asp Phe Met Gly
               180                 185                 190
   Cys Asp Gly Pro
           195
<210> 42
   <211> 196
   <212> PRT
   <213> Mus musculus
<400> 42
   Pro Asn Ile Cys Asp Gly Asn Phe Asn Thr Leu Ala Ile Leu Arg Arg
   1               5                   10                  15
   Glu Met Phe Val Phe Lys Asp Gln Trp Phe Trp Arg Val Arg Asn Asn
               20                  25                  30
   Arg Val Met Asp Gly Tyr Pro Met Gln Ile Thr Tyr Phe Trp Arg Gly
           35                  40                  45
   Leu Pro Pro Ser Ile Asp Ala Val Tyr Glu Asn Ser Asp Gly Asn Phe
       50                  55                  60
   Val Phe Phe Lys Gly Asn Lys Tyr Trp Val Phe Lys Asp Thr Thr Leu
   65                  70                  75                  80
   Gln Pro Gly Tyr Pro His Asp Leu Ile Thr Leu Gly Asn Gly Ile Pro
                   85                  90                  95
   Pro His Gly Ile Asp Ser Ala Ile Trp Trp Glu Asp Val Gly Lys Thr
               100                 105                 110
   Tyr Phe Phe Lys Gly Asp Arg Tyr Trp Arg Tyr Ser Glu Glu Met Lys
           115                 120                 125
   Thr Met Asp Pro Gly Tyr Pro Lys Pro Ile Thr Ile Trp Lys Gly Ile
       130                 135                 140
   Pro Glu Ser Pro Gln Gly Ala Phe Val His Lys Glu Asn Gly Phe Thr
   145                 150                 155                 160
   Tyr Phe Tyr Lys Gly Lys Glu Tyr Trp Lys Phe Asn Asn Gln Ile Leu
                   165                 170                 175
   Lys Val Glu Pro Gly Tyr Pro Arg Ser Ile Leu Lys Asp Phe Met Gly
               180                 185                 190
   Cys Asp Gly Pro
                           195
<210> 43
   <211> 196
   <212> PRT
   <213> Rattus norvegicus
<400> 43
   Pro Asn Ile Cys Asp Gly Asn Phe Asn Thr Leu Ala Ile Leu Arg Arg
   1               5                   10                  15
   Glu Met Phe Val Phe Lys Asp Gln Trp Phe Trp Arg Val Arg Asn Asn
               20                  25                  30
   Arg Val Met Asp Gly Tyr Pro Met Gln Ile Thr Tyr Phe Trp Arg Gly
           35                  40                  45
   Leu Pro Pro Ser Ile Asp Ala Val Tyr Glu Asn Ser Asp Gly Asn Phe
       50                  55                  60
   Val Phe Phe Lys Gly Asn Lys Tyr Trp Val Phe Lys Asp Thr Thr Leu
   65                  70                  75                  80
   Gln Pro Gly Tyr Pro His Asp Leu Ile Thr Leu Gly Asn Gly Ile Pro
                   85                  90                  95
   Pro His Gly Ile Asp Ser Ala Ile Trp Trp Glu Asp Val Gly Lys Thr
               100                 105                 110
   Tyr Phe Phe Lys Gly Asp Arg Tyr Trp Arg Tyr Ser Glu Glu Met Lys
           115                 120                 125
   Thr Met Asp Pro Gly Tyr Pro Lys Pro Ile Thr Ile Trp Lys Gly Ile
       130                 135                 140
   Pro Glu Ser Pro Gln Gly Ala Phe Val His Lys Glu Asn Gly Phe Thr
   145                 150                 155                 160
   Tyr Phe Tyr Lys Gly Lys Glu Tyr Trp Lys Phe Asn Asn Gln Ile Leu
                   165                 170                 175
   Lys Val Glu Pro Gly Tyr Pro Arg Ser Ile Leu Lys Asp Phe Met Gly
               180                 185                 190
   Cys Asp Gly Pro
               195
<210> 44
   <211> 198
   <212> PRT
   <213> Homo sapiens
<400> 44
   Pro His Arg Cys Ser Thr His Phe Asp Ala Val Ala Gln Ile Arg Gly
   1               5                   10                  15
   Glu Ala Phe Phe Phe Lys Gly Lys Tyr Phe Trp Arg Leu Thr Arg Asp
               20                  25                  30
   Arg His Leu Val Ser Leu Gln Pro Ala Gln Met His Arg Phe Trp Arg
           35                  40                  45
   Gly Leu Pro Leu His Leu Asp Ser Val Asp Ala Val Tyr Glu Arg Thr
       50                  55                  60
   Ser Asp His Lys Ile Val Phe Phe Lys Gly Asp Arg Tyr Trp Val Phe
   65                  70                  75                  80
   Lys Asp Asn Asn Val Glu Glu Gly Tyr Pro Arg Pro Val Ser Asp Phe
                   85                  90                  95
   Ser Leu Pro Pro Gly Gly Ile Asp Ala Ala Phe Ser Trp Ala His Asn
               100                 105                 110
   Asp Arg Thr Tyr Phe Phe Lys Asp Gln Leu Tyr Trp Arg Tyr Asp Asp
           115                 120                 125
   His Thr Arg His Met Asp Pro Gly Tyr Pro Ala Gln Ser Pro Leu Trp
       130                 135                 140
   Arg Gly Val Pro Ser Thr Leu Asp Asp Ala Met Arg Trp Ser Asp Gly
   145                 150                 155                 160
   Ala Ser Tyr Phe Phe Arg Gly Gln Glu Tyr Trp Lys Val Leu Asp Gly
                   165                 170                 175
   Glu Leu Glu Val Ala Pro Gly Tyr Pro Gln Ser Thr Ala Arg Asp Trp
               180                 185                 190
   Leu Val Cys Gly Asp Ser
           195
<210> 45
   <211> 198
   <212> PRT
   <213> Mus musculus
<400> 45
   Pro His Arg Cys Thr Ala His Phe Asp Ala Val Ala Gln Ile Arg Gly
   1               5                   10                  15
   Glu Ala Phe Phe Phe Lys Gly Lys Tyr Phe Trp Arg Leu Thr Arg Asp
               20                  25                  30
   Arg His Leu Val Ser Leu Gln Pro Ala Gln Met His Arg Phe Trp Arg
           35                  40                  45
   Gly Leu Pro Leu His Leu Asp Ser Val Asp Ala Val Tyr Glu Arg Thr
       50                  55                  60
   Ser Asp His Lys Ile Val Phe Phe Lys Gly Asp Arg Tyr Trp Val Phe
   65                  70                  75                  80
   Lys Asp Asn Asn Val Glu Glu Gly Tyr Pro Arg Pro Val Ser Asp Phe
                   85                  90                  95
   Ser Leu Pro Pro Gly Gly Ile Asp Ala Val Phe Ser Trp Ala His Asn
               100                 105                 110
   Asp Arg Thr Tyr Phe Phe Lys Asp Gln Leu Tyr Trp Arg Tyr Asp Asp
           115                 120                 125
   His Thr Arg Arg Met Asp Pro Gly Tyr Pro Ala Gln Gly Pro Leu Trp
       130                 135                 140
   Arg Gly Val Pro Ser Met Leu Asp Asp Ala Met Arg Trp Ser Asp Gly
   145                 150                 155                 160
   Ala Ser Tyr Phe Phe Arg Gly Gln Glu Tyr Trp Lys Val Leu Asp Gly
                   165                 170                 175
   Glu Leu Glu Ala Ala Pro Gly Tyr Pro Gln Ser Thr Ala Arg Asp Trp
                   180                 185                 190
   Leu Val Cys Gly Glu Pro
           195
<210> 46
   <211> 191
   <212> PRT
   <213> Xenopus laevis
<400> 46
   Pro Ser Arg Cys Asp Pro Asn Val Val Phe Asn Ala Val Thr Thr Met
   1               5                   10                  15
   Arg Gly Glu Leu Ile Phe Phe Val Lys Arg Phe Leu Trp Arg Lys His
               20                  25                  30
   Pro Gln Ala Ser Glu Ala Glu Leu Met Phe Val Gln Ala Phe Trp Pro
           35                  40                  45
   Ser Leu Pro Thr Asn Ile Asp Ala Ala Tyr Glu Asn Pro Ile Thr Glu
       50                  55                  60
   Gln Ile Leu Val Phe Lys Gly Ser Lys Tyr Thr Ala Leu Asp Gly Phe
   65                  70                  75                  80
   Asp Val Val Gln Gly Tyr Pro Arg Asn Ile Tyr Ser Leu Gly Phe Pro
                   85                  90                  95
   Lys Thr Val Lys Arg Ile Asp Ala Ala Val His Ile Glu Gln Leu Gly
               100                 105                 110
   Lys Thr Tyr Phe Phe Ala Ala Lys Lys Tyr Trp Ser Tyr Asp Glu Asp
           115                 120                 125
   Lys Lys Gln Met Asp Lys Gly Phe Pro Lys Gln Ile Ser Asn Asp Phe
       130                 135                 140
   Pro Gly Ile Pro Asp Lys Ile Asp Ala Ala Phe Tyr Tyr Arg Gly Arg
   145                 150                 155                 160
   Leu Tyr Phe Phe Ile Gly Arg Ser Gln Phe Glu Tyr Asn Ile Asn Ser
                   165                 170                 175
   Lys Arg Ile Val Gln Val Leu Arg Ser Asn Ser Trp Leu Gly Cys
               180                 185                 190
<210> 47
   <211> 190
   <212> PRT
   <213> Homo sapiens
<400> 47
   Pro Asp Pro Cys Ser Ser Glu Leu Asp Ala Met Met Leu Gly Pro Arg
   1               5                   10                  15
   Gly Lys Thr Tyr Ala Phe Lys Gly Asp Tyr Val Trp Thr Val Ser Asp
               20                  25                  30
   Ser Gly Pro Gly Pro Leu Phe Arg Val Ser Ala Leu Trp Glu Gly Leu
           35                  40                  45
   Pro Gly Asn Leu Asp Ala Ala Val Tyr Ser Pro Arg Thr Gln Trp Ile
       50                  55                  60
   His Phe Phe Lys Gly Asp Lys Val Trp Arg Tyr Ile Asn Phe Lys Met
   65                  70                  75                  80
   Ser Pro Gly Phe Pro Lys Lys Leu Asn Arg Val Glu Pro Asn Leu Asp
                   85                  90                  95
   Ala Ala Leu Tyr Trp Pro Leu Asn Gln Lys Val Phe Leu Phe Lys Gly
               100                 105                 110
   Ser Gly Tyr Trp Gln Trp Asp Glu Leu Ala Arg Thr Asp Phe Ser Ser
           115                 120                 125
   Tyr Pro Lys Pro Ile Lys Gly Leu Phe Thr Gly Val Pro Asn Gln Pro
       130                 135                 140
   Ser Ala Ala Met Ser Trp Gln Asp Gly Arg Val Tyr Phe Phe Lys Gly
   145                 150                 155                 160
   Lys Val Tyr Trp Arg Leu Asn Gln Gln Leu Arg Val Glu Lys Gly Tyr
                   165                 170                 175
   Pro Arg Asn Ile Ser His Asn Trp Met His Cys Arg Pro Arg
               180                 185                 190
<210> 48
   <211> 189
   <212> PRT
   <213> Mus musculus
<400> 48
   Pro Asn Pro Cys Ser Gly Glu Val Asp Ala Met Val Leu Gly Pro Arg
   1               5                   10                  15
   Gly Lys Thr Tyr Ala Phe Lys Gly Asp Tyr Val Trp Thr Val Thr Asp
               20                  25                  30
   Ser Gly Pro Gly Pro Leu Phe Gln Ile Ser Ala Leu Trp Glu Gly Leu
           35                  40                  45
   Pro Gly Asn Leu Asp Ala Ala Val Tyr Ser Pro Arg Thr Arg Arg Thr
       50                  55                  60
   His Phe Phe Lys Gly Asn Lys Val Trp Arg Tyr Val Asp Phe Lys Met
   65                  70                  75                  80
   Ser Pro Gly Phe Pro Met Lys Phe Asn Arg Val Glu Pro Asn Leu Asp
                   85                  90                  95
   Ala Ala Leu Tyr Trp Pro Val Asn Gln Lys Val Phe Leu Phe Lys Gly
               100                 105                 110
   Ser Gly Tyr Trp Gln Trp Asp Glu Leu Ala Arg Thr Asp Leu Ser Arg
           115                 120                 125
   Tyr Pro Lys Pro Ile Lys Glu Leu Phe Thr Gly Val Pro Asp Arg Pro
       130                 135                 140
   Ser Ala Ala Met Ser Trp Gln Asp Gly Gln Val Tyr Phe Phe Lys Gly
   145                 150                 155                 160
   Lys Glu Tyr Trp Arg Leu Asn Gln Gln Leu Arg Val Ala Lys Gly Tyr
                   165                 170                 175
   Pro Arg Asn Thr Thr His Trp Met His Cys Gly Ser Gln
               180                 185
<210> 49
   <211> 191
   <212> PRT
   <213> Bos taurus
<400> 49
   Pro Asp Leu Cys Asp Ser Asn Leu Ser Phe Asp Ala Val Thr Met Leu
   1               5                   10                  15
   Gly Lys Glu Leu Leu Leu Phe Arg Asp Arg Ile Phe Trp Arg Arg Gln
               20                  25                  30
   Val His Leu Met Ser Gly Ile Arg Pro Ser Thr Ile Thr Ser Ser Phe
           35                  40                  45
   Pro Gln Leu Met Ser Asn Val Asp Ala Ala Tyr Glu Val Ala Glu Arg
       50                  55                  60
   Gly Thr Ala Tyr Phe Phe Lys Gly Pro His Tyr Trp Ile Thr Arg Gly
   65                  70                  75                  80
   Phe Gln Met Gln Gly Pro Pro Arg Thr Ile Tyr Asp Phe Gly Phe Pro
                   85                  90                  95
   Arg Tyr Val Gln Arg Ile Asp Ala Ala Val Tyr Leu Lys Asp Ala Gln
               100                 105                 110
   Lys Thr Leu Phe Phe Val Gly Asp Glu Tyr Tyr Ser Tyr Asp Glu Arg
           115                 120                 125
   Lys Arg Lys Met Glu Lys Asp Tyr Pro Lys Ser Thr Glu Glu Glu Phe
       130                 135                 140
   Ser Gly Val Asn Gly Gln Ile Asp Ala Ala Val Glu Leu Asn Gly Tyr
   145                 150                 155                 160
   Ile Tyr Phe Phe Ser Gly Pro Lys Ala Tyr Lys Ser Asp Thr Glu Lys
                   165                 170                 175
   Glu Asp Val Val Ser Glu Leu Lys Ser Ser Ser Trp Ile Gly Cys
               180                 185                 190
<210> 50
   <211> 191
   <212> PRT
   <213> Homo sapiens
<400> 50
   Pro Asp Leu Cys Asp Ser Ser Ser Ser Phe Asp Ala Val Thr Met Leu
   1               5                   10                  15
   Gly Lys Glu Leu Leu Leu Phe Lys Asp Arg Ile Phe Trp Arg Arg Gln
               20                  25                  30
   Val His Leu Arg Thr Gly Ile Arg Pro Ser Thr Ile Thr Ser Ser Phe
           35                  40                  45
   Pro Gln Leu Met Ser Asn Val Asp Ala Ala Tyr Glu Val Ala Glu Arg
       50                  55                  60
   Gly Thr Ala Tyr Phe Phe Lys Gly Pro His Tyr Trp Ile Thr Arg Gly
   65                  70                  75                  80
   Phe Gln Met Gln Gly Pro Pro Arg Thr Ile Tyr Asp Phe Gly Phe Pro
                   85                  90                  95
   Arg His Val Gln Gln Ile Asp Ala Ala Val Tyr Leu Arg Glu Pro Gln
               100                 105                 110
   Lys Thr Leu Phe Phe Val Gly Asp Glu Tyr Tyr Ser Tyr Asp Glu Arg
           115                 120                 125
   Lys Arg Lys Met Glu Lys Asp Tyr Pro Lys Asn Thr Glu Glu Glu Phe
       130                 135                 140
   Ser Gly Val Asn Gly Gln Ile Asp Ala Ala Val Glu Leu Asn Gly Tyr
   145                 150                 155                 160
   Ile Tyr Phe Phe Ser Gly Pro Lys Thr Tyr Lys Tyr Asp Thr Glu Lys
                   165                 170                 175
   Glu Asp Val Val Ser Val Val Lys Ser Ser Ser Trp Ile Gly Cys
               180                 185                 190
<210> 51
   <211> 191
   <212> PRT
   <213> Mus musculus
<400> 51
   Pro Asp Leu Cys Asp Ser Ser Ser Ser Phe Asp Ala Val Thr Met Leu
   1               5                   10                  15
   Gly Lys Glu Leu Leu Phe Phe Lys Asp Arg Ile Phe Trp Arg Arg Gln
               20                  25                  30
   Val His Leu Pro Thr Gly Ile Arg Pro Ser Thr Ile Thr Ser Ser Phe
           35                  40                  45
   Pro Gln Leu Met Ser Asn Val Asp Ala Ala Tyr Glu Val Ala Glu Arg
       50                  55                  60
   Gly Ile Ala Phe Phe Phe Lys Gly Pro His Tyr Trp Val Thr Arg Gly
   65                  70                  75                  80
   Phe His Met Gln Gly Pro Pro Arg Thr Ile Tyr Asp Phe Gly Phe Pro
                   85                  90                  95
   Arg His Val Gln Arg Ile Asp Ala Ala Val Tyr Leu Lys Glu Pro Gln
               100                 105                 110
   Lys Thr Leu Phe Phe Val Gly Glu Glu Tyr Tyr Ser Tyr Asp Glu Arg
           115                 120                 125
   Lys Lys Lys Met Glu Lys Asp Tyr Pro Lys Asn Thr Glu Glu Glu Phe
       130                 135                 140
   Ser Gly Val Ser Gly His Ile Asp Ala Ala Val Glu Leu Asn Gly Tyr
   145                 150                 155                 160
   Ile Tyr Phe Phe Ser Gly Arg Lys Thr Phe Lys Tyr Asp Thr Glu Lys
                   165                 170                 175
   Glu Asp Val Val Ser Val Val Lys Ser Ser Ser Trp Ile Gly Cys
               180                 185                 190
<210> 52
   <211> 191
   <212> PRT
   <213> Sus scrofa
<400> 52
   Pro Asp Ile Cys Asp Ser Ser Ser Ser Phe Asp Ala Val Thr Met Leu
   1               5                   10                  15
   Gly Lys Glu Leu Leu Phe Phe Arg Asp Arg Ile Phe Trp Arg Arg Gln
   20 25 30
   Val His Leu Met Ser Gly Ile Arg Pro Ser Thr Ile Thr Ser Ser Phe
   35 40 45
   Pro Gln Leu Met Ser Asn Val Asp Ala Ala Tyr Glu Val Ala Asp Arg
   50 55 60
   Gly Met Ala Tyr Phe Phe Lys Gly Pro His Tyr Trp Ile Thr Arg Gly
   65 70 75 80
   Phe Gln Met Gln Gly Pro Pro Arg Thr Ile Tyr Asp Phe Gly Phe Pro
   85 90 95
   Arg Tyr Val Gln Arg Ile Asp Ala Ala Val His Leu Lys Asp Thr Gln
   100 105 110
   Lys Thr Leu Phe Phe Val Gly Asp Glu Tyr Tyr Ser Tyr Asp Glu Arg
   115 120 125
   Lys Arg Lys Met Asp Lys Asp Tyr Pro Lys Asn Thr Glu Glu Glu Phe
   130 135 140
   Ser Gly Val Asn Gly Gln Ile Asp Ala Ala Val Glu Leu Asn Gly Tyr
   145 150 155 160
   Ile Tyr Phe Phe Ser Gly Pro Lys Ala Tyr Lys Tyr Asp Thr Glu Lys
   165 170 175
   Glu Asp Val Val Ser Val Leu Lys Ser Asn Ser Trp Ile Gly Cys
   180 185 190
<210> 53
   <211> 196
   <212> PRT
   <213> Homo sapiens
<400> 53
   Pro Asn Ile Cys Asp Gly Asn Phe Asn Thr Val Ala Leu Phe Arg Gly
   1 5 10 15
   Glu Met Phe Val Phe Lys Asp Arg Trp Phe Trp Arg Leu Arg Asn Asn
   20 25 30
   Arg Val Gln Glu Gly Tyr Pro Met Gln Ile Glu Gln Phe Trp Lys Gly
   35 40 45
   Leu Pro Ala Arg Ile Asp Ala Ala Tyr Glu Arg Ala Asp Gly Arg Phe
   50 55 60
   Val Phe Phe Lys Gly Asp Lys Tyr Trp Val Phe Lys Glu Val Thr Val
   65 70 75 80
   Glu Pro Gly Tyr Pro His Ser Leu Gly Glu Leu Gly Ser Cys Leu Pro
   85 90 95
   Arg Glu Gly Ile Asp Thr Ala Leu Arg Trp Glu Pro Val Gly Lys Thr
   100 105 110
   Tyr Phe Phe Lys Gly Glu Arg Tyr Trp Arg Tyr Ser Glu Glu Arg Arg
   115 120 125
   Ala Thr Asp Pro Gly Tyr Pro Lys Pro Ile Thr Val Trp Lys Gly Ile
   130 135 140
   Pro Gln Ala Pro Gln Gly Ala Phe Ile Ser Lys Glu Gly Tyr Tyr Thr
   145 150 155 160
   Tyr Phe Tyr Lys Gly Arg Asp Tyr Trp Lys Phe Asp Asn Gln Lys Leu
   165 170 175
   Ser Val Glu Pro Gly Tyr Pro Arg Asn Ile Leu Arg Asp Trp Met Gly
   180 185 190
   Cys Asn Gln Lys
   195
<210> 54
   <211> 196
   <212> PRT
   <213> Mus musculus
<400> 54
   Pro Asn Ile Cys Asp Gly Asn Phe Asn Thr Val Ala Leu Phe Arg Gly
   1 5 10 15
   Glu Met Phe Val Phe Lys Asp Arg Trp Phe Trp Arg Leu Arg Asn Asn
   20 25 30
   Arg Val Gln Glu Gly Tyr Pro Met Gln Ile Glu Gln Phe Trp Lys Gly
       35                  40                  45
   Leu Pro Ala Arg Ile Asp Ala Ala Tyr Glu Arg Ala Asp Gly Arg Phe
       50                  55                  60
   Val Phe Phe Lys Gly Asp Lys Tyr Trp Val Phe Lys Glu Val Thr Val
   65                  70                  75                  80
   Glu Pro Gly Tyr Pro His Ser Leu Gly Glu Leu Gly Ser Cys Leu Pro
                   85                  90                  95
   Arg Glu Gly Ile Asp Thr Ala Leu Arg Trp Glu Pro Val Gly Lys Thr
               100                 105                 110
   Tyr Phe Phe Lys Gly Glu Arg Tyr Trp Arg Tyr Ser Glu Glu Arg Arg
           115                 120                 125
   Ala Thr Asp Pro Gly Tyr Pro Lys Pro Ile Thr Val Trp Lys Gly Ile
       130                 135                 140
   Pro Gln Ala Pro Gln Gly Ala Phe Ile Ser Lys Glu Gly Tyr Tyr Thr
   145                 150                 155                 160
   Tyr Phe Tyr Lys Gly Arg Asp Tyr Trp Lys Phe Asp Asn Gln Lys Leu
                   165                 170                 175
   Ser Val Glu Pro Gly Tyr Pro Arg Asn Ile Leu Arg Asp Trp Met Gly
               180                 185                 190
   Cys Lys Gln Lys
           195
<210> 55
   <211> 196
   <212> PRT
   <213> Rattus norvegicus
<400> 55
   Pro Asn Ile Cys Asp Gly Asn Phe Asn Thr Val Ala Leu Phe Arg Gly
   1               5                   10                  15
   Glu Met Phe Val Phe Lys Asp Arg Trp Phe Trp Arg Leu Arg Asn Asn
   20 25 30
   Arg Val Gln Glu Gly Tyr Pro Met Gln Ile Glu Gln Phe Trp Lys Gly
   35 40 45
   Leu Pro Ala Arg Ile Asp Ala Ala Tyr Glu Arg Ala Asp Gly Arg Phe
   50 55 60
   Val Phe Phe Lys Gly Asp Lys Tyr Trp Val Phe Lys Glu Val Thr Val
   65 70 75 80
   Glu Pro Gly Tyr Pro His Ser Leu Gly Glu Leu Gly Ser Cys Leu Pro
   85 90 95
   Arg Glu Gly Ile Asp Thr Ala Leu Arg Trp Glu Pro Val Gly Lys Thr
   100 105 110
   Tyr Phe Phe Lys Gly Glu Arg Tyr Trp Arg Tyr Ser Glu Glu Arg Arg
   115 120 125
   Ala Thr Asp Pro Gly Tyr Pro Lys Pro Ile Thr Val Trp Lys Gly Ile
   130 135 140
   Pro Gln Ala Pro Gln Gly Ala Phe Ile Ser Lys Glu Gly Tyr Tyr Thr
   145 150 155 160
   Tyr Phe Tyr Lys Gly Arg Asp Tyr Trp Lys Phe Asp Asn Gln Lys Leu
   165 170 175
   Ser Val Glu Pro Gly Tyr Pro Arg Asn Ile Leu Arg Asp Trp Met Gly
   180 185 190
   Cys Lys Gln Lys
   195
<210> 56
   <211> 198
   <212> PRT
   <213> Homo sapiens
<400> 56
   Pro Asp Arg Cys Glu Gly Asn Phe Asp Ala Ile Ala Asn Ile Arg Gly
   1 5 10 15
   Glu Thr Phe Phe Phe Lys Gly Pro Trp Phe Trp Arg Leu Gln Pro Ser
   20 25 30
   Gly Gln Leu Val Ser Pro Arg Pro Ala Arg Leu His Arg Phe Trp Glu
   35 40 45
   Gly Leu Pro Ala Gln Val Arg Val Val Gln Ala Ala Tyr Ala Arg His
   50 55 60
   Arg Asp Gly Arg Ile Leu Leu Phe Ser Gly Pro Gln Phe Trp Val Phe
   65 70 75 80
   Gln Asp Arg Gln Leu Glu Gly Gly Ala Arg Pro Leu Thr Glu Leu Gly
   85 90 95
   Leu Pro Pro Gly Glu Glu Val Asp Ala Val Phe Ser Trp Pro Gln Asn
   100 105 110
   Gly Lys Thr Tyr Leu Val Arg Gly Arg Gln Tyr Trp Arg Tyr Asp Glu
   115 120 125
   Ala Ala Ala Arg Pro Asp Pro Gly Tyr Pro Arg Asp Leu Ser Leu Trp
   130 135 140
   Glu Gly Ala Pro Pro Ser Pro Asp Asp Val Thr Val Ser Asn Ala Gly
   145 150 155 160
   Asp Thr Tyr Phe Phe Lys Gly Ala His Tyr Trp Arg Phe Pro Lys Asn
   165 170 175
   Ser Ile Lys Thr Glu Pro Asp Ala Pro Gln Pro Met Gly Pro Asn Trp
   180 185 190
   Leu Asp Cys Pro Ala Pro
   195
<210> 57
   <211> 193
   <212> PRT
   <213> Homo sapiens
<400> 57
   Phe Asp Ala Ile Thr Val Asp Arg Gln Gln Gln Leu Tyr Ile Phe Lys
   1 5 10 15
   Gly Ser His Phe Trp Glu Val Ala Ala Asp Gly Asn Val Ser Glu Pro
   20 25 30
   Arg Pro Leu Gln Glu Arg Trp Val Gly Leu Pro Pro Asn Ile Glu Ala
   35 40 45
   Ala Ala Val Ser Leu Asn Asp Gly Asp Phe Tyr Phe Phe Lys Gly Gly
   50 55 60
   Arg Cys Trp Arg Phe Arg Gly Pro Lys Pro Val Trp Gly Leu Pro Gln
   65 70 75 80
   Leu Cys Arg Ala Gly Gly Leu Pro Arg His Pro Asp Ala Ala Leu Phe
   85 90 95
   Phe Pro Pro Leu Arg Arg Leu Ile Leu Phe Lys Gly Ala Arg Tyr Tyr
   100 105 110
   Val Leu Ala Arg Gly Gly Leu Gln Val Glu Pro Tyr Tyr Pro Arg Ser
   115 120 125
   Leu Gln Asp Trp Gly Gly Ile Pro Glu Glu Val Ser Gly Ala Leu Pro
   130 135 140
   Arg Pro Asp Gly Ser Ile Ile Phe Phe Arg Asp Asp Arg Tyr Trp Arg
   145 150 155 160
   Leu Asp Gln Ala Lys Leu Gln Ala Thr Thr Ser Gly Arg Trp Ala Thr
   165 170 175
   Glu Leu Pro Trp Met Gly Cys Trp His Ala Asn Ser Gly Ser Ala Leu
   180 185 190
   Phe
<210> 58
   <211> 191
   <212> PRT
   <213> Homo sapiens
<400> 58
   Pro Ser Gln Glu Glu Cys Glu Gly Ser Ser Leu Ser Ala Val Phe Glu
   1 5 10 15
   His Phe Ala Met Met Gln Arg Asp Ser Trp Glu Asp Ile Phe Glu Leu
   20 25 30
   Leu Phe Trp Gly Arg Thr Ser Ala Gly Thr Arg Gln Pro Gln Phe Ile
   35 40 45
   Ser Arg Asp Trp His Gly Val Pro Gly Gln Val Asp Ala Ala Met Ala
   50 55 60
   Gly Arg Ile Tyr Ile Ser Gly Met Ala Pro Arg Pro Ser Leu Ala Lys
   65 70 75 80
   Lys Gln Arg Phe Arg His Arg Asn Arg Lys Gly Tyr Arg Ser Gln Arg
   85 90 95
   Gly His Ser Arg Gly Arg Asn Gln Asn Ser Arg Arg Pro Ser Arg Ala
   100 105 110
   Thr Trp Leu Ser Leu Phe Ser Ser Glu Glu Ser Asn Leu Gly Ala Asn
   115 120 125
   Asn Tyr Asp Asp Tyr Arg Met Asp Trp Leu Val Pro Ala Thr Cys Glu
   130 135 140
   Pro Ile Gln Ser Val Phe Phe Phe Ser Gly Asp Lys Tyr Tyr Arg Val
   145 150 155 160
   Asn Leu Arg Thr Arg Arg Val Asp Thr Val Asp Pro Pro Tyr Pro Arg
   165 170 175
   Ser Ile Ala Gln Tyr Trp Leu Gly Cys Pro Ala Pro Gly His Leu
   180 185 190
<210> 59
   <211> 192
   <212> PRT
   <213> Mus musculus
<400> 59
   Pro Ser Gln Glu Glu Cys Glu Gly Ser Ser Leu Ser Ala Val Phe Glu
   1 5 10 15
   His Phe Ala Leu Leu Gln Arg Asp Ser Trp Glu Asn Ile Phe Glu Leu
               20                  25                  30
   Leu Phe Trp Gly Arg Ser Ser Asp Gly Ala Arg Glu Pro Gln Phe Ile
           35                  40                  45
   Ser Arg Asn Trp His Gly Val Pro Gly Lys Val Asp Ala Ala Met Ala
       50                  55                  60
   Gly Arg Ile Tyr Val Thr Gly Ser Leu Ser His Ser Ala Gln Ala Lys
   65                  70                  75                  80
   Lys Gln Lys Ser Lys Arg Arg Ser Arg Lys Arg Tyr Arg Ser Arg Arg
                   85                  90                  95
   Gly Arg Gly His Arg Arg Ser Gln Ser Ser Asn Ser Arg Arg Ser Ser
               100                 105                 110
   Arg Ser Ile Trp Phe Ser Leu Phe Ser Ser Glu Glu Ser Gly Leu Gly
           115                 120                 125
   Thr Tyr Asn Asn Tyr Asp Tyr Asp Met Asp Trp Leu Val Pro Ala Thr
       130                 135                 140
   Cys Glu Pro Ile Gln Ser Val Tyr Phe Phe Ser Gly Asp Lys Tyr Tyr
   145                 150                 155                 160
   Arg Val Asn Leu Arg Thr Arg Arg Val Asp Ser Val Asn Pro Pro Tyr
                   165                 170                 175
   Pro Arg Ser Ile Ala Gln Tyr Trp Leu Gly Cys Pro Thr Ser Glu Lys
               180                 185                 190
<210> 60
   <211> 195
   <212> PRT
   <213> Sus scrofa
<400> 60
   Pro Ser Arg Glu Glu Cys Glu Gly Ser Ser Pro Ser Asp Val Phe Ala
   1               5                   10                  15
   His Phe Ala Leu Met Gln Arg Asp Ser Trp Glu Asp Ile Phe Arg Leu
               20                  25                  30
   Leu Phe Trp Ser His Ser Phe Gly Gly Ala Ile Glu Pro Arg Val Ile
   35 40 45
   Ser Gln Asp Trp Leu Gly Leu Pro Glu Gln Val Asp Ala Ala Met Ala
   50 55 60
   Gly Gln Ile Tyr Ile Ser Gly Ser Ala Leu Lys Pro Ser Gln Pro Lys
   65 70 75 80
   Met Thr Lys Ser Ala Arg Arg Ser Gly Lys Arg Tyr Arg Ser Arg Arg
   85 90 95
   Gly Arg Gly Arg Gly Arg Gly His Ser Arg Ser Gln Lys Ser His Arg
   100 105 110
   Gln Ser Arg Ser Thr Trp Leu Pro Trp Phe Ser Ser Glu Glu Thr Gly
   115 120 125
   Pro Gly Gly Tyr Asn Tyr Asp Asp Tyr Lys Met Asp Trp Leu Val Pro
   130 135 140
   Ala Thr Cys Glu Pro Ile Gln Ser Val Tyr Phe Phe Ser Gly Glu Glu
   145 150 155 160
   Tyr Tyr Arg Val Asn Leu Arg Thr Gln Arg Val Asp Thr Val Thr Pro
   165 170 175
   Pro Tyr Pro Arg Ser Ile Ala Gln Tyr Trp Leu Gly Cys Pro Val Pro
   180 185 190
   Asp Gln Lys
   195
<210> 61
   <211> 188
   <212> PRT
   <213> Oryctolagus cuniculus
<400> 61
   Pro Ser Gln Glu Glu Cys Glu Gly Ser Ser Leu Ser Ala Val Phe Glu
   1 5 10 15
   His Phe Ala Met Leu His Arg Asp Ser Trp Glu Asp Ile Phe Lys Leu
                   20                  25                  30
   Leu Phe Trp Gly Arg Pro Ser Gly Gly Ala Arg Gln Pro Gln Phe Ile
           35                  40                  45
   Ser Arg Asp Trp His Gly Val Pro Gly Lys Val Asp Ala Ala Met Ala
       50                  55                  60
   Gly Arg Ile Tyr Ile Ser Gly Leu Thr Pro Ser Pro Ser Ala Lys Lys
   65                  70                  75                  80
   Gln Lys Ser Arg Arg Arg Ser Arg Lys Arg Tyr Arg Ser Arg Tyr Gly
                   85                  90                  95
   Arg Gly Arg Ser Gln Asn Ser Arg Arg Leu Ser Arg Ser Ile Ser Arg
               100                 105                 110
   Leu Trp Phe Ser Ser Glu Glu Val Ser Leu Gly Pro Tyr Asn Tyr Glu
           115                 120                 125
   Asp Tyr Glu Thr Ser Trp Leu Lys Pro Ala Thr Ser Glu Pro Ile Gln
       130                 135                 140
   Ser Val Tyr Phe Phe Ser Gly Asp Lys Tyr Tyr Arg Val Asn Leu Arg
   145                 150                 155                 160
   Thr Gln Arg Val Asp Thr Val Asn Pro Pro Tyr Pro Arg Ser Ile Ala
                   165                 170                 175
   Gln Tyr Trp Leu Gly Cys Pro Ala Pro Gly Gly Gln
               180                 185
<210> 62
   <211> 345
   <212> DNA
   <213> Artificial
<220>
   <223> Ribosome Display Spacer
<400> 62
   gctggctctg gagctggtgc aggctctggt gctggcgcag gttctggcgc tggtgctggt 60
   tctggcactg gtgcttctcc ggcagctgtt ccggcagcgg ttccagcagc ggtgccggca 120
   gcagttcctg ctgcggtggg cgaaggagaa ggagaaggcg agggagaggg cgaaggatac 180
   ccgtacgacg taccggacta cgccgaaggt ggtggtggct ccgagcagaa gctcatctcc 240
   gaagaagacc tggagggtgg tggtggctcc acagactaca aggacgacga cgacaaatcc 300
   caaatcaaac gaaaggccca gtcgaaaaac tgggcctttc gattt 345
<210> 63
   <211> 22
   <212> DNA
   <213> Artificial
<220>
   <223> PEX2forw
<400> 63
   atgcctgaaa tctgcaaaca gg 22
<210> 64
   <211> 54
   <212> DNA
   <213> Artificial
<220>
   <223> PEXR4
<220>
   <221> misc feature
   <222> (25)..(26)
   <223> n is a, c, g, or t
<220>
   <221> misc feature
   <222> (28)..(29)
   <223> n is a, c, g, or t
<400> 64
   cctgcaaaga acacagcttt ctcmnnmnnt ggggcctcgt ataccgcatc aatc 54
<210> 65
   <211> 42
   <212> DNA
   <213> Artificial
<220>
   <223> PEXF4
<400> 65
   ggaccctggc ttccccaagc tcatcgcaga tgcctggaat gc 42
<210> 66
   <211> 19
   <212> DNA
   <213> Artificial
<220>
   <223> PEX2rev
<400> 66
   ggagctcgct cgagtcagc 19
<210> 67
   <211> 60
   <212> DNA
   <213> Artificial
<220>
   <223> PEXF1
<400> 67
   gagaaagctg tgttctttgc agggaatgaa tactggatct actcagcgag caccttggag 60
<210> 68
   <211> 60
   <212> DNA
   <213> Artificial
<220>
   <223> PEXR1
<220>
   <221> misc feature
   <222> (29)..(30)
   <223> n is a, c, g, or t
<220>
   <221> misc feature
   <222> (35)..(36)
   <223> n is a, c, g, or t
<400> 68
   gagcttgggg aagccagggt ccattttmnn cttmnnctcg ttgtacctcc agaacttgtc 60
<210> 69
   <211> 57
   <212> DNA
   <213> Artificial
<220>
   <223> PEXR3
<220>
   <221> misc feature
   <222> (37)..(38)
   <223> n is a, c, g, or t
<400> 69
   ggcagtccga ggctagtcag tggtttggga taaccmnnct ccaaggtgct cgctgag 57
<210> 70
   <211> 52
   <212> DNA
   <213> Artificial
<220>
   <223> PEXR2
<220>
   <221> misc feature
   <222> (23)..(24)
   <223> n is a, c, g, or t
<220>
   <221> misc_feature
   <222> (26)..(27)
   <223> n is a, c, g, or t
<220>
   <221> misc feature
   <222> (29)..(30)
   <223> n is a, c, g, or t
<400> 70
   gccagcgaag atgtatgtct tmnnmnnmnn gctccagtta aaggctgcat cc 52
<210> 71
   <211> 60
   <212> DNA
   <213> Artificial
<220>
   <223> PEXF2
<400> 71
   ctgactagcc tcggactgcc ccctgatgtt caacgtgtgg atgcagcctt taactggagc 60
<210> 72
   <211> 55
   <212> DNA
   <213> Artificial
<220>
   <223> T7P_PEX2
<400> 72
   gtttaacttt aagaaggaga tatacatatg cctgaaatct gcaaacagga tatcg 55
<210> 73
   <211> 49
   <212> DNA
   <213> Artificial
<220>
   <223> PEX2_RD
<400> 73
   cctgcaccag ctccagagcc agcgcagcct agccagtcgg atttgatgc 49
<210> 74
   <211> 186
   <212> DNA
   <213> Artificial
<220>
   <223> T7Pg10epsilon
<400> 74
   ggtgatgtcg gcgatatagg cgccagcaac cgcacctgtg gcgccggtga tgccggccac      60
   gatgcgtccg gcgtagagga tcgagatctc gatcccgcga aattaatacg actcactata     120
   gggagaccac aacggtttcc ctctagaaat aattttgttt aactttaaga aggagatata     180
   catatg                                                                186
<210> 75
   <211> 27
   <212> DNA
   <213> Artificial
<220>
   <223> T7Pfor
<400> 75
   ggtgatgtcg gcgatatagg cgccagc 27
<210> 76
   <211> 23
   <212> DNA
   <213> Artificial
<220>
   <223> R1A
<400> 76
   aaatcgaaag gcccagtttt tcg 23
<210> 77
   <211> 55
   <212> DNA
   <213> Artificial
<220>
   <223> bridging primer
<400> 77
   gtttaacttt aagaaggaga tatacatatg cctgaaatct gcaaacagga tatcg 55
<210> 78
   <211> 50
   <212> DNA
   <213> Artificial
<220>
   <223> bridging primer
<400> 78
   gtcgttcaga ccacgaccct cgatgcagcc tagccagtcg gatttgatgc 50
<210> 79
   <211> 243
   <212> DNA
   <213> Artificial
<220>
   <223> T7PAviTagFXa
<400> 79
   ggtgatgtcg gcgatatagg cgccagcaac cgcacctgtg gcgccggtga tgccggccac     60
   gatgcgtccg gcgtagagga tcgagatctc gatcccgcga aattaatacg actcactata    120
   gggagaccac aacggtttcc ctctagaaat aattttgttt aactttaaga aggagatata    180
   catatgggtc ttaatgatat ttttgaagct cagaaaatcg aatggcacga aatcgagggt    240
   cgt                                                                  243
<210> 80
   <211> 332
   <212> DNA
   <213> Artificial
<220>
   <223> T7T
<400> 80
   taggaagctc agaaaatcga atggcacgaa taatgagctc ccgggagcgc ttggagccac     60
   ccgcagttcg aaaaataata agggcctccc actgactgct cttctgtcag tgggctactc    120
   ctggactcgg caccagattg cctcattttt ctcctctggc attttgtata aatccacctt    180
   gactggggaa attctcctgg ggtcaggtgg caccagcctg gatccggctg ctaacaaagc    240
   ccgaaaggaa gctgagttgg ctgctgccac cgctgagcaa taactagcat aaccccttgg    300
   ggcctctaaa cgggtcttga ggggtttttt gc                                  332
<210> 81
   <211> 30
   <212> DNA
   <213> Artificial
<220>
   <223> T7Trev
<400> 81
   gcaaaaaacc cctcaagacc cgtttagagg 30
<210> 82
   <211> 22
   <212> DNA
   <213> Artificial
<220>
   <223> T7Pfor
<400> 82
   ggtgatgtcg gcgatatagg cg 22
<210> 83
   <211> 37
   <212> DNA
   <213> Artificial
<220>
   <223> NdeI-PEX2for
<400> 83
   ctgagagtgc accatatgcc tgaaatctgc aaacagg 37
<210> 84
   <211> 33
   <212> DNA
   <213> Artificial
<220>
   <223> EcoRI-PEX2rev
<400> 84
   ccatgattac gaattcgcag cctagccagt cgg 33
<210> 85
   <211> 38
   <212> DNA
   <213> Artificial
<220>
   <223> anti ssrA-oligonucleotide
<400> 85
   ttaagctgct aaagcgtagt tttcgtcgtt tgcgacta 38
<210> 86
   <211> 28
   <212> DNA
   <213> Artificial
<220>
   <223> primer RT
<400> 86
   cagagcctgc accagctcca gagccagc 28
<210> 87
   <211> 30
   <212> DNA
   <213> Artificial
<220>
   <223> primer F1
<400> 87
   gtttaacttt aagaaggaga tatacatatg 30
<210> 88
   <211> 210
   <212> PRT
   <213> Artificial
<220>
   <223> consensus sequence - Xaa denotes a hyphen (-)
<220>
   <221> misc feature
   <222> (20)..(20)
   <223> Xaa can be any naturally occurring amino acid
<220>
   <221> misc feature
   <222> (34)..(34)
   <223> Xaa can be any naturally occurring amino acid
<220>
   <221> misc feature
   <222> (55)..(57)
   <223> Xaa can be any naturally occurring amino acid
<220>
   <221> misc_feature
   <222> (97)..(97)
   <223> Xaa can be any naturally occurring amino acid
<220>
   <221> misc_feature
   <222> (119)..(120)
   <223> Xaa can be any naturally occurring amino acid
<220>
   <221> misc_feature
   <222> (189)..(195)
   <223> Xaa can be any naturally occurring amino acid
<400> 88
   Pro Glu Ile Cys Lys Gln Asp Ile Val Phe Asp Gly Ile Ala Gln Ile
   1               5                   10                  15
   Arg Gly Glu Xaa Ile Phe Phe Phe Lys Asp Arg Phe Ile Trp Arg Thr
               20                  25                  30
   Val Xaa Thr Pro Arg Asp Lys Pro Met Gly Pro Leu Leu Val Ala Thr
           35                  40                  45
   Phe Trp Pro Glu Leu Pro Xaa Xaa Xaa Glu Lys Ile Asp Ala Val Tyr
       50                  55                  60
   Glu Ala Pro Gln Glu Glu Lys Ala Val Phe Phe Ala Gly Asn Glu Tyr
   65                  70                  75                  80
   Trp Ile Tyr Ser Ala Ser Thr Leu Glu Arg Gly Tyr Pro Lys Pro Leu
                   85                  90                  95
   Xaa Thr Ser Leu Gly Leu Pro Pro Asp Val Gln Arg Val Asp Ala Ala
               100                 105                 110
   Phe Asn Trp Ser Lys Asn Xaa Xaa Lys Lys Thr Tyr Ile Phe Ala Gly
           115                 120                 125
   Asp Lys Phe Trp Arg Tyr Asn Glu Val Lys Lys Lys Met Asp Pro Gly
       130                 135                 140
   Phe Pro Lys Leu Ile Ala Asp Ala Trp Asn Ala Ile Pro Asp Asn Leu
   145                 150                 155                 160
   Asp Ala Val Val Asp Leu Gln Gly Gly Gly His Ser Tyr Phe Phe Lys
                   165                 170                 175
   Gly Ala Tyr Tyr Leu Lys Leu Glu Asn Gln Ser Leu Xaa Xaa Xaa Xaa
               180                 185                 190
   Xaa Xaa Xaa Lys Ser Val Lys Phe Gly Ser Ile Lys Ser Asp Trp Leu
           195                 200                 205
   Gly Cys
       210

## Description

The invention concerns a method for identifying a nucleic acid encoding a polypeptide with specific binding properties to a predetermined target molecule which are not naturally inherent to that polypeptide. At the same time a process for the optimization of the binding specifity and a process of production are described. The invention further discloses a method for the identification and modification of alterable amino acid positions within a polypeptide scaffold.

### Technological Background

In recent years the number of applications and publications related to affinity reagents steadily increased. The majority thereof is related to antibodies, i.e. monoclonal or polyclonal immunoglobulines. Only a minor part deals with possible alternatives. One of these is the use of protein scaffolds. This concept requires a stable protein architecture tolerating multiple substitutions or insertions at the primary structural level (Nygren, P-A., Skerra, A., J. Immun. Meth. 290 (2004) 3-28).

Modified protein scaffolds can overcome existing problems and extent the application area of affinity reagents. Only one problem among many others is the intracellular application of antibodies. The bottleneck of this protein knockout technology is that not all antibodies expressed within cells perform well. This is called the "disulphide bond problem". To overcome this problem time-consuming experiments have to be performed to optimize a complex list of parameters (Visintin, M., et al., J. Immun. Meth. 290 (2004) 135-153).

In this regard proteins possess several advantages. Among these are low molecular weight, ease of production by microorganisms, simplicity to modify and broad applicability. Different protein scaffolds have been described in this context, e.g. Zinc-finger proteins for DNA recognition (Segal, D.J., et al., Biochem. 42 (2003) 2137-2148); Thioredoxin based peptide aptamers modified by introduction of variable polypeptide sequences in the active-site loop (Klevenz, B., et al., Cell. Mol. Life Sci. 59 (2002) 1993-1998); Protein A as "Affibody" scaffold (Sandström) K., et al., Prot. Eng. 16 (2003) 691-697; Andersson, M., et al., J. Immun. Meth. 283 (2003) 225-234); mRNA-protein molecules of the tenth fibronectin type III domain (Xu, L., et al., Chem. Biol. 9 (2002) 933-942) or alpha-Amylase inhibitor based binding molecules (McConnell, S.J., and Hoess, R.H., J. Mol. Biol. 250 (1995) 460-470).

Two criteria substantially characterize an applicable protein-scaffold: First, the protein should belong to a family which reveals a well defined hydrophobic core. A close relationship between the individual family members is beneficial (Skerra, A., J. Mol. Recognit. 13 (2000) 167-187). Second, the protein should possess a spatially separated and functionally independent accessible active site or binding pocket. This should not contribute to the intrinsic core-stability (Predki, P.F., et al., Nature Struct. Biol. 3 (1996) 54-58). Ideally, this protein-family is inherently involved in the recognition of multiple, non-related targets.

As described in Nygren, P-A., and Skerra, A., J. Immun. Meth. 290 (2004) 3-28 several polypeptide scaffolds have been employed for the development of novel affinity proteins. These scaffolds can be divided into three groups: (i) single peptide loops, (ii) engineered interfaces and (iii) non-contiguous hyper variable loops.

With the scaffolds of the first group either single amino acids in an exposed loop are diversified or small polypeptide sequences are inserted into this exposed loop (see e.g. Roberts, B.L., et al., PNAS 89 (1992) 2429-2433 and Gene 121 (1992) 9-16; Röttgen) P., and Collins, J., Gene 164 (1995) 243; Lu, Z., et al., Bio/Technology 13 (1995) 366-372). One drawback of this approach is, that affinity, if any, to a completely novel target is difficult to achieve (Klevenz, B., et al., Cell. Mol. Life Sci. 59 (2002) 1993-1998). The intrinsic binding affinity to the natural or closely related targets can be modified, but the target or the target class can hardly be changed. Another drawback is that in the case of insertion of small randomized polypeptides, the target has to be known and these sequences have to be generated beforehand based on already established knowledge.

To the scaffolds of the second group belong e.g. the immunoglobulin binding domain of Staphylococcal protein A (e.g. Sandström) K., et al., Prot. Eng. 16 (2003) 691-697), the C-terminal cellulose-binding domain of cellobiohydrolase I of the fungus T. reesei (Smith, G.P., et al., J. Mol. Biol. 277 (1998) 317-322) and the gamma-crystallines (Fiedler, U., and Rudolph, R., WO 01/04144).

The third class is represented by the immunoglobulin itself and the distantly related fibronectin type III domain as well as some classes of neurotoxins.

Beside the suitability as scaffold for the generation of specific binding characteristics to predetermined target molecules, the application conditions have to be considered. Among other things especially the stability, selectivity, solubility and functional production of the affinity polypeptide have to be taken into account. As an example, already mentioned above, the bottleneck of the protein knockout technology is that not all antibodies expressed as affinity molecules within cells are functionally produced ("disulphide bond problem", Visintin, M., et al., J. Immun. Meth. 290 (2004) 135-153).

Therefore it is the objective of the current invention to overcome these drawbacks by providing an alternative polypeptide scaffold with specific binding properties to a predetermined target molecule which are not naturally inherent to that polypeptide. This comprises the randomization of amino acids, the optimization of the binding characteristics and a method of production of the optimized polypeptide with specific binding properties.

WO 02/088171 discloses methods for identifying binding domains with a desired property by screening combinational libraries.

### Summary of the invention

The predetermined target molecule comprises a member of one of the groups consisting of hedgehog proteins, bone morphogenetic proteins, growth factors, erythropoietin, thrombopoietin, G-CSF, interleukins and interferons.

The invention provides a method for identifying a nucleic acid encoding a polypeptide which specifically binds a target molecule from a DNA-library, wherein the method comprises the steps of
a) selecting the sequence of SEQ ID NO:02;
b) preparing a DNA-library of the selected sequence in which at least one amino acid position according to table V is altered;
c) screening the prepared DNA-library for encoded polypeptides specifically binding a predetermined target molecule;
d) choosing the nucleic acid encoding one specific binder identified in step c);
e) repeating the steps b) to d) for two to five times; and
f) isolating said nucleic acid encoding a polypeptide specifically binding a predetermined target molecule.

In another embodiment the method for identifying a nucleic acid encoding a polypeptide which specifically binds a target molecule from a DNA-library, comprises linear expression elements.

In another embodiment the library of the polypeptide is expressed by display on ribosomes.

In another embodiment the library of the polypeptide is expressed by display on bacteriophages.

### Detailed description of the invention

The present invention provides a method for identifying a nucleic acid encoding a polypeptide which specifically binds a predetermined target molecule from a DNA-library as defined in claim 1 and discloses a method for the determination of alterable amino acid positions in a polypeptide.

The polypeptide can be defined by its amino acid sequence and by the DNA sequence derived there from.

The use of recombinant DNA technology enables the production of numerous derivatives of the polypeptide. Such derivatives can, for example, be modified in individual or several amino acid positions by substitution, alteration or exchange. The derivatisation can, for example, be carried out by means of site directed mutagenesis. Such variations can easily be carried out by a person skilled in the art (Sambrook, J., et al., Molecular Cloning: A laboratory manual (1999) Cold Spring Harbor Laboratory Press, New York, USA; Hames, B.D., and Higgins, S.G., Nucleic acid hybridization - a practical approach (1985) IRL Press, Oxford, England).

For expression in eukaryotic or prokaryotic host cells, the nucleic acid, encoding the amino acid sequence of the polypeptide, is integrated into suitable expression vectors, according to methods familiar to a person skilled in the art. Such an expression vector preferably contains a regulable or inducible promoter. These recombinant vectors are then introduced for expression into suitable host cells such as, e.g., E.coli as a prokaryotic host cell or Saccharomyces cerevisiae, insect cells or CHO cells as eukaryotic host cells and the transformed or transduced host cells are cultured under conditions which allow expression of the heterologous gene.

The polypeptide can be isolated and purified after recombinant production by methods known to a person skilled in the art, e.g. by affinity chromatography using known protein purification techniques, including immunoprecipitation, gel filtration, ion exchange chromatography, chromatofocussing, isoelectric focusing, selective precipitation, electrophoresis, or the like (see e.g. Ausubel, I., and Frederick, M., Curr. Prot. Mol. Biol. (1992) John Wiley and Sons, New York; Sambrook, J., et al., Molecular Cloning: A laboratory manual (1999) Cold Spring Harbor Laboratory Press, New York, USA; Hames, B.D., and Higgins, S.G., Nucleic acid hybridization - a practical approach (1985) IRL Press, Oxford, England).

The following abbreviations and definitions are used within this invention.

A "polypeptide" is a polymer of amino acid residues joined by peptide bonds, whether produced naturally or synthetically. Polypeptides of less than about 20 amino acid residues may be referred to as "peptides"; polypeptides of more than about 100 amino acid residues may be referred to as "proteins".

The term "hemopexin-like domain" (PEX) stands for a polypeptide which displays sequence and structure homology to the blood protein hemopexin. This domain has a mean sequence of about 200 amino acids and consists of four repeating subdomains.

The abbreviation "PEX2" stands for the C-terminal domain of human matrix-metalloproteinase 2, comprising the amino acid positions 466 to 660 of the full length protein.

The term "consensus sequence" stands for a deduced sequence, either nucleotide or amino acid sequence. This sequence represents a plurality of similar sequences. Each position in the consensus sequence corresponds to the most frequently occurring base or amino acid at that position which is determined by aligning three or more sequences.

The term "alter" stands for a process in which a defined position in a sequence, either nucleic acid sequence or amino acid sequence, is modified. This comprises the replacement of an amino acid or a nucleic acid (nucleotide) with a different amino acid or nucleic acid (nucleotide) as well as the deletion or insertion.

The expression "a polypeptide binding a molecule" stands for a polypeptide that has the ability to bind a target molecule. The term "specifically binds" stands for a binding activity with an affinity constant of more than 10 E 7 (10⁷) liters/mole.

The expression "predetermined target molecule" denotes a molecule which is a member of the groups of proteins comprising hedgehog proteins, bone morphogenetic proteins, growth factors, erythropoietin, thrombopoietin, G-CSF, interleukins and interferons, immunoglobulins, enzymes, inhibitors, activators, and cell surface proteins.

The term "expression vector" or "vector" stands for a natural or artificial DNA sequence comprising at least a nucleic acid sequence encoding the amino acid sequence of a polypeptide, a promoter sequence, a terminator sequence, a selection marker and an origin of replication.

The term "nucleic acid molecule" or "nucleic acid" stands for a polynucleotide molecule which can be, e.g., DNA, RNA or derivatives thereof. Due to the degeneracy of the genetic code, different nucleic acid sequences encode the same polypeptide. These variations are also included.

The term "amino acid" stands for alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The term "amino acid diversity number" stands for the number of different amino acids present at a specific position of an amino acid sequence. This number is determined by aligning the sequences of an assembly of a plurality of sequences of polypeptides which are homologous in structure and/or function from the same and/or different organisms to a reference or consensus sequence and identifying the total number of different amino acids present in all aligned sequences at the specific position.

The term "aligning" stands for the process of lining up two or more sequences to achieve maximal levels of identity and conservation. It comprises the determination of positional homology for molecular sequences, involving the juxtaposition of amino acids or nucleotides in homologous molecules. As a result the compared sequences are presented in a form that the regions of greatest statistical similarity are shown. During this process it may be found that some sequences do not contain all positions of other aligned sequences, i.e. it may be possible that sequences contain one or more deletions. To achieve maximal levels of identity and conservation gaps can be introduced in these sequences. The gaps are denoted by hyphens in the illustration of the alignment.

The terms "Overlapping Extension Ligation PCR" (OEL-PCR) and "Linear Expression Element" (LEE) stand for a method to ligate DNA fragments and describe linear DNA fragments used in and obtained by this method (see e.g. Ho, S.N., et al., Gene 77 (1989) 51-59; Kain, K.C., et al., Biotechniques 10 (1991) 366-374; Shuldiner, A.R., et al., Anal. Biochem. 194 (1991) 9-15). A gene-transcript is segmented into the modules "promotor-module", "gene-module" and "terminator-module". The promotor-module encodes the T7 phage transcription promotor sequence, a translation control sequence (RBS = ribosomal binding site) and the T7 phage enhancer sequence (g10epsilon) (Lee, S.S., and Kang, C., Kor. Biochem. J. 24 (1991) 673-679). These regulatory sequences enable a coupled transcription and translation in a rapid translation system, e.g. RTS 100 E.coli HY System from Roche Applied Sciences, Mannheim, Germany. The terminator-module encodes a translation stop-codon and a palindromic T7 phage termination-motif (T7T). Optionally, these modules comprised DNA sequences encoding polypeptides, which can be used in subsequent affinity purification or labeling procedures. Linear Expression Elements (Sykes, K.F., and Johnston, S.A., Nature Biotechnol. 17 (1999) 355-359) were assembled by these modules by a two-step PCR. In a first standard PCR using the Pyrococcus woesii DNA-polymerase (PWO-PCR) an intron-less open reading frame, i.e. the gene-module, is amplified by sequence-specific flanking primer oligonucleotides, which introduce overlapping complementary sequences to the promotor- and terminator-modules. The PCR-mediated ligation of these DNA fragments requires a free hybridization energy of the complementary sequences, which has to be lower than a delta G of -25 kcal/mol. This is achieved by sequence extensions, which are in average 25 bp in length. The primer oligonucleotides are designed to hybridize with the gene template at a temperature between 48°C to 55 °C. This enforces the use of primer oligonucleotides with an average length of 45 bp to 55 bp. After 30 PCR cycles the PCR mixture containing approximately 50 ng of the elongated gene-module DNA is transferred into a second PCR mixture. This PCR is supplied with 50 ng to 100 ng of the respective promotor- and terminator-DNA-modules and sequence specific terminal primers. In the presence of a DNA-polymerase the 3'-ends of the hybridized complementary DNA-fragments are enzymatic elongated (Barik, S., Meth. Mol. Biol. 192 (2002) 185-196) to a full length DNA transcript comprising all three modules.

The term "microorganism" denotes prokaryotic microorganisms and eukaryotic microorganisms. The "microorganism" is preferably selected from the group consisting of E.coli strains, Bacillus subtilis strains, Klebsiella strains, Salmonella strains, Pseudomonas strains or Streptomyces strains and yeast strains. For example, E.coli strains comprises E.coli-K12, UT5600, HB101, XL1, X1776, W3110; yeast strains, e.g., comprises Saccharomyces, Pichia, Hansenula, Kluyveromyces and Schizosaccharomyces.

Two criteria substantially characterize an applicable protein-scaffold: First, the protein should belong to a family which reveals a well defined hydrophobic core. A close relationship between the individual family members is beneficial (Skerra, A., J. Mol. Recognit. 13 (2000) 167-187). Second, the protein should posses a spatially separated and functionally independent accessible active site or binding pocket. This should not contribute to the intrinsic core-stability (Predki, P.F., et al., Nature Struct. Biol. 3 (1996) 54-58). Ideally, this protein-family is inherently involved in the recognition of multiple, non-related targets.

The hemopexin-like (PEX) protein scaffold fulfills these criteria. This structural motive is present in a plurality of different proteins and protein families, e.g. hemopexin (Altruda, F. et al., Nucleic Acids Res. 13 (1985) 3841-3859), vitronectin (Jenne, D., and Stanley, K.K., Biochemistry 26 (1987) 6735-6742) or pea seed albumin 2 (Jenne, D., Biochem. Biophys. Res. Commun. 176 (1991) 1000-1006).

The crystal structure analyses of proteins containing hemopexin-like domains show that this domain adopts a four bladed beta-propeller topology (Li, J., et al., Structure 3 (1995) 541-549; Faber, H.R., et al., Structure 3 (1995) 551-559). The blades are each composed of four beta-sheets in an anti-parallel orientation. Together they form a cavity in the center of the molecule. The four blades are linked together via loops from the fourth outermost beta-strand of the preceding blade to the first innermost beta-strand of the next blade. A disulphide bond connects the terminal ends of the structure, i.e. blade 4 and blade 1.

The PEX scaffold is involved in different, but quite specific protein-protein- and protein-ligand-interactions. Therefore the hemopexin-like structure forms a versatile framework for molecular recognition (Bode, W., Structure 3 (1995) 527-530). For example, binding sites for ions like calcium, sodium and chloride (Libson, A.M., et al., Nat. Struct. Biol. 2 (1995) 938-942; Gohlke, U., et al., FEBS Lett. 378 (1996) 126-130) as well as for interaction with fibronectin, TIMP-1/2 (tissue inactivator of human matrix metalloproteinase 1/2), integrins and heparin are known (Wallon, U.M., and Overall, C.M., J. Biol. Chem. 272 (1997) 7473-7481; Willenbrock, F., et al., Biochemistry 32 (1993) 4330-4337; Brooks, P.C., et al., Cell 92 (1998) 391-400; Bode, W., Structure 3 (1995) 527-530).

The hemopexin-like protein domain offers a high structural homology among its protein family members. High structural equivalence of the hemopexin-domains of e.g. human Matrix-metalloproteinases 1, 2 and 13 has been reported (Gomis-Ruth, F.X., et al., J. Mol. Biol. 264 (1996) 556-566). The predominantly hydrophobic interactions between the adjacent and perpendicularly oriented beta-sheets provide most of the required structural stability (Gomis-Ruth, F.X., et al., J. Mol. Biol. 264 (1996) 556-566; Fulop, V., and Jones, D.T., Curr. Opin. Struct. Biol. 9 (1999) 715-721).

Protein-databases like SMART (Schultz, J., et al., PNAS 95 (1998) 5857-5864; Letunic, I., et al., Nuc. Acids Res. 30 (2002) 242-244) were recently used to compare homologous sequences and protein-folds in order to identify non-conserved, and thus theoretically alterable, i.e. randomizable, amino acid positions in suitable protein frameworks (Binz, H.K., et al., J. Mol. Biol. 332 (2003) 489-503; Forrer, P., et al., ChemBioChem 5 (2004) 183-189).

To identify potentially alterable, i.e. randomizable, amino acid positions in the PEX fold, a similar approach was performed using the SMART database. Amino acid positions were identified in the PEX domain, which are randomizable without affecting the proteins structure, functional conformation and stability.

From the SMART database 60 PEX-domains, that are listed in the following table I, from different species were aligned with the Pretty bioinformatics tool (GCG) using the scoring matrix blosum 62.

**Table I: Listing of the 60 proteins containing the PEX-domain.**

| Protein family | PEX-fold in PDB data bank code | swissprot data bank number | sequence id of the hemopexin domain as used in this invention (SEQ ID NO:) |
|---|---|---|---|
| peroxisome | PEX2_mouse | P55098 | 03 |
| assembly factor | PEX2_rat | P24392 | 04 |
| matrix | MM01_Bovin | P28053 | 06 |
| metalloproteinase | MM01_HORSE | Q9XSZ5 | 07 |
| 1 | MM01_human | P03956 | 08 |
| | MM01_PIG | P21692 | 09 |
| matrix | MM02_chick (chicken) | Q90611 | 02 |
| metalloproteinase | MM02_human | P08253 | 10 |
| 2 | MM02_rabbit | P50757 | 05 |
| matrix | MM03_human | P08254 | 11 |
| metalloproteinase | MM03_MOUSE | P28862 | 12 |
| 3 | MM03_RABIT | P28863 | 13 |
| | MM03_RAT | P03957 | 14 |
| matrix | MM08_human | P22894 | 15 |
| metalloproteinase | MM08_MOUSE | 070138 | 16 |
| 8 | MM08_RAT | 088766 | 17 |
| matrix | MM09_BOVIN | P52176 | 18 |
| metalloproteinase | MM09_CANFA | 018733 | 19 |
| 9 | (canis familiaris, dog) | | |
| | MM09_human | P14780 | 20 |
| | MM09_MOUSE | P41245 | 21 |
| metalloproteinase | MM10_human | P09238 | 22 |
| 10 | MM10_MOUSE | 055123 | 23 |
| matrix | | | |
| metalloproteinase | MM11_human | P24347 | 24 |
| 11 | MM11_MOUSE | Q02853 | 25 |
| matrix | MM12_human | P39900 | 26 |
| metalloproteinase | MM12_MOUSE | P34960 | 27 |
| 12 | MM12_RABIT | P79227 | 28 |
| | MM12_RAT | Q63341 | 29 |
| matrix | MM13_BOVIN | 077656 | 30 |
| metalloproteinase | MM13_HORSE | 018927 | 31 |
| 13 | MM13_human | P45452 | 32 |
| | MM13_RABIT | 062806 | 33 |
| matrix | MM14_human | P50281 | 34 |
| metalloproteinase | MM14_mouse | P53690 | 35 |
| 14 | MM14_PIG | Q9XT90 | 36 |
| | MM14_RABIT | Q95220 | 37 |
| | MM14_RAT | Q10739 | 38 |
| matrix | MM15_human | P51511 | 39 |
| metalloproteinase | MM15_MOUSE | 054732 | 40 |
| 15 | | | |
| matrix | MM16_human | P51512 | 41 |
| metalloproteinase | MM16_MOUSE | Q9WTR0 | 42 |
| 16 | MM16_RAT | 035548 | 43 |
| matrix metalloproteinase | MM17_human | Q9ULZ9 | 44 |
| 17 | MM17_MOUSE | Q9R0S3 | 45 |
| matrix metalloproteinase | MM18_XENLA | | |
| 18 | (Xenopus laevis, African clawed frog) | 013065 | 46 |
| matrix metalloproteinase | MM19_human | Q99542 | 47 |
| 19 | MM19_MOUSE | Q9JHI0 | 48 |
| matrix | MM20_BOVIN | 018767 | 49 |
| metalloproteinase | MM20_human | 060882 | 50 |
| 20 | MM20_MOUSE | P57748 | 51 |
| | MM20_PIG | P79287 | 52 |
| matrix | MM24_human | Q9Y5R2 | 53 |
| metalloproteinase | MM24_MOUSE | Q9R0S2 | 54 |
| 24 | MM24_RAT | Q99PW6 | 55 |
| matrix | | | |
| metalloproteinase | MM25_human | Q9NPA2 | 56 |
| 25 | | | |
| matrix | | | |
| metalloproteinase | MM28_human | Q9H239 | 57 |
| 28 | | | |
| vitronectin | VTNC_human | P04004 | 58 |
| | VTNC_MOUSE | P29788 | 59 |
| | VTNC_PIG | P48819 | 60 |
| | VTNC_RABIT | P22458 | 61 |

| | | | |
|---|---|---|---|
| (Table I end). | | | |

The hemopexin-like domain just accounts for a small part of the full length protein. The following table lists the location of the aligned hemopexin-like domain in the full length proteins.

**Table II: Location of the hemopexin-like domains in the proteins of table I.**

| protein | sequence amino acids | hemopexin-like domain | |
|---|---|---|---|
| | total | start | end |
| SEQ ID NO:02 | 663 | 469 | 663 |
| SEQ ID NO:03 | 305 | 97 | 280 |
| SEQ ID NO:04 | 305 | 97 | 280 |
| SEQ ID NO:05 | 662 | 468 | 662 |
| SEQ ID NO:06 | 469 | 275 | 469 |
| SEQ ID NO:07 | 469 | 275 | 469 |
| SEQ ID NO:08 | 469 | 275 | 469 |
| SEQ ID NO:09 | 469 | 275 | 469 |
| SEQ ID NO:10 | 660 | 466 | 660 |
| SEQ ID NO:11 | 477 | 287 | 477 |
| SEQ ID NO:12 | 477 | 287 | 477 |
| SEQ ID NO:13 | 478 | 288 | 478 |
| SEQ ID NO:14 | 475 | 285 | 475 |
| SEQ ID NO:15 | 467 | 276 | 467 |
| SEQ ID NO:16 | 465 | 276 | 465 |
| SEQ ID NO:17 | 466 | 277 | 466 |
| SEQ ID NO:18 | 712 | 518 | 712 |
| SEQ ID NO:19 | 704 | 510 | 704 |
| SEQ ID NO:20 | 707 | 513 | 707 |
| SEQ ID NO:21 | 730 | 531 | 730 |
| SEQ ID NO:22 | 476 | 286 | 476 |
| SEQ ID NO:23 | 476 | 286 | 476 |
| SEQ ID NO:24 | 488 | 291 | 483 |
| SEQ ID NO:25 | 492 | 295 | 487 |
| SEQ ID NO:26 | 470 | 279 | 470 |
| SEQ ID NO:27 | 462 | 272 | 462 |
| SEQ ID NO:28 | 464 | 274 | 464 |
| SEQ ID NO:29 | 465 | 275 | 465 |
| SEQ ID NO:30 | 471 | 281 | 471 |
| SEQ ID NO:31 | 472 | 282 | 472 |
| SEQ ID NO:32 | 471 | 281 | 471 |
| SEQ ID NO:33 | 471 | 281 | 471 |
| SEQ ID NO:34 | 582 | 316 | 511 |
| SEQ ID NO:35 | 582 | 316 | 511 |
| SEQ ID NO:36 | 580 | 314 | 509 |
| SEQ ID NO:37 | 582 | 316 | 511 |
| SEQ ID NO:38 | 582 | 316 | 511 |
| SEQ ID NO:39 | 669 | 367 | 562 |
| SEQ ID NO:40 | 657 | 365 | 558 |
| SEQ ID NO:41 | 607 | 340 | 535 |
| SEQ ID NO:42 | 607 | 340 | 535 |
| SEQ ID NO:43 | 607 | 340 | 535 |
| SEQ ID NO:44 | 606 | 332 | 529 |
| SEQ ID NO:45 | 578 | 333 | 530 |
| SEQ ID NO:46 | 467 | 277 | 467 |
| SEQ ID NO:47 | 508 | 286 | 475 |
| SEQ ID NO:48 | 527 | 286 | 474 |
| SEQ ID NO:49 | 481 | 291 | 481 |
| SEQ ID NO:50 | 483 | 293 | 483 |
| SEQ ID NO:51 | 482 | 292 | 482 |
| SEQ ID NO:52 | 483 | 293 | 483 |
| SEQ ID NO:53 | 645 | 377 | 572 |
| SEQ ID NO:54 | 618 | 350 | 545 |
| SEQ ID NO:55 | 618 | 350 | 545 |
| SEQ ID NO:56 | 562 | 314 | 511 |
| SEQ ID NO:57 | 520 | 328 | 520 |
| SEQ ID NO:58 | 478 | 288 | 478 |
| SEQ ID NO:59 | 478 | 287 | 478 |
| SEQ ID NO:60 | 459 | 265 | 459 |
| SEQ ID NO:61 | 475 | 288 | 475 |

| | | | |
|---|---|---|---|
| (Table II end). | | | |

A consensus sequence of 210 positions has been determined by the alignment of the above listed hemopexin-like domains (SEQ ID NO:01, SEQ ID NO:88).

The number of different amino acids per position has been determined in order to compile an amino acid diversity number ("determination of variability"; see table III). Gaps in the sequence are marked by a hyphen (-) (see table IV for the alignment of all sequences). For every position of the consensus sequence the number of different amino acids (amino acid diversity number) is given. The maximum possible number is 21 (20 different amino acids + 1 gap). A low diversity number indicates a highly conserved position. A high diversity number indicates flexibility at this position.

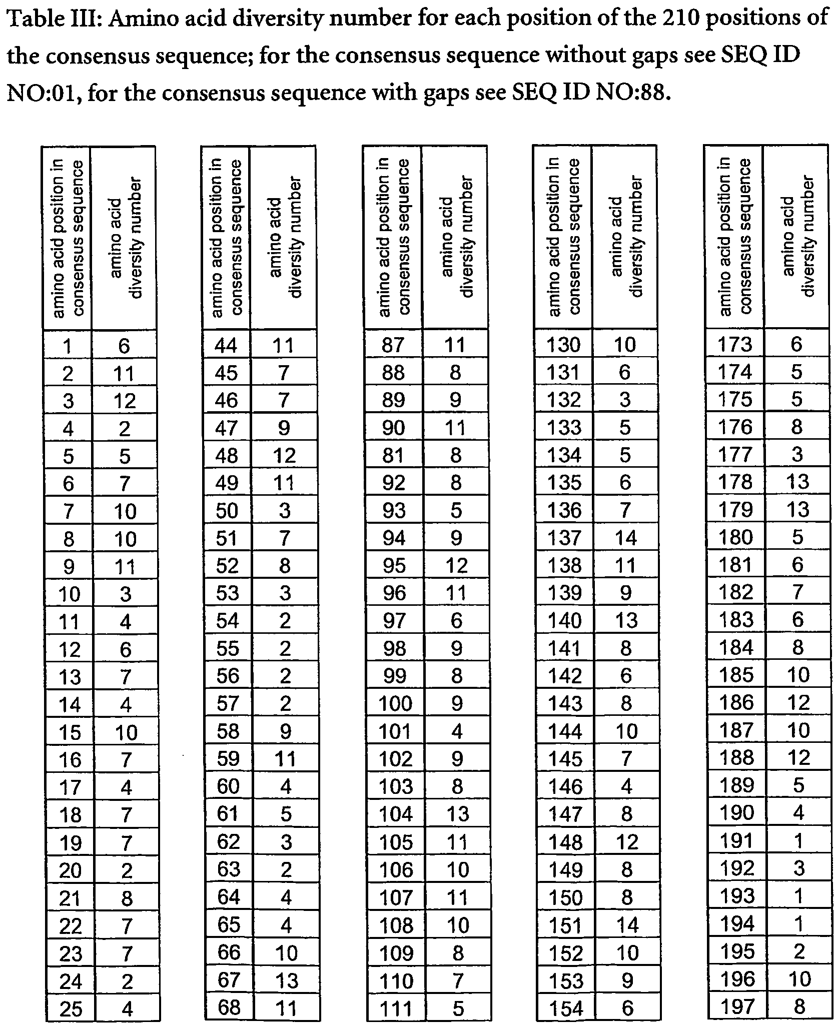

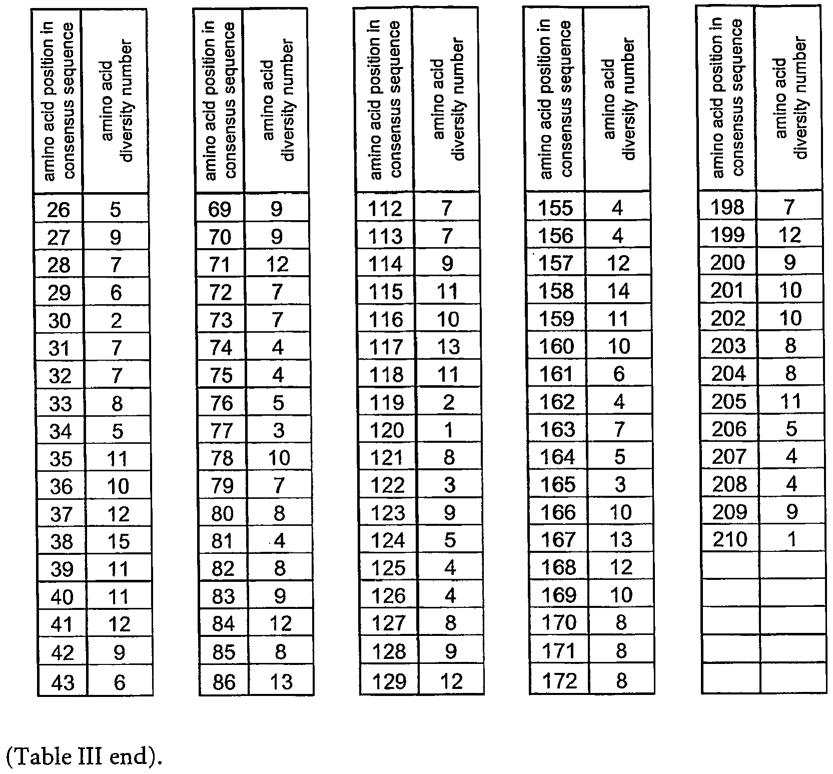

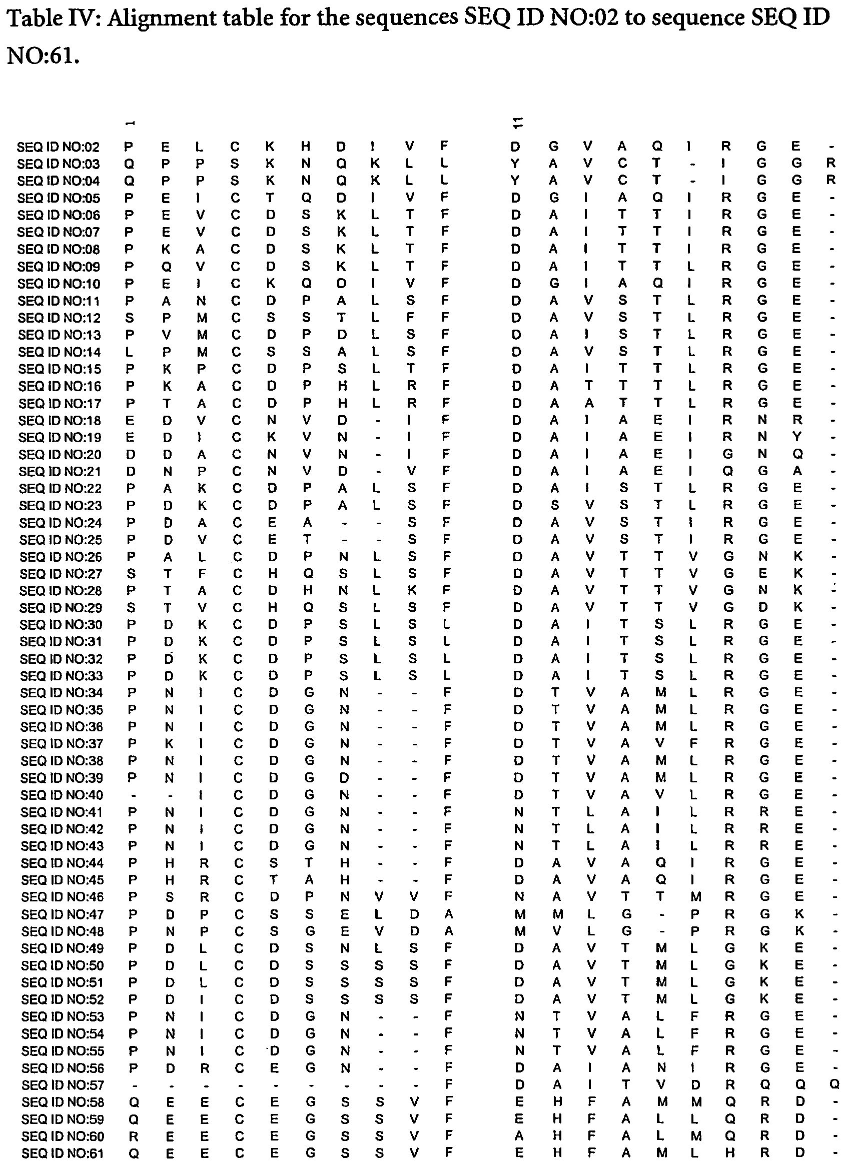

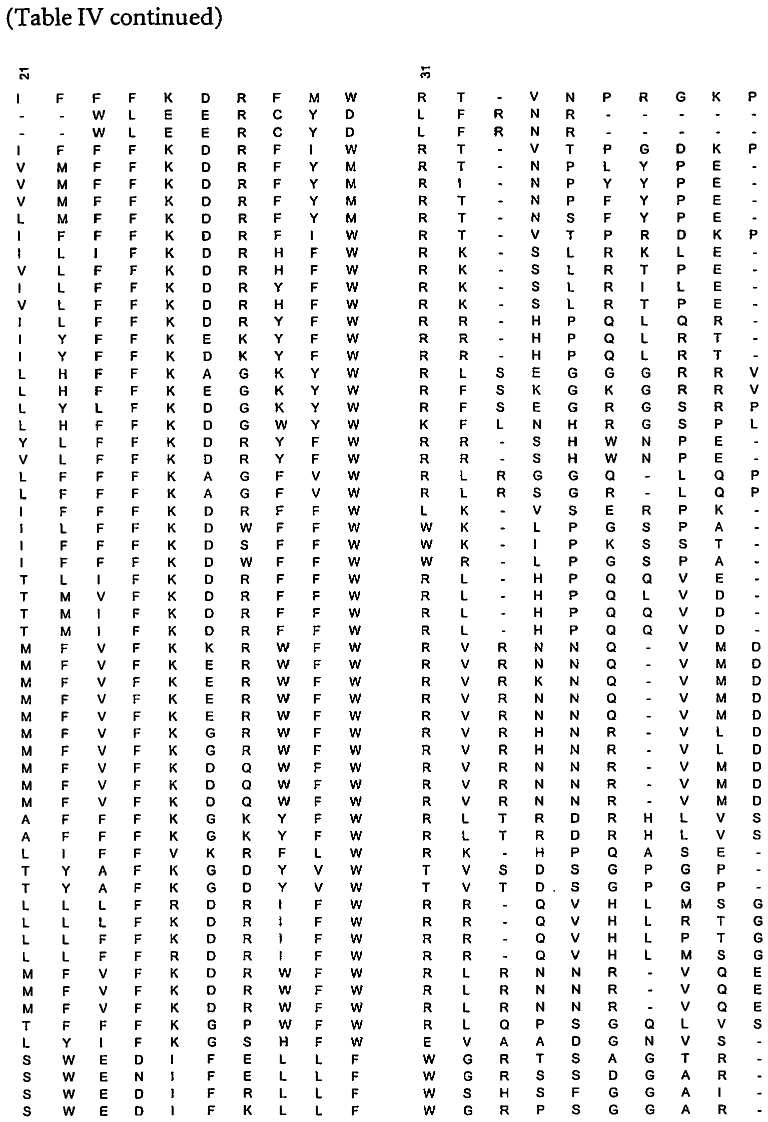

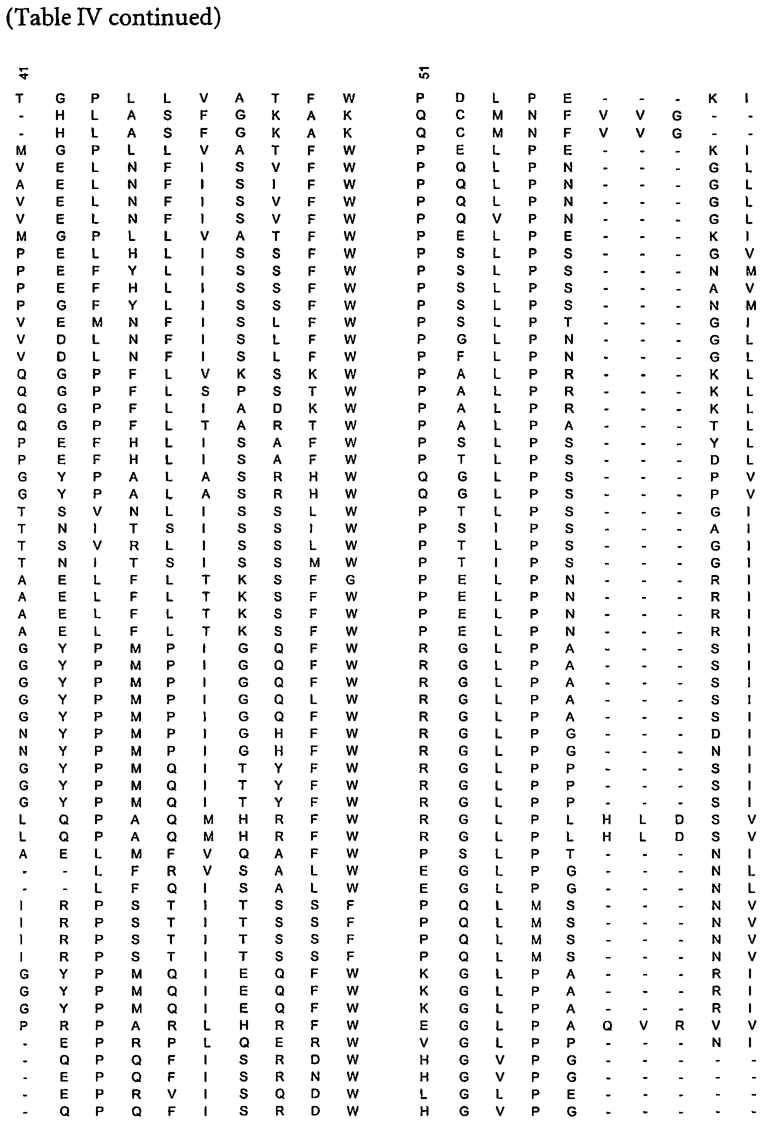

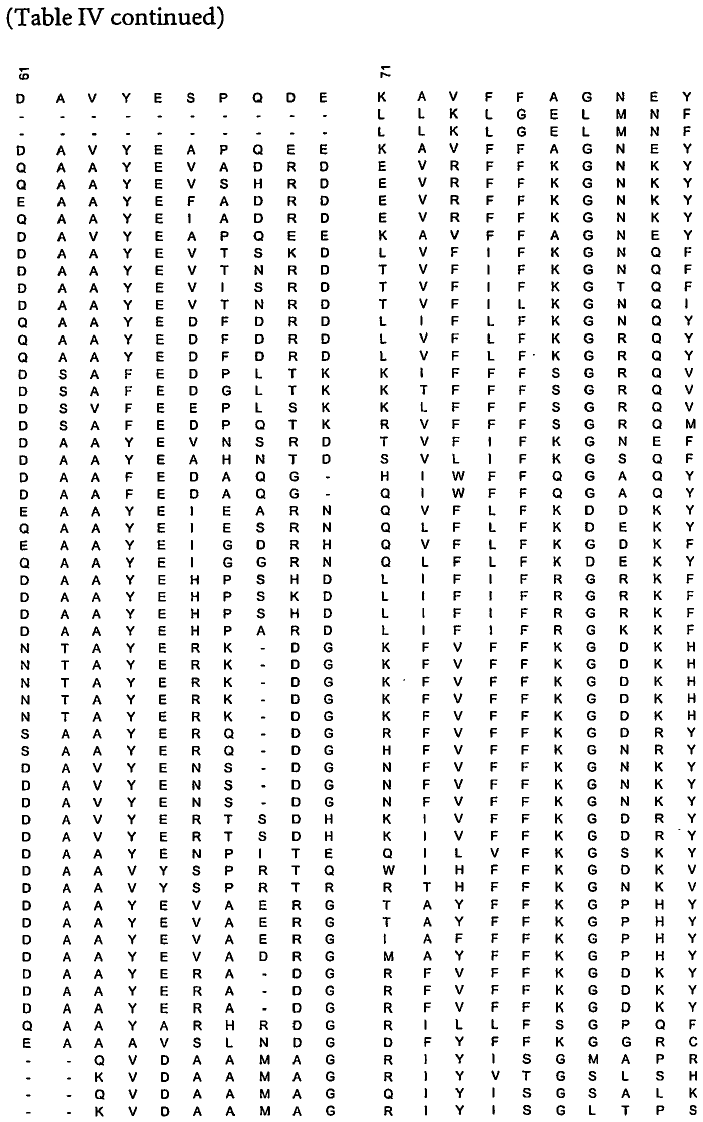

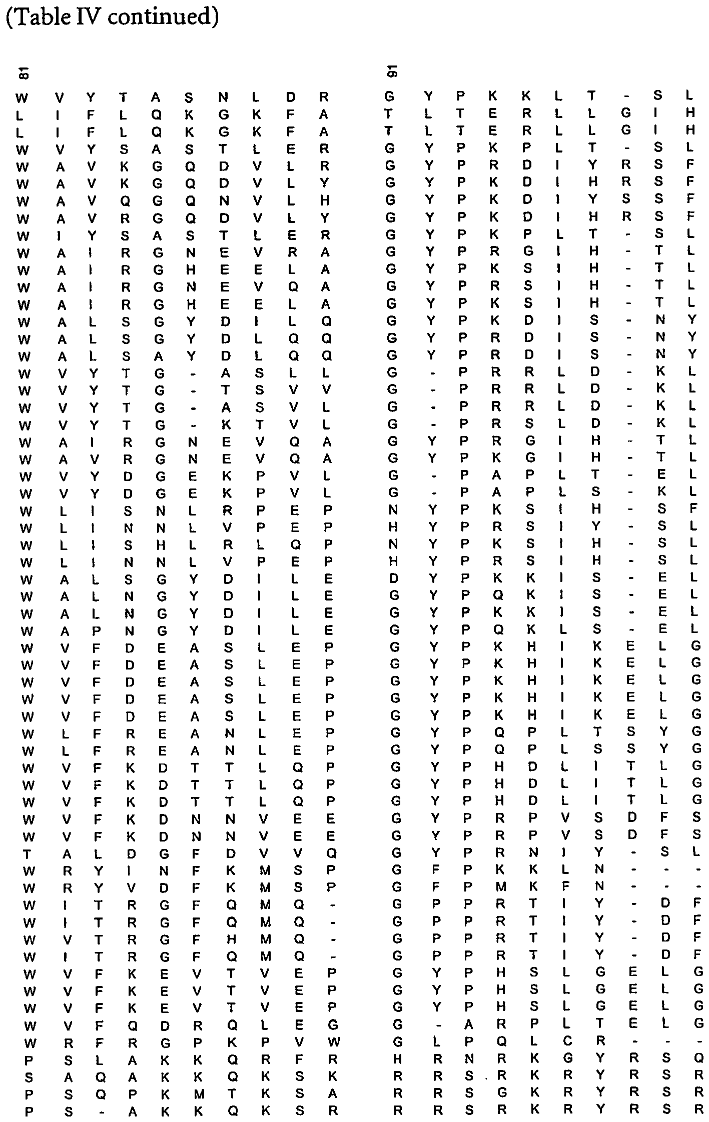

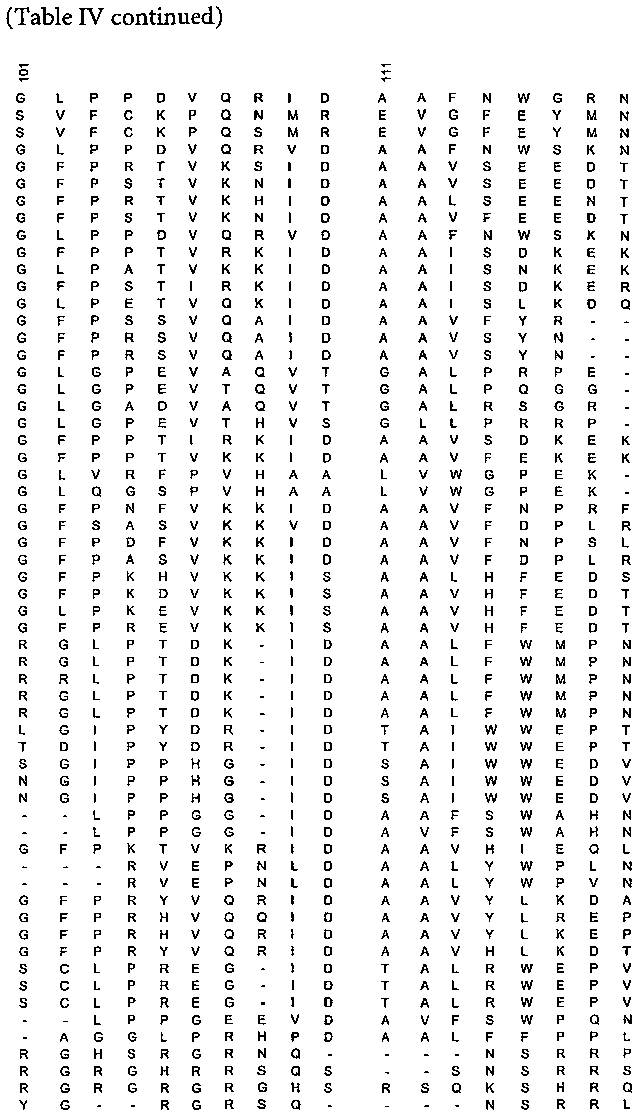

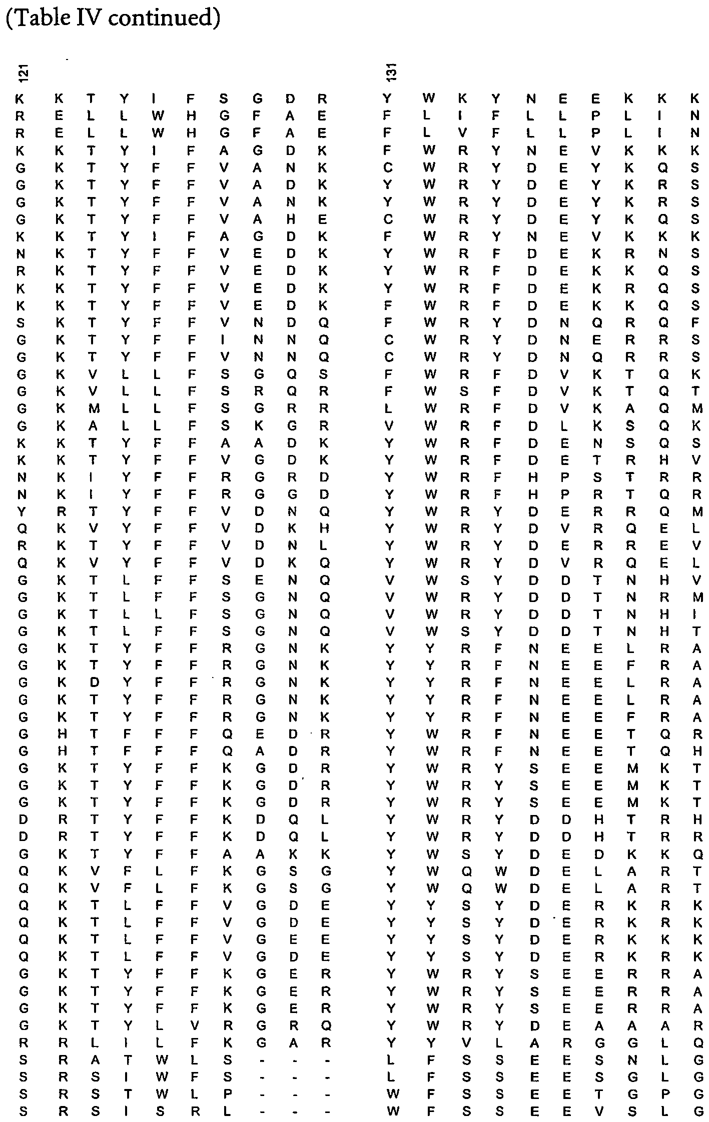

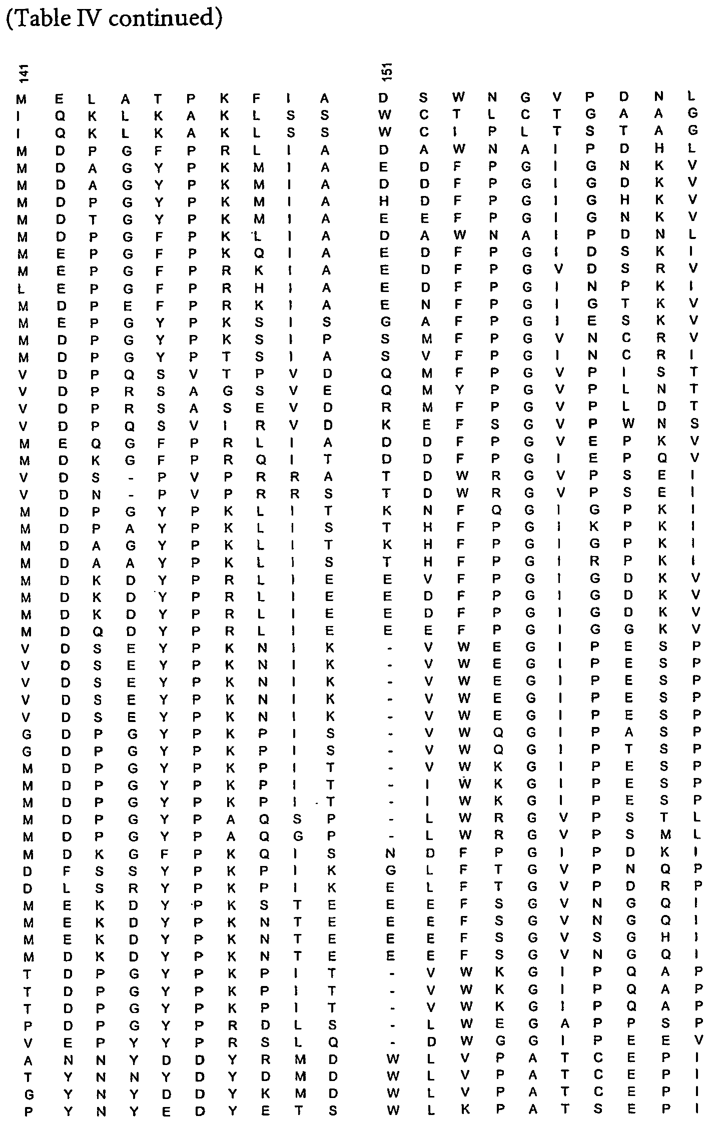

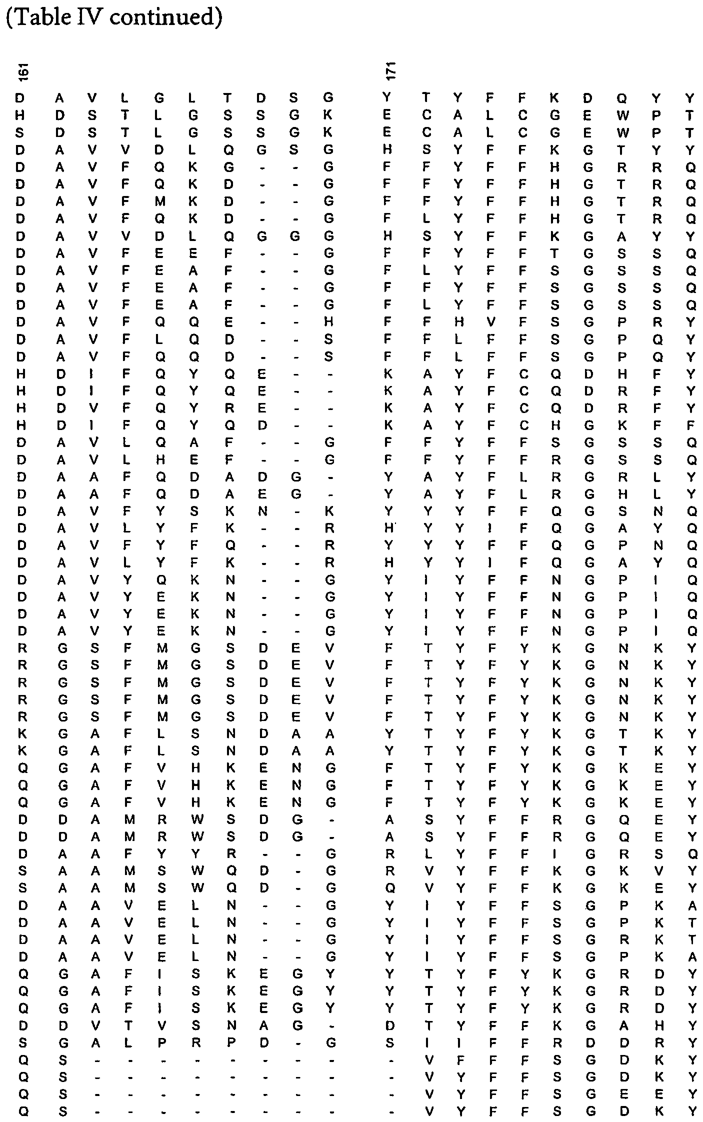

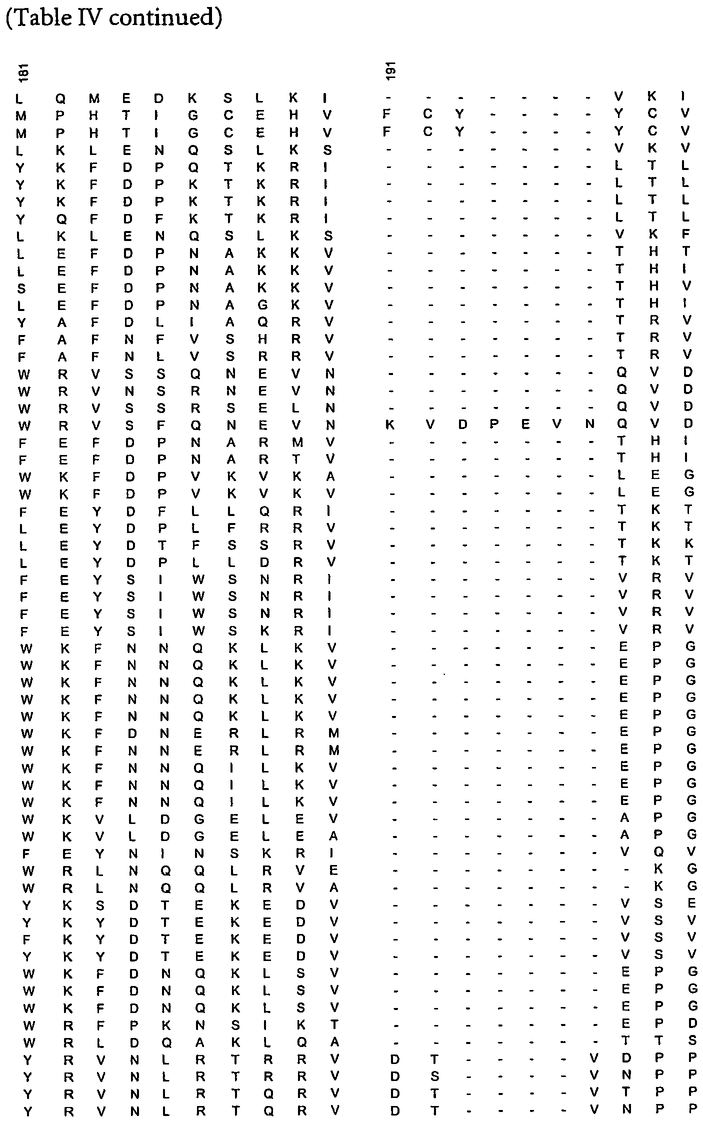

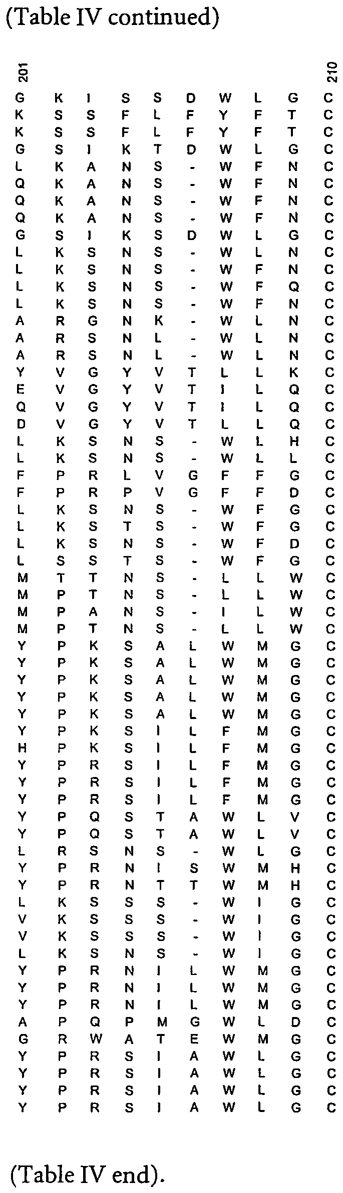

This method for the determination of the amino acid diversity number is an all-purpose method and generally applicable and not limited to a specific amino acid sequence, polypeptide, domain or protein. Thus, the proceeding can be applied similarly and accordingly with other sequences to determine and identify amino acid positions with a high variability and which are accessible for alteration without having a strong influence on the stability and functionality of the structure.

With the amino acid diversity calculation amino acid positions with a low diversity number, i.e. smaller than 6, have been identified. This low diversity number resembles a high conservation like e.g. for the cysteine residue in position Nr. 4 (see table III), which was found to be conserved in 57 of the 60 sequences analyzed (see table IV). The cysteine residue in position Nr. 210 was found to be conserved in all analyzed hemopexin-like sequences. This demonstrates the excellent applicability of this approach, as these two cysteine residues are of high importance in the scaffold. These two residues form the disulphide bond that is essential for the formation of the hemopexin-like structure by linking the fourth blade with the first blade of the polypeptide.

From the amino acid diversity number as compiled and listed table III amino acid positions in the consensus sequence with a high diversity/variability can be identified. Because from the identified high diversity amino acid numbers of the consensus sequence the corresponding amino acid numbers of the full length polypeptide cannot be obtained directly, table V lists the amino acid numbers of the identified high diversity amino acids, i.e. alterable amino acids, of the full length polypeptides of SEQ ID NO:02 to SEQ ID NO:61.

**Table V: Listing of the alterable amino acid positions in each of the sequences SEQ ID NO:02 to sequence SEQ ID NO:61. The numbering of the positions in each sequence is in consistency with the amino acid numbering of the corresponding full length protein.**

| SEQ ID NO:02: | | | | | | | | | | |
|---|---|---|---|---|---|---|---|---|---|---|
| | 470 | 471 | 475 | 476 | 477 | 484 | 501 | 502 | 503 | 504 |
| | 505 | 506 | 507 | 510 | 514 | 515 | 522 | 529 | 530 | 531 |
| | 534 | 541 | 547 | 549 | 550 | 553 | 558 | 559 | 566 | 567 |
| | 568 | 569 | 570 | 577 | 578 | 579 | 580 | 589 | 590 | 597 |
| | 598 | 600 | 604 | 608 | 611 | 612 | 617 | 618 | 619 | 620 |
| | 626 | 627 | 628 | 629 | 638 | 639 | 645 | 646 | 647 | 648 |
| | 650 | 652 | 654 | 655 | 658 | 663 | | | | |
| | | | | | | | | | | |

| SEQ ID NO:03: | | | | | | | | | | |
|---|---|---|---|---|---|---|---|---|---|---|
| | | | | | | | | | | |
| | 98 | 99 | 103 | 104 | 105 | 111 | 128 | 131 | 135 | 136 |
| | 145 | 146 | 153 | 159 | 161 | 162 | 165 | 170 | 171 | 179 |
| | 180 | 181 | 182 | 183 | 190 | 191 | 192 | 193 | 202 | 203 |
| | 210 | 211 | 213 | 217 | 221 | 224 | 225 | 230 | 231 | 232 |
| | 233 | 239 | 240 | 241 | 242 | 251 | 252 | 258 | 259 | 260 |
| | 261 | 266 | 268 | 270 | 271 | 274 | 279 | | | |
| | | | | | | | | | | |

| SEQ ID NO:04: | | | | | | | | | | |
|---|---|---|---|---|---|---|---|---|---|---|
| | | | | | | | | | | |
| | 98 | 99 | 103 | 104 | 105 | 111 | 128 | 131 | 135 | 136 |
| | 145 | 146 | 153 | 159 | 161 | 162 | 165 | 170 | 171 | 179 |
| | 180 | 181 | 182 | 183 | 190 | 191 | 192 | 193 | 202 | 203 |
| | 210 | 211 | 213 | 217 | 221 | 224 | 225 | 230 | 231 | 232 |
| | 233 | 239 | 240 | 241 | 242 | 251 | 252 | 258 | 259 | 260 |
| | 261 | 266 | 268 | 270 | 271 | 274 | 279 | | | |
| | | | | | | | | | | |

| SEQ ID NO:05: | | | | | | | | | | |
|---|---|---|---|---|---|---|---|---|---|---|
| | 469 | 470 | 474 | 475 | 476 | 483 | 500 | 501 | 502 | 503 |
| | 504 | 505 | 506 | 509 | 513 | 514 | 521 | 528 | 529 | 530 |
| | 533 | 540 | 546 | 548 | 549 | 552 | 557 | 558 | 565 | 566 |
| | 567 | 568 | 569 | 576 | 577 | 578 | 579 | 588 | 589 | 596 |
| | 597 | 599 | 603 | 607 | 610 | 611 | 616 | 617 | 618 | 619 |
| | 625 | 626 | 627 | 628 | 637 | 638 | 644 | 645 | 646 | 647 |
| | 649 | 651 | 653 | 654 | 657 | 662 | | | | |
| | | | | | | | | | | |

| SEQ ID NO:06: | | | | | | | | | | |
|---|---|---|---|---|---|---|---|---|---|---|
| | 276 | 277 | 281 | 282 | 283 | 290 | 307 | 308 | 309 | 310 |
| | 311 | 312 | 315 | 319 | 320 | 327 | 334 | 335 | 336 | 339 |
| | 346 | 352 | 354 | 355 | 358 | 363 | 364 | 372 | 373 | 374 |
| | 375 | 376 | 383 | 384 | 385 | 386 | 395 | 396 | 403 | 404 |
| | 406 | 410 | 414 | 417 | 418 | 423 | 424 | 425 | 426 | 432 |
| | 433 | 442 | 443 | 449 | 450 | 451 | 452 | 456 | 458 | 460 |
| | 461 | 464 | 468 | | | | | | | |
| | | | | | | | | | | |

| SEQ ID NO:07: | | | | | | | | | | |
|---|---|---|---|---|---|---|---|---|---|---|
| | 276 | 277 | 281 | 282 | 283 | 290 | 307 | 308 | 309 | 310 |
| | 311 | 312 | 315 | 319 | 320 | 327 | 334 | 335 | 336 | 339 |
| | 346 | 352 | 354 | 355 | 358 | 363 | 364 | 372 | 373 | 374 |
| | 375 | 376 | 383 | 384 | 385 | 386 | 395 | 396 | 403 | 404 |
| | 406 | 410 | 414 | 417 | 418 | 423 | 424 | 425 | 426 | 432 |
| | 433 | 442 | 443 | 449 | 450 | 451 | 452 | 456 | 458 | 460 |
| | 461 | 464 | 468 | | | | | | | |
| | | | | | | | | | | |

| SEQ ID NO:08: | | | | | | | | | | |
|---|---|---|---|---|---|---|---|---|---|---|
| | 276 | 277 | 281 | 282 | 283 | 290 | 307 | 308 | 309 | 310 |
| | 311 | 312 | 315 | 319 | 320 | 327 | 334 | 335 | 336 | 339 |
| | 346 | 352 | 354 | 355 | 358 | 363 | 364 | 372 | 373 | 374 |
| | 375 | 376 | 383 | 384 | 385 | 386 | 395 | 396 | 403 | 404 |
| | 406 | 410 | 414 | 417 | 418 | 423 | 424 | 425 | 426 | 432 |
| | 433 | 442 | 443 | 449 | 450 | 451 | 452 | 456 | 458 | 460 |
| | 461 | 464 | 468 | | | | | | | |
| | | | | | | | | | | |

| SEQ ID NO:09: | | | | | | | | | | |
|---|---|---|---|---|---|---|---|---|---|---|
| | 276 | 277 | 281 | 282 | 283 | 290 | 307 | 308 | 309 | 310 |
| | 311 | 312 | 315 | 319 | 320 | 327 | 334 | 335 | 336 | 339 |
| | 346 | 352 | 354 | 355 | 358 | 363 | 364 | 372 | 373 | 374 |
| | 375 | 376 | 383 | 384 | 385 | 386 | 395 | 396 | 403 | 404 |
| | 406 | 410 | 414 | 417 | 418 | 423 | 424 | 425 | 426 | 432 |
| | 433 | 442 | 443 | 449 | 450 | 451 | 452 | 456 | 458 | 460 |
| | 461 | 464 | 468 | | | | | | | |
| | | | | | | | | | | |

| SEQ ID NO:10: | | | | | | | | | | |
|---|---|---|---|---|---|---|---|---|---|---|
| | 467 | 468 | 472 | 473 | 474 | 481 | 498 | 499 | 500 | 501 |
| | 502 | 503 | 504 | 507 | 511 | 512 | 519 | 526 | 527 | 528 |
| | 529 | 531 | 538 | 544 | 546 | 547 | 550 | 555 | 556 | 563 |
| | 564 | 565 | 566 | 567 | 574 | 575 | 576 | 577 | 578 | 586 |
| | 587 | 594 | 595 | 596 | 597 | 601 | 605 | 608 | 609 | 614 |
| | 615 | 616 | 617 | 623 | 624 | 625 | 626 | 635 | 636 | 642 |
| | 643 | 644 | 645 | 647 | 649 | 651 | 652 | 655 | 660 | |
| | | | | | | | | | | |

| SEQ ID NO:11: | | | | | | | | | | |
|---|---|---|---|---|---|---|---|---|---|---|
| | | | | | | | | | | |
| | 288 | 289 | 293 | 294 | 295 | 302 | 319 | 320 | 321 | 322 |
| | 323 | 324 | 327 | 331 | 332 | 339 | 346 | 347 | 348 | 351 |
| | 358 | 364 | 366 | 367 | 370 | 375 | 376 | 383 | 384 | 385 |
| | 386 | 387 | 394 | 395 | 396 | 397 | 406 | 407 | 414 | 415 |
| | 417 | 421 | 425 | 428 | 429 | 434 | 435 | 436 | 437 | 443 |
| | 444 | 454 | 455 | 461 | 462 | 463 | 464 | 467 | 469 | 471 |
| | 472 | 475 | 479 | | | | | | | |
| | | | | | | | | | | |

| SEQ ID NO:12: | | | | | | | | | | |
|---|---|---|---|---|---|---|---|---|---|---|
| | | | | | | | | | | |
| | 288 | 289 | 293 | 294 | 295 | 302 | 319 | 320 | 321 | 322 |
| | 323 | 324 | 327 | 331 | 332 | 339 | 346 | 347 | 348 | 351 |
| | 358 | 364 | 366 | 367 | 370 | 375 | 376 | 383 | 384 | 385 |
| | 386 | 387 | 394 | 395 | 396 | 397 | 406 | 407 | 414 | 415 |
| | 417 | 421 | 425 | 428 | 429 | 434 | 435 | 436 | 437 | 443 |
| | 444 | 454 | 455 | 461 | 462 | 463 | 464 | 467 | 469 | 471 |
| | 472 | 475 | 479 | | | | | | | |
| | | | | | | | | | | |

| SEQ ID NO:13: | | | | | | | | | | |
|---|---|---|---|---|---|---|---|---|---|---|
| | 289 | 290 | 294 | 295 | 296 | 303 | 320 | 321 | 322 | 323 |
| | 324 | 325 | 328 | 332 | 333 | 340 | 347 | 348 | 349 | 352 |
| | 359 | 365 | 367 | 368 | 371 | 376 | 377 | 384 | 385 | 386 |
| | 387 | 388 | 395 | 396 | 397 | 398 | 407 | 408 | 415 | 416 |
| | 418 | 422 | 426 | 429 | 430 | 435 | 436 | 437 | 438 | 444 |
| | 445 | 455 | 456 | 462 | 463 | 464 | 465 | 468 | 470 | 472 |
| | 473 | 476 | 480 | | | | | | | |
| | | | | | | | | | | |

| SEQ ID NO:14: | | | | | | | | | | |
|---|---|---|---|---|---|---|---|---|---|---|
| | | | | | | | | | | |
| | 286 | 287 | 291 | 292 | 293 | 300 | 317 | 318 | 319 | 320 |
| | 321 | 322 | 325 | 329 | 330 | 337 | 344 | 345 | 346 | 349 |
| | 356 | 362 | 364 | 365 | 368 | 373 | 374 | 381 | 382 | 383 |
| | 384 | 385 | 392 | 393 | 394 | 395 | 404 | 405 | 412 | 413 |
| | 415 | 419 | 423 | 426 | 427 | 432 | 433 | 434 | 435 | 441 |
| | 442 | 452 | 453 | 459 | 460 | 461 | 462 | 465 | 467 | 469 |
| | 470 | 473 | 477 | | | | | | | |
| | | | | | | | | | | |

| SEQ ID NO:15: | | | | | | | | | | |
|---|---|---|---|---|---|---|---|---|---|---|
| | | | | | | | | | | |
| | 277 | 278 | 282 | 283 | 284 | 291 | 308 | 309 | 310 | 311 |
| | 312 | 313 | 316 | 320 | 321 | 328 | 335 | 336 | 337 | 340 |
| | 347 | 353 | 355 | 356 | 359 | 364 | 365 | 372 | 373 | 374 |
| | 375 | 376 | 383 | 384 | 394 | 395 | 402 | 403 | 405 | 409 |
| | 413 | 416 | 417 | 422 | 423 | 424 | 425 | 431 | 432 | 441 |
| | 442 | 448 | 449 | 450 | 451 | 453 | 455 | 457 | 458 | 461 |
| | 465 | | | | | | | | | |
| | | | | | | | | | | |

| SEQ ID NO:16: | | | | | | | | | | |
|---|---|---|---|---|---|---|---|---|---|---|
| | | | | | | | | | | |
| | 277 | 278 | 282 | 283 | 284 | 291 | 308 | 309 | 310 | 311 |
| | 312 | 313 | 316 | 320 | 321 | 328 | 335 | 336 | 337 | 340 |
| | 347 | 353 | 355 | 356 | 359 | 364 | 365 | 372 | 373 | 374 |
| | 375 | 376 | 383 | 384 | 394 | 395 | 402 | 403 | 405 | 409 |
| | 413 | 416 | 417 | 422 | 423 | 424 | 425 | 431 | 432 | 441 |
| | 442 | 448 | 449 | 450 | 451 | 453 | 455 | 457 | 458 | 461 |
| | 465 | | | | | | | | | |
| | | | | | | | | | | |

| SEQ ID NO:17: | | | | | | | | | | |
|---|---|---|---|---|---|---|---|---|---|---|
| | | | | | | | | | | |
| | 278 | 279 | 283 | 284 | 285 | 292 | 309 | 310 | 311 | 312 |
| | 313 | 314 | 317 | 321 | 322 | 329 | 336 | 337 | 338 | 341 |
| | 348 | 354 | 356 | 357 | 360 | 365 | 366 | 373 | 374 | 375 |
| | 376 | 377 | 384 | 385 | 395 | 396 | 403 | 404 | 406 | 410 |
| | 414 | 417 | 418 | 423 | 424 | 425 | 426 | 432 | 433 | 442 |
| | 443 | 449 | 450 | 451 | 452 | 454 | 456 | 458 | 459 | 462 |
| | 466 | | | | | | | | | |
| | | | | | | | | | | |

| SEQ ID NO:18: | | | | | | | | | | |
|---|---|---|---|---|---|---|---|---|---|---|
| | | | | | | | | | | |
| | 519 | 520 | 524 | 525 | 532 | 550 | 551 | 552 | 553 | 554 |
| | 555 | 556 | 559 | 563 | 564 | 571 | 578 | 579 | 580 | 583 |
| | 590 | 596 | 597 | 598 | 601 | 605 | 606 | 613 | 614 | 615 |
| | 616 | 617 | 624 | 625 | 626 | 635 | 636 | 643 | 644 | 646 |
| | 650 | 654 | 657 | 658 | 663 | 664 | 665 | 666 | 672 | 673 |
| | 674 | 682 | 683 | 689 | 690 | 691 | 692 | 694 | 696 | 698 |
| | 699 | 702 | 707 | | | | | | | |
| | | | | | | | | | | |

| SEQ ID NO:19: | | | | | | | | | | |
|---|---|---|---|---|---|---|---|---|---|---|
| | | | | | | | | | | |
| | 511 | 512 | 516 | 517 | 524 | 542 | 543 | 544 | 545 | 546 |
| | 547 | 548 | 551 | 555 | 556 | 563 | 570 | 571 | 572 | 575 |
| | 582 | 588 | 589 | 590 | 593 | 597 | 598 | 605 | 606 | 607 |
| | 608 | 609 | 616 | 617 | 618 | 627 | 628 | 635 | 636 | 638 |
| | 642 | 646 | 649 | 650 | 655 | 656 | 657 | 658 | 664 | 665 |
| | 666 | 674 | 675 | 681 | 682 | 683 | 684 | 686 | 688 | 690 |
| | 691 | 694 | 699 | | | | | | | |
| | | | | | | | | | | |

| SEQ ID NO:20: | | | | | | | | | | |
|---|---|---|---|---|---|---|---|---|---|---|
| | | | | | | | | | | |
| | 514 | 515 | 519 | 520 | 527 | 545 | 546 | 547 | 548 | 549 |
| | 550 | 551 | 554 | 558 | 559 | 566 | 573 | 574 | 575 | 578 |
| | 585 | 591 | 592 | 593 | 596 | 600 | 601 | 608 | 609 | 610 |
| | 611 | 612 | 619 | 620 | 621 | 630 | 631 | 638 | 639 | 641 |
| | 645 | 649 | 652 | 653 | 658 | 659 | 660 | 661 | 667 | 668 |
| | 669 | 677 | 678 | 684 | 685 | 686 | 687 | 695 | 698 | 700 |
| | 701 | 704 | 709 | | | | | | | |
| | | | | | | | | | | |

| SEQ ID NO:21: | | | | | | | | | | |
|---|---|---|---|---|---|---|---|---|---|---|
| | | | | | | | | | | |
| | 532 | 533 | 537 | 538 | 545 | 563 | 564 | 565 | 566 | 567 |
| | 568 | 569 | 572 | 576 | 577 | 584 | 591 | 592 | 593 | 596 |
| | 603 | 609 | 610 | 611 | 614 | 618 | 619 | 626 | 627 | 628 |
| | 629 | 630 | 637 | 638 | 639 | 648 | 649 | 656 | 657 | 659 |
| | 663 | 667 | 670 | 671 | 676 | 677 | 678 | 679 | 685 | 686 |
| | 687 | 695 | 696 | 702 | 703 | 704 | 705 | 707 | 709 | 711 |
| | 712 | 715 | 720 | | | | | | | |
| | | | | | | | | | | |

| SEQ ID NO:22: | | | | | | | | | | |
|---|---|---|---|---|---|---|---|---|---|---|
| | | | | | | | | | | |
| | 287 | 288 | 291 | 294 | 301 | 316 | 317 | 318 | 319 | 320 |
| | 321 | 324 | 328 | 329 | 336 | 343 | 344 | 345 | 348 | 355 |
| | 361 | 363 | 364 | 367 | 372 | 373 | 380 | 381 | 382 | 383 |
| | 384 | 391 | 392 | 393 | 394 | 403 | 404 | 411 | 412 | 414 |
| | 418 | 422 | 425 | 426 | 431 | 432 | 433 | 434 | 440 | 441 |
| | 450 | 451 | 457 | 458 | 459 | 460 | 462 | 464 | 466 | 467 |
| | 470 | 474 | | | | | | | | |
| | | | | | | | | | | |

| SEQ ID NO:23: | | | | | | | | | | |
|---|---|---|---|---|---|---|---|---|---|---|
| | | | | | | | | | | |
| | 287 | 288 | 291 | 294 | 301 | 316 | 317 | 318 | 319 | 320 |
| | 321 | 324 | 328 | 329 | 336 | 343 | 344 | 345 | 348 | 355 |
| | 361 | 363 | 364 | 367 | 372 | 373 | 380 | 381 | 382 | 383 |
| | 384 | 391 | 392 | 393 | 394 | 403 | 404 | 411 | 412 | 414 |
| | 418 | 422 | 425 | 426 | 431 | 432 | 433 | 434 | 440 | 441 |
| | 450 | 451 | 457 | 458 | 459 | 460 | 462 | 464 | 466 | 467 |
| | 470 | 474 | | | | | | | | |
| | | | | | | | | | | |

| SEQ ID NO:24: | | | | | | | | | | |
|---|---|---|---|---|---|---|---|---|---|---|
| | | | | | | | | | | |
| | 292 | 293 | 296 | 297 | 304 | 322 | 323 | 324 | 325 | 326 |
| | 327 | 330 | 334 | 335 | 342 | 349 | 350 | 351 | 353 | 360 |
| | 366 | 368 | 369 | 372 | 376 | 377 | 384 | 385 | 386 | 387 |
| | 388 | 395 | 396 | 397 | 406 | 407 | 414 | 415 | 417 | 420 |
| | 424 | 427 | 428 | 433 | 434 | 435 | 436 | 442 | 443 | 444 |
| | 445 | 453 | 454 | 460 | 461 | 462 | 463 | 465 | 467 | 469 |
| | 470 | 473 | 478 | | | | | | | |
| | | | | | | | | | | |

| SEQ ID NO:25: | | | | | | | | | | |
|---|---|---|---|---|---|---|---|---|---|---|
| | | | | | | | | | | |
| | 296 | 297 | 300 | 301 | 308 | 326 | 327 | 328 | 329 | 330 |
| | 331 | 334 | 338 | 339 | 346 | 353 | 354 | 355 | 357 | 364 |
| | 370 | 372 | 373 | 376 | 380 | 381 | 388 | 389 | 390 | 391 |
| | 392 | 399 | 400 | 401 | 410 | 411 | 418 | 419 | 421 | 424 |
| | 428 | 431 | 432 | 437 | 438 | 439 | 440 | 446 | 447 | 448 |
| | 449 | 457 | 458 | 464 | 465 | 466 | 467 | 469 | 471 | 473 |
| | 474 | 477 | 482 | | | | | | | |
| | | | | | | | | | | |

| SEQ ID NO:26: | | | | | | | | | | |
|---|---|---|---|---|---|---|---|---|---|---|
| | | | | | | | | | | |
| | 280 | 281 | 285 | 286 | 287 | 294 | 311 | 312 | 313 | 314 |
| | 315 | 316 | 319 | 323 | 324 | 331 | 338 | 339 | 340 | 343 |
| | 350 | 356 | 358 | 359 | 362 | 367 | 368 | 375 | 376 | 377 |
| | 378 | 379 | 386 | 387 | 388 | 389 | 398 | 399 | 406 | 407 |
| | 409 | 413 | 417 | 420 | 421 | 426 | 427 | 428 | 429 | 435 |
| | 436 | 437 | 446 | 447 | 453 | 454 | 455 | 456 | 458 | 460 |
| | 462 | 463 | 466 | 470 | | | | | | |
| | | | | | | | | | | |

| SEQ ID NO:27: | | | | | | | | | | |
|---|---|---|---|---|---|---|---|---|---|---|
| | | | | | | | | | | |
| | 273 | 274 | 278 | 279 | 280 | 287 | 304 | 305 | 306 | 307 |
| | 308 | 309 | 312 | 316 | 317 | 324 | 331 | 332 | 333 | 336 |
| | 343 | 349 | 351 | 352 | 355 | 360 | 361 | 368 | 369 | 370 |
| | 371 | 372 | 379 | 380 | 381 | 382 | 391 | 392 | 399 | 400 |
| | 402 | 406 | 410 | 413 | 414 | 419 | 420 | 421 | 422 | 428 |
| | 429 | 438 | 439 | 445 | 446 | 447 | 448 | 450 | 452 | 454 |
| | 455 | 458 | 462 | | | | | | | |
| | | | | | | | | | | |

| SEQ ID NO:28: | | | | | | | | | | |
|---|---|---|---|---|---|---|---|---|---|---|
| | | | | | | | | | | |
| | 275 | 276 | 280 | 281 | 282 | 289 | 306 | 307 | 308 | 309 |
| | 310 | 311 | 314 | 318 | 319 | 326 | 333 | 334 | 335 | 338 |
| | 345 | 351 | 353 | 354 | 357 | 362 | 363 | 370 | 371 | 372 |
| | 373 | 374 | 381 | 382 | 383 | 384 | 393 | 394 | 401 | 402 |
| | 404 | 408 | 412 | 415 | 416 | 421 | 422 | 423 | 424 | 430 |
| | 431 | 440 | 441 | 447 | 448 | 449 | 450 | 452 | 454 | 456 |
| | 457 | 460 | 464 | | | | | | | |
| | | | | | | | | | | |

| SEQ ID NO:29: | | | | | | | | | | |
|---|---|---|---|---|---|---|---|---|---|---|
| | | | | | | | | | | |
| | 276 | 277 | 281 | 282 | 283 | 290 | 307 | 308 | 309 | 310 |
| | 311 | 312 | 315 | 319 | 320 | 327 | 334 | 335 | 336 | 339 |
| | 346 | 352 | 354 | 355 | 358 | 363 | 364 | 371 | 372 | 373 |
| | 374 | 375 | 382 | 383 | 384 | 385 | 394 | 395 | 402 | 403 |
| | 405 | 409 | 413 | 416 | 417 | 422 | 423 | 424 | 425 | 431 |
| | 432 | 441 | 442 | 448 | 449 | 450 | 451 | 453 | 455 | 457 |
| | 458 | 461 | 465 | | | | | | | |
| | | | | | | | | | | |

| SEQ ID NO:30: | | | | | | | | | | |
|---|---|---|---|---|---|---|---|---|---|---|
| | | | | | | | | | | |
| | 282 | 283 | 287 | 288 | 289 | 296 | 313 | 314 | 315 | 316 |
| | 317 | 318 | 321 | 325 | 326 | 333 | 340 | 341 | 342 | 345 |
| | 352 | 358 | 360 | 361 | 364 | 369 | 370 | 377 | 378 | 379 |
| | 380 | 381 | 388 | 389 | 390 | 391 | 400 | 401 | 408 | 409 |
| | 411 | 415 | 419 | 422 | 423 | 428 | 429 | 430 | 431 | 437 |
| | 438 | 447 | 448 | 454 | 455 | 456 | 457 | 459 | 461 | 463 |
| | 464 | 467 | 471 | | | | | | | |
| | | | | | | | | | | |

| SEQ ID NO:31: | | | | | | | | | | |
|---|---|---|---|---|---|---|---|---|---|---|
| | | | | | | | | | | |
| | 283 | 284 | 288 | 289 | 290 | 297 | 314 | 315 | 316 | 317 |
| | 318 | 319 | 322 | 326 | 327 | 334 | 341 | 342 | 343 | 346 |
| | 353 | 359 | 361 | 362 | 365 | 370 | 371 | 378 | 379 | 380 |
| | 381 | 382 | 389 | 390 | 391 | 392 | 401 | 402 | 409 | 410 |
| | 412 | 416 | 420 | 423 | 424 | 429 | 430 | 431 | 432 | 438 |
| | 439 | 448 | 449 | 455 | 456 | 457 | 458 | 460 | 462 | 464 |
| | 465 | 468 | 472 | | | | | | | |
| | | | | | | | | | | |

| SEQ ID NO:32: | | | | | | | | | | |
|---|---|---|---|---|---|---|---|---|---|---|
| | | | | | | | | | | |
| | 282 | 283 | 287 | 288 | 289 | 296 | 313 | 314 | 315 | 316 |
| | 317 | 318 | 321 | 325 | 326 | 333 | 340 | 341 | 342 | 345 |
| | 352 | 358 | 360 | 361 | 364 | 369 | 370 | 377 | 378 | 379 |
| | 380 | 381 | 388 | 389 | 390 | 391 | 400 | 401 | 408 | 409 |
| | 411 | 415 | 419 | 422 | 423 | 428 | 429 | 430 | 431 | 437 |
| | 438 | 447 | 448 | 454 | 455 | 456 | 457 | 459 | 461 | 463 |
| | 464 | 467 | 471 | | | | | | | |
| | | | | | | | | | | |

| SEQ ID NO:33: | | | | | | | | | | |
|---|---|---|---|---|---|---|---|---|---|---|
| | | | | | | | | | | |
| | 282 | 283 | 287 | 288 | 289 | 296 | 313 | 314 | 315 | 316 |
| | 317 | 318 | 321 | 325 | 326 | 333 | 340 | 341 | 342 | 345 |
| | 352 | 358 | 360 | 361 | 364 | 369 | 370 | 377 | 378 | 379 |
| | 380 | 381 | 388 | 389 | 390 | 391 | 400 | 401 | 408 | 409 |
| | 411 | 415 | 419 | 422 | 423 | 428 | 429 | 430 | 431 | 437 |
| | 438 | 447 | 448 | 454 | 455 | 456 | 457 | 459 | 461 | 463 |
| | 464 | 467 | 471 | | | | | | | |
| | | | | | | | | | | |

| SEQ ID NO:34: | | | | | | | | | | |
|---|---|---|---|---|---|---|---|---|---|---|
| | | | | | | | | | | |
| | 317 | 318 | 322 | 329 | 347 | 348 | 349 | 350 | 351 | 352 |
| | 355 | 359 | 360 | 367 | 374 | 375 | 378 | 385 | 391 | 393 |
| | 394 | 397 | 402 | 403 | 411 | 412 | 413 | 414 | 421 | 422 |
| | 423 | 424 | 433 | 434 | 441 | 442 | 444 | 448 | 452 | 454 |
| | 455 | 460 | 461 | 462 | 463 | 469 | 470 | 471 | 472 | 481 |
| | 482 | 488 | 489 | 490 | 491 | 493 | 495 | 497 | 498 | 501 |
| | 506 | | | | | | | | | |
| | | | | | | | | | | |

| SEQ ID NO:35: | | | | | | | | | | |
|---|---|---|---|---|---|---|---|---|---|---|
| | | | | | | | | | | |
| | 317 | 318 | 322 | 329 | 347 | 348 | 349 | 350 | 351 | 352 |
| | 355 | 359 | 360 | 367 | 374 | 375 | 378 | 385 | 391 | 393 |
| | 394 | 397 | 402 | 403 | 411 | 412 | 413 | 414 | 421 | 422 |
| | 423 | 424 | 433 | 434 | 441 | 442 | 444 | 448 | 452 | 454 |
| | 455 | 460 | 461 | 462 | 463 | 469 | 470 | 471 | 472 | 481 |
| | 482 | 488 | 489 | 490 | 491 | 493 | 495 | 497 | 498 | 501 |
| | 506 | | | | | | | | | |
| | | | | | | | | | | |

| SEQ ID NO:36: | | | | | | | | | | |
|---|---|---|---|---|---|---|---|---|---|---|
| | | | | | | | | | | |
| | 315 | 316 | 320 | 327 | 345 | 346 | 347 | 348 | 349 | 350 |
| | 353 | 357 | 358 | 365 | 372 | 373 | 376 | 383 | 389 | 391 |
| | 392 | 395 | 400 | 401 | 409 | 410 | 411 | 412 | 419 | 420 |
| | 421 | 422 | 431 | 432 | 439 | 440 | 442 | 446 | 450 | 452 |
| | 453 | 458 | 459 | 460 | 461 | 467 | 468 | 469 | 470 | 479 |
| | 480 | 486 | 487 | 488 | 489 | 491 | 493 | 495 | 496 | 499 |
| | 504 | | | | | | | | | |
| | | | | | | | | | | |

| SEQ ID NO:37: | | | | | | | | | | |
|---|---|---|---|---|---|---|---|---|---|---|
| | | | | | | | | | | |
| | 317 | 318 | 322 | 329 | 347 | 348 | 349 | 350 | 351 | 352 |
| | 355 | 359 | 360 | 367 | 374 | 375 | 378 | 385 | 391 | 393 |
| | 394 | 397 | 402 | 403 | 411 | 412 | 413 | 414 | 421 | 422 |
| | 423 | 424 | 433 | 434 | 441 | 442 | 444 | 448 | 452 | 454 |
| | 455 | 460 | 461 | 462 | 463 | 469 | 470 | 471 | 472 | 481 |
| | 482 | 488 | 489 | 490 | 491 | 493 | 495 | 497 | 498 | 501 |
| | 506 | | | | | | | | | |
| | | | | | | | | | | |

| SEQ ID NO:38: | | | | | | | | | | |
|---|---|---|---|---|---|---|---|---|---|---|
| | | | | | | | | | | |
| | 317 | 318 | 322 | 329 | 347 | 348 | 349 | 350 | 351 | 352 |
| | 355 | 359 | 360 | 367 | 374 | 375 | 378 | 385 | 391 | 393 |
| | 394 | 397 | 402 | 403 | 411 | 412 | 413 | 414 | 421 | 422 |
| | 423 | 424 | 433 | 434 | 441 | 442 | 444 | 448 | 452 | 454 |
| | 455 | 460 | 461 | 462 | 463 | 469 | 470 | 471 | 472 | 481 |
| | 482 | 488 | 489 | 490 | 491 | 493 | 495 | 497 | 498 | 501 |
| | 506 | | | | | | | | | |
| | | | | | | | | | | |

| SEQ ID NO:39: | | | | | | | | | | |
|---|---|---|---|---|---|---|---|---|---|---|
| | | | | | | | | | | |
| | 368 | 369 | 373 | 380 | 398 | 399 | 400 | 401 | 402 | 403 |
| | 406 | 410 | 411 | 418 | 425 | 426 | 429 | 436 | 442 | 444 |
| | 445 | 448 | 453 | 454 | 462 | 463 | 464 | 465 | 472 | 473 |
| | 474 | 475 | 484 | 485 | 492 | 493 | 495 | 499 | 503 | 505 |
| | 506 | 511 | 512 | 513 | 514 | 520 | 521 | 522 | 523 | 532 |
| | 533 | 539 | 540 | 541 | 542 | 544 | 546 | 548 | 549 | 552 |
| | 557 | | | | | | | | | |
| | | | | | | | | | | |

| SEQ ID NO:40: | | | | | | | | | | |
|---|---|---|---|---|---|---|---|---|---|---|
| | | | | | | | | | | |
| | 364 | 365 | 369 | 376 | 394 | 395 | 396 | 397 | 398 | 399 |
| | 402 | 406 | 407 | 415 | 422 | 423 | 426 | 433 | 439 | 441 |
| | 442 | 445 | 450 | 451 | 459 | 460 | 461 | 462 | 469 | 470 |
| | 471 | 472 | 481 | 482 | 489 | 490 | 492 | 496 | 500 | 502 |
| | 503 | 508 | 509 | 510 | 511 | 517 | 518 | 519 | 520 | 529 |
| | 530 | 536 | 537 | 538 | 539 | 541 | 543 | 545 | 546 | 549 |
| | 554 | | | | | | | | | |
| | | | | | | | | | | |

| SEQ ID NO:41: | | | | | | | | | | |
|---|---|---|---|---|---|---|---|---|---|---|
| | | | | | | | | | | |
| | 341 | 342 | 346 | 353 | 371 | 372 | 373 | 374 | 375 | 376 |
| | 379 | 383 | 384 | 391 | 398 | 399 | 402 | 409 | 415 | 417 |
| | 418 | 421 | 426 | 427 | 435 | 436 | 437 | 438 | 445 | 446 |
| | 447 | 448 | 457 | 458 | 465 | 466 | 468 | 472 | 476 | 478 |
| | 479 | 484 | 485 | 486 | 487 | 493 | 494 | 495 | 496 | 505 |
| | 506 | 512 | 513 | 514 | 515 | 517 | 519 | 521 | 522 | 525 |
| | 530 | | | | | | | | | |
| | | | | | | | | | | |

| SEQ ID NO:42: | | | | | | | | | | |
|---|---|---|---|---|---|---|---|---|---|---|
| | | | | | | | | | | |
| | 341 | 342 | 346 | 353 | 371 | 372 | 373 | 374 | 375 | 376 |
| | 379 | 383 | 384 | 391 | 398 | 399 | 402 | 409 | 415 | 417 |
| | 418 | 421 | 426 | 427 | 435 | 436 | 437 | 438 | 445 | 446 |
| | 447 | 448 | 457 | 458 | 465 | 466 | 468 | 472 | 476 | 478 |
| | 479 | 484 | 485 | 486 | 487 | 493 | 494 | 495 | 496 | 505 |
| | 506 | 512 | 513 | 514 | 515 | 517 | 519 | 521 | 522 | 525 |
| | 530 | | | | | | | | | |
| | | | | | | | | | | |

| SEQ ID NO:43: | | | | | | | | | | |
|---|---|---|---|---|---|---|---|---|---|---|
| | | | | | | | | | | |
| | 341 | 342 | 346 | 353 | 371 | 372 | 373 | 374 | 375 | 376 |
| | 379 | 383 | 384 | 391 | 398 | 399 | 402 | 409 | 415 | 417 |
| | 418 | 421 | 426 | 427 | 435 | 436 | 437 | 438 | 445 | 446 |
| | 447 | 448 | 457 | 458 | 465 | 466 | 468 | 472 | 476 | 478 |
| | 479 | 484 | 485 | 486 | 487 | 493 | 494 | 495 | 496 | 505 |
| | 506 | 512 | 513 | 514 | 515 | 517 | 519 | 521 | 522 | 525 |
| | 530 | | | | | | | | | |
| | | | | | | | | | | |

| SEQ ID NO:44: | | | | | | | | | | |
|---|---|---|---|---|---|---|---|---|---|---|
| | | | | | | | | | | |
| | 333 | 334 | 338 | 345 | 363 | 364 | 365 | 366 | 367 | 368 |
| | 369 | 372 | 376 | 377 | 387 | 394 | 395 | 396 | 399 | 406 |
| | 412 | 414 | 415 | 418 | 423 | 424 | 430 | 431 | 432 | 433 |
| | 440 | 441 | 442 | 443 | 452 | 453 | 460 | 461 | 463 | 467 |
| | 471 | 473 | 474 | 479 | 480 | 481 | 482 | 488 | 489 | 490 |
| | 491 | 499 | 500 | 506 | 507 | 508 | 509 | 511 | 513 | 515 |
| | 516 | 519 | 524 | | | | | | | |
| | | | | | | | | | | |

| SEQ ID NO:45: | | | | | | | | | | |
|---|---|---|---|---|---|---|---|---|---|---|
| | | | | | | | | | | |
| | 334 | 335 | 339 | 346 | 364 | 365 | 366 | 367 | 368 | 369 |
| | 370 | 373 | 377 | 378 | 388 | 395 | 396 | 397 | 400 | 407 |
| | 413 | 415 | 416 | 419 | 424 | 425 | 431 | 432 | 433 | 434 |
| | 441 | 442 | 443 | 444 | 453 | 454 | 461 | 462 | 464 | 468 |
| | 472 | 474 | 475 | 480 | 481 | 482 | 483 | 489 | 490 | 491 |
| | 492 | 500 | 501 | 507 | 508 | 509 | 510 | 512 | 514 | 516 |
| | 517 | 520 | 525 | | | | | | | |
| | | | | | | | | | | |

| SEQ ID NO:46: | | | | | | | | | | |
|---|---|---|---|---|---|---|---|---|---|---|
| | | | | | | | | | | |
| | 278 | 279 | 283 | 284 | 285 | 292 | 309 | 310 | 311 | 312 |
| | 313 | 314 | 317 | 321 | 322 | 329 | 336 | 337 | 338 | 341 |
| | 348 | 354 | 356 | 357 | 360 | 365 | 366 | 373 | 374 | 375 |
| | 376 | 377 | 384 | 385 | 386 | 387 | 396 | 397 | 404 | 405 |
| | 407 | 411 | 415 | 418 | 419 | 424 | 425 | 426 | 427 | 433 |
| | 434 | 443 | 444 | 450 | 451 | 452 | 453 | 455 | 457 | 459 |
| | 460 | 463 | 467 | | | | | | | |
| | | | | | | | | | | |

| SEQ ID NO:47: | | | | | | | | | | |
|---|---|---|---|---|---|---|---|---|---|---|
| | | | | | | | | | | |
| | 287 | 288 | 292 | 293 | 294 | 300 | 318 | 319 | 320 | 321 |
| | 322 | 324 | 328 | 329 | 336 | 343 | 344 | 345 | 348 | 355 |
| | 361 | 363 | 364 | 367 | 372 | 373 | 375 | 376 | 377 | 378 |
| | 379 | 386 | 387 | 388 | 389 | 398 | 399 | 406 | 407 | 409 |
| | 413 | 417 | 420 | 421 | 426 | 427 | 428 | 429 | 435 | 436 |
| | 437 | 437 | 446 | 447 | 453 | 454 | 455 | 456 | 458 | 460 |
| | 462 | 463 | 466 | 471 | | | | | | |
| | | | | | | | | | | |

| SEQ ID NO:48: | | | | | | | | | | |
|---|---|---|---|---|---|---|---|---|---|---|
| | | | | | | | | | | |
| | 287 | 288 | 292 | 293 | 294 | 300 | 318 | 319 | 320 | 321 |
| | 322 | 324 | 328 | 329 | 336 | 343 | 344 | 345 | 348 | 355 |
| | 361 | 363 | 364 | 367 | 372 | 373 | 375 | 376 | 377 | 378 |
| | 379 | 386 | 387 | 388 | 389 | 398 | 399 | 406 | 407 | 409 |
| | 413 | 417 | 420 | 421 | 426 | 427 | 428 | 429 | 435 | 436 |
| | 437 | 446 | 447 | 453 | 454 | 455 | 456 | 458 | 460 | 462 |
| | 463 | 466 | 471 | | | | | | | |
| | | | | | | | | | | |

| SEQ ID NO:49: | | | | | | | | | | |
|---|---|---|---|---|---|---|---|---|---|---|
| | | | | | | | | | | |
| | 292 | 293 | 297 | 298 | 299 | 306 | 323 | 324 | 325 | 326 |
| | 327 | 328 | 329 | 332 | 336 | 337 | 344 | 351 | 352 | 353 |
| | 356 | 363 | 369 | 371 | 372 | 374 | 379 | 380 | 387 | 388 |
| | 389 | 390 | 391 | 398 | 399 | 400 | 401 | 410 | 411 | 418 |
| | 419 | 421 | 425 | 429 | 432 | 433 | 438 | 439 | 440 | 441 |
| | 447 | 448 | 457 | 458 | 464 | 465 | 466 | 467 | 469 | 471 |
| | 473 | 474 | 477 | 481 | | | | | | |
| | | | | | | | | | | |

| SEQ ID NO:50: | | | | | | | | | | |
|---|---|---|---|---|---|---|---|---|---|---|
| | | | | | | | | | | |
| | 294 | 295 | 299 | 300 | 301 | 308 | 325 | 326 | 327 | 328 |
| | 329 | 330 | 331 | 334 | 338 | 339 | 346 | 353 | 354 | 355 |
| | 358 | 365 | 371 | 373 | 374 | 376 | 381 | 382 | 389 | 390 |
| | 391 | 392 | 393 | 400 | 401 | 402 | 403 | 412 | 413 | 420 |
| | 421 | 423 | 427 | 431 | 434 | 435 | 440 | 441 | 442 | 443 |
| | 449 | 450 | 459 | 460 | 466 | 467 | 468 | 469 | 471 | 473 |
| | 475 | 476 | 479 | 483 | | | | | | |
| | | | | | | | | | | |

| SEQ ID NO:51: | | | | | | | | | | |
|---|---|---|---|---|---|---|---|---|---|---|
| | | | | | | | | | | |
| | 293 | 294 | 298 | 299 | 300 | 307 | 324 | 325 | 326 | 327 |
| | 328 | 329 | 330 | 333 | 337 | 338 | 345 | 352 | 353 | 354 |
| | 357 | 364 | 370 | 372 | 373 | 375 | 380 | 381 | 388 | 389 |
| | 390 | 391 | 392 | 399 | 400 | 401 | 402 | 411 | 412 | 419 |
| | 420 | 422 | 426 | 430 | 433 | 434 | 439 | 440 | 441 | 442 |
| | 448 | 449 | 458 | 459 | 465 | 466 | 467 | 468 | 470 | 472 |
| | 474 | 475 | 478 | 482 | | | | | | |
| | | | | | | | | | | |

| SEQ ID NO:52: | | | | | | | | | | |
|---|---|---|---|---|---|---|---|---|---|---|
| | | | | | | | | | | |
| | 294 | 295 | 299 | 300 | 301 | 308 | 325 | 326 | 327 | 328 |
| | 329 | 330 | 331 | 334 | 338 | 339 | 346 | 353 | 354 | 355 |
| | 358 | 365 | 371 | 373 | 374 | 376 | 381 | 382 | 389 | 390 |
| | 391 | 392 | 393 | 400 | 401 | 402 | 403 | 412 | 413 | 420 |
| | 421 | 423 | 427 | 431 | 434 | 435 | 440 | 441 | 442 | 443 |
| | 449 | 450 | 459 | 460 | 466 | 467 | 468 | 469 | 471 | 473 |
| | 475 | 476 | 479 | 483 | | | | | | |
| | | | | | | | | | | |

| SEQ ID NO:53: | | | | | | | | | | |
|---|---|---|---|---|---|---|---|---|---|---|
| | | | | | | | | | | |
| | 378 | 379 | 383 | 390 | 408 | 409 | 410 | 411 | 412 | 413 |
| | 416 | 420 | 421 | 428 | 435 | 436 | 439 | 446 | 452 | 454 |
| | 455 | 458 | 463 | 464 | 472 | 473 | 474 | 475 | 482 | 483 |
| | 484 | 485 | 494 | 495 | 502 | 503 | 505 | 509 | 513 | 515 |
| | 516 | 521 | 522 | 523 | 524 | 530 | 531 | 532 | 533 | 542 |
| | 543 | 549 | 550 | 551 | 552 | 554 | 556 | 558 | 559 | 562 |
| | 567 | | | | | | | | | |
| | | | | | | | | | | |

| SEQ ID NO:54: | | | | | | | | | | |
|---|---|---|---|---|---|---|---|---|---|---|
| | | | | | | | | | | |
| | 351 | 352 | 356 | 363 | 381 | 382 | 383 | 384 | 385 | 386 |
| | 389 | 393 | 394 | 401 | 408 | 409 | 412 | 419 | 425 | 427 |
| | 428 | 431 | 436 | 437 | 445 | 446 | 447 | 448 | 455 | 456 |
| | 457 | 458 | 467 | 468 | 475 | 476 | 478 | 482 | 486 | 488 |
| | 489 | 494 | 495 | 496 | 497 | 503 | 504 | 505 | 506 | 515 |
| | 516 | 522 | 523 | 524 | 525 | 527 | 529 | 531 | 532 | 535 |
| | 540 | | | | | | | | | |
| | | | | | | | | | | |

| SEQ ID NO:55: | | | | | | | | | | |
|---|---|---|---|---|---|---|---|---|---|---|
| | | | | | | | | | | |
| | 351 | 352 | 356 | 363 | 381 | 382 | 383 | 384 | 385 | 386 |
| | 389 | 393 | 394 | 401 | 408 | 409 | 412 | 419 | 425 | 427 |
| | 428 | 431 | 436 | 437 | 445 | 446 | 447 | 448 | 455 | 456 |
| | 457 | 458 | 467 | 468 | 475 | 476 | 478 | 482 | 486 | 488 |
| | 489 | 494 | 495 | 496 | 497 | 503 | 504 | 505 | 506 | 515 |
| | 516 | 522 | 523 | 524 | 525 | 527 | 529 | 531 | 532 | 535 |
| | 540 | | | | | | | | | |
| | | | | | | | | | | |

| SEQ ID NO:56: | | | | | | | | | | |
|---|---|---|---|---|---|---|---|---|---|---|
| | | | | | | | | | | |
| | 315 | 316 | 320 | 327 | 345 | 346 | 347 | 348 | 349 | 350 |
| | 351 | 354 | 358 | 359 | 369 | 376 | 377 | 378 | 381 | 388 |
| | 394 | 396 | 397 | 400 | 404 | 405 | 411 | 412 | 413 | 414 |
| | 415 | 422 | 423 | 424 | 425 | 434 | 435 | 442 | 443 | 445 |
| | 449 | 453 | 455 | 456 | 461 | 462 | 463 | 464 | 470 | 471 |
| | 472 | 473 | 481 | 482 | 488 | 489 | 490 | 491 | 493 | 495 |
| | 497 | 498 | 501 | 506 | | | | | | |
| | | | | | | | | | | |

| SEQ ID NO:57: | | | | | | | | | | |
|---|---|---|---|---|---|---|---|---|---|---|
| | | | | | | | | | | |
| | 327 | 334 | 353 | 354 | 355 | 356 | 357 | 360 | 364 | 365 |
| | 372 | 379 | 380 | 381 | 384 | 391 | 397 | 399 | 400 | 403 |
| | 408 | 409 | 413 | 414 | 415 | 416 | 417 | 424 | 425 | 426 |
| | 427 | 436 | 437 | 444 | 445 | 447 | 451 | 455 | 457 | 458 |
| | 463 | 464 | 465 | 466 | 472 | 473 | 474 | 483 | 484 | 490 |
| | 491 | 492 | 493 | 495 | 497 | 499 | 500 | 503 | 508 | |
| | | | | | | | | | | |

| SEQ ID NO:58: | | | | | | | | | | |
|---|---|---|---|---|---|---|---|---|---|---|
| | | | | | | | | | | |
| | 291 | 292 | 296 | 297 | 298 | 305 | 323 | 324 | 325 | 326 |
| | 327 | 330 | 334 | 335 | 341 | 345 | 346 | 347 | 350 | 357 |
| | 363 | 365 | 366 | 369 | 374 | 375 | 383 | 384 | 385 | 386 |
| | 387 | 390 | 391 | 392 | 393 | 400 | 407 | 408 | 410 | 414 |
| | 418 | 421 | 422 | 427 | 428 | 429 | 430 | 432 | 439 | 440 |
| | 446 | 447 | 448 | 449 | 453 | 456 | 458 | 459 | 462 | 467 |
| | | | | | | | | | | |

| SEQ ID NO:59: | | | | | | | | | | |
|---|---|---|---|---|---|---|---|---|---|---|
| | | | | | | | | | | |
| | 290 | 291 | 295 | 296 | 297 | 304 | 322 | 323 | 324 | 325 |
| | 326 | 329 | 333 | 334 | 340 | 344 | 345 | 346 | 349 | 356 |
| | 362 | 364 | 365 | 368 | 373 | 374 | 382 | 383 | 384 | 385 |
| | 386 | 391 | 392 | 393 | 394 | 401 | 408 | 409 | 411 | 415 |
| | 419 | 422 | 423 | 428 | 429 | 430 | 431 | 433 | | 440 |
| | 441 | 447 | 448 | 449 | 450 | 454 | 457 | 459 | 460 | 463 |
| | 468 | | | | | | | | | |
| | | | | | | | | | | |

| SEQ ID NO:60: | | | | | | | | | | |
|---|---|---|---|---|---|---|---|---|---|---|
| | | | | | | | | | | |
| | 268 | 269 | 273 | 274 | 275 | 282 | 300 | 301 | 302 | 303 |
| | 304 | 307 | 311 | 312 | 318 | 322 | 323 | 324 | 327 | 334 |
| | 340 | 342 | 343 | 346 | 351 | 352 | 360 | 361 | 362 | 363 |
| | 364 | 371 | 372 | 373 | 374 | 381 | 388 | 389 | 391 | 395 |
| | 399 | 402 | 403 | 408 | 409 | 410 | 411 | 413 | 420 | 421 |
| | 427 | 428 | 429 | 430 | 434 | 437 | 439 | 440 | 443 | 448 |
| | | | | | | | | | | |

| SEQ ID NO:61: | | | | | | | | | | |
|---|---|---|---|---|---|---|---|---|---|---|
| | | | | | | | | | | |
| | 291 | 292 | 296 | 297 | 298 | 305 | 323 | 324 | 325 | 326 |
| | 327 | 330 | 334 | 335 | 341 | 345 | 346 | 347 | 350 | 357 |
| | 362 | 364 | 365 | 368 | 373 | 374 | 380 | 381 | 382 | 383 |
| | 384 | 387 | 388 | 389 | 390 | 397 | 404 | 405 | 407 | 411 |
| | 415 | 418 | 419 | 424 | 425 | 426 | 427 | 429 | 436 | 437 |
| | 443 | 444 | 445 | 446 | 450 | 453 | 455 | 456 | 459 | 464 |
| | | | | | | | | | | |

Positions with a high diversity number, i.e. equal or higher than 8, or even 10, have also been determined. The analysis revealed that these are mainly located in loop regions. These expose a high variability, i.e. flexibility, and as a result spatially bring together several surface exposed amino acids from the blade connecting loops. The results also suggest not using the interior surface of the tunnel for randomization experiments. The inner three beta-sheets of each blade were also critical, because they resemble a high conservation and contributed to the core stability of the protein. Thus, solvent-exposed amino acids, which do not contribute to the hydrophobic core stability of the protein, which revealed a sufficient high diversity number and hence a low conservation, are in the focus of interest for a mutagenesis approach.

With this method it is possible to obtain a list of variable, i.e. alterable, amino acid positions in and for all proteins, which have been employed in the alignment, at the same time. For the hemopexin-like domain, as exemplified before, hemopexin-like domains of sixty proteins have been employed and thus for all sixty domains the positions of alterable, i.e. variable, amino acids have been identified. These positions are listed in table V (the numbering is according to the full length polypeptide/protein).

In table V the variable amino acid positions in the sixty hemopexin-like domains (SEQ ID NO:02 to SEQ ID NO:61) are listed. The amino acid positions are numbered according to the full length sequence of the protein containing the hemopexin-like domain. For example, for the hemopexin-like domain according to SEQ ID NO:02 these are the amino acid positions listed after the subheading SEQ ID NO:02 of table V and are accordingly 470, 471, 475, 476, 477, 484, 501, 502, 503, 504, 505, 506, 507, 510, 514, 515, 522, 529, 530, 531, 534, 541, 547, 549, 550, 553, 558, 559, 566, 567, 568, 569, 570, 577, 578, 579, 580, 589, 590, 597, 598, 600, 604, 608, 611, 612, 617, 618, 619, 620, 626, 627, 628, 629, 638, 639, 645, 646, 647, 648, 650, 652, 654, 655, 658, 663. The alterable amino acid positions for SEQ ID NO:03 to SEQ ID NO:61 are accordingly listed in table V after the respective subheading.

With the alterable amino acid positions available for SEQ ID NO:01 to SEQ ID NO:61 each of these sequences can be taken as starting point for further operations.

The cell-free production and analysis of rationally engineered protein variants can be automated, but the library size of rationally designed protein-constructs to be processed remains always limited by the technical throughput of each system. Therefore the analysis of the binding-properties of a vast multitude of gene-products demands for further efforts.

For display and screening of a polypeptide library multiple techniques are available, as e.g. phage-, ribosome- or bacterial-display (Smith, G.P., Science 228 (1985) 1315-1317; Hanes, J., and Pluckthun, A., PNAS 94 (1997) 4937-4942; Stahl, S., and Uhlen, M., TIBTECH 15 (1997) 185-192).

The current invention will be exemplified with the ribosome display technique (see e.g. Hanes, J., and Pluckthun, A., PNAS 94 (1997) 4937-4942; Mattheakis, L.C., et al., PNAS 91 (1994) 9022-9026; He, M., and Taussig, M.J., Nuc. Acids Res. 25 (1997) 5132-5134), but other techniques are also applicable.

Directed evolutionary techniques are well suited to complement the technical capability of a high throughput protein production platform. Based on the cell-free protein synthesis technology, ribosome display is an excellent method to be implemented into a high throughput protein production and analysis process. The aim of ribosome display is the generation of ternary complexes, in which the genotype, characterized by its messenger-RNA (mRNA), is physically linked by the ribosome to its encoded phenotype, characterized by the expressed polypeptides.

For this purpose, a linear DNA-template, which encodes a gene-library is transcribed and translated in vitro. Downstream of the gene-sequence a spacer sequence is fused, where the predominant feature is the lack of a translational stop codon. This spacer domain facilitates the display of the nascent translated and co-translationally folded polypeptide, which remains tethered to the ribosome. These complexes are subjected to a panning procedure, in which the ribosome-displayed polypeptide is allowed to bind to a predetermined ligand molecule. The mRNA from tightly bound complexes is isolated, reversibly transcribed and amplified by PCR. Sub cloning of the PCR products into a vector system and consecutive DNA sequencing reveals information about the genotype related to the phenotype of the bound polypeptide. In repeated cycles of mutagenesis and ribosome display specific protein-binders from libraries in the range of up to 1014 members can be identified (Mattheakis, L.C., et al., PNAS 91 (1994) 9022-9026; Hanes, J., and Pluckthun, A., PNAS 94 (1997) 4937-4942; Lamla, T., and Erdmann, V.A., J. Mol. Biol. 329 (2003) 381-388).

In general, ribosome display requires the stalling of the ribosome while reaching the 3'-end of the mRNA without the dissociation of the ribosomal subunits. After the ribosome has encountered the 3'- end of the mRNA the ribosome's transfer-RNA (tRNA) entry site (A-site) is unoccupied. In prokaryotes, this state results in the activation of the ribosome rescue mechanism, induced by tmRNA (transfer messenger RNA; Abo, T., et al. EMBO J. 19 (2000) 3762-3769; Hayes, C.S., and Sauer, R.T., Mol. Cell. 12 (2003) 903-911; Keiler, K.C., et al., Science 271 (1996) 990-993). With regard to a ribosome display selection, this mechanism lowers the amount of functional ternary complexes and the PCR-product yield is significantly reduced (Hanes, J., and Pluckthun, A., PNAS 94 (1997) 4937-4942).

This tmRNA induced ribosome rescue mechanism can be bypassed, when the ribosome translation machinery has been forced to stall before the 3'-end of the mRNA was encountered by the ribosome. Due to the induced translation arrest the ribosome A-site is still occupied. The display spacer of the ribosome display construct has the sequence as denoted in SEQ ID NO:62. With this spacer the translation can be arrested after the full polypeptide is translated and before the ribosome rescue mechanism is set off.

This has been achieved by removing translation stop codons (Mattheakis, L.C., et al., PNAS 91 (1994) 9022-9026; Hanes, J., and Pluckthun, A., PNAS 94 (1997) 4937-4942) from the DNA spacer sequence of the ribosome display construct. As a consequence a high molecular weight complex consisting of mRNA, the ribosome and the translationally stalled polypeptide is generated.

For the generation of libraries numerous techniques are known to a person skilled in the art. An exemplified proceeding is outlined below.

Linear Expression Elements (LEE) as basis of a DNA-library were produced in a modular manner. To rapidly support the overlapping extension ligation PCR (OEL-PCR) with the randomized DNA-fragments, a library of DNA-modules was pre-produced. In order to obtain sufficient PCR-product yield it was a prerequisite to use HPLC purified primer oligonucleotides and a DNA polymerase with a 3'-5' exonucleolytic activity, producing blunt-end DNA fragments (Garrity, P.A., and Wold, B.J., PNAS 89 (1992) 1021-1025).

Exemplarily, the genes encoding the proteins PEX2 (c-terminal hemopexin-like domain of human matrix metalloproteinase 2), TIMP2 (tissue inhibitor of human matrix metalloproteinase 2), HDAC-I (human histone deacylase I), BirA (E.coli biotin holoenzyme ligase) and GFP (green fluorescent protein) were fused to different combinations of DNA-modules. The concentration of the PCR-products was determined by a comparative densitometric quantification using the LUMI Imager System (Roche Applied Sciences, Mannheim, Germany). The average PCR-product yield of the obtained Linear Expression Elements was about 60 ng/µl ± 20ng/µl (ng per µl of PCR-mixture). Using the P. woesii DNA polymerase (PWO) it was possible to generate LEEs up to 2000 bp in length.

In a comparative example a small library in which 8 amino acid positions of the PEX2 polypeptide were randomized was generated. For this purpose these positions and accordingly the following amino acids were chosen from the list for SEQ ID NO:10 as listed in table V: 528 (Gln), 529 (Glu), 550 (Arg), 576 (Lys), 577 (Asn), 578 (Lys), 594 (Val) and 596 (Lys). The library was generated by template free PCR synthesis as described in example 2. A ribosome display template was assembled e.g. by the modules T7P-g10epsilon-ATG (SEQ ID NO:74), a polypeptide from the generated library and a ribosome display spacer (SEQ ID NO:62).

A prerequisite for a suitable protein scaffold is its capability to stably fold in its active conformation, even under conditions where it has to carry the burden of multiple substituted amino acids. This can be examined by targeting the library versus a known protein-binding partner. In an example the PEX2 library was displayed to recognize the tissue inhibitor of metalloproteinase 2 (TIMP2) protein ligand. The randomized polypeptides from the PEX2-library were still able to recognize their inherent TIMP2 binding partner in a ribosome display approach. This indicated that the structure-function of the scaffold was maintained despite that the scaffold was multiply mutated.

To prepare and optimize the binding properties of a specific binder based on a polypeptide scaffold to a predetermined target molecule, which is not inherently bound by the scaffold, a cycle comprising four main steps has to be passed through several times. These steps are (i) alteration of at least one amino acid position according to table V, (ii) preparation of the display construct, (iii) display and selection of a specific binding variant and (iv) isolation and sequencing of the selected variant. Generally between two and five cycles are necessary to establish new specific binding characteristics in a scaffold.

The predetermined target molecule is not limited to a specific group of polypeptides. The predetermined polypeptide can belong e.g. to one of the groups of hedgehog proteins, bone morphogenetic proteins, growth factors, erythropoietin, thrombopoietin, G-CSF, interleukins and interferons, as well as to the groups of immunoglobulins, enzymes, inhibitors, activators, and cell surface proteins.

In an example, the non-PEX2 binder IGF-I was chosen as predetermined target molecule, for the generation of a specific binder, based on the PEX2 scaffold. The target molecule was plate-presented as a biotinylated ligand. After the second cycle of ribosome display with the PEX2 library a visible PCR-product signal was retained. This shows that the library is well suited for the selection of proteins/polypeptides specifically binding a predetermined target molecule not inherently bound by the protein/polypeptide.

The following examples, references and sequence listings are provided to aid the understanding of the present invention, the true scope of which is set forth in the appended claims.

### Examples

The examples are based on SEQ ID NO 10 (not claimed).

### Example 1

### Overlapping Extension Ligation PCR (OEL-PCR)

Linear Expression Elements were modularly assembled by a two step-PCR protocol, using the overlapping DNA ligation principle. In a standard PWO-PCR an intron-less open reading frame was amplified by sequence-specific terminal bridging primers, which generated overlapping homologous sequences to flanking DNA sequences. Two µl of the first PCR mixture containing approximately 50 ng of the elongated gene-fragment (gene-module) were transferred into a second PWO-PCR mixture. The mixture was supplied with 50 ng to 100 ng of pre-produced DNA-fragments (promotor- and terminator-module) and respective sequence specific, terminal primers at 1 µM each. Typically, this second PCR-step was comprised 30 cycles. The physical parameters of the PCR profiles were adjusted according to the requirements of the DNA-fragments to be ligated.

### Example 2

### Synthesis of the PEX2 DNA library

The PEX2 triplet codons coding for the amino acid coordinates of the hemopexin-like domain 64, 65, 86, 112, 113, 114, 130 and 132 (equal to 528 (Gln), 529 (Glu), 550 (Arg), 576 (Lys), 577 (Asn), 578 (Lys), 594 (Val) and 596 (Lys ) in the full length human matrix metalloproteinase 2) were randomized by NNK-motives. The human wild-type PEX2 DNA sequence was divided up into three sequence sections. A standard PWO-PCR, which was supplied with 10 ng vector-template pIVEX2.1MCS PEX2 and the primers PEX2forw (SEQ ID NO:63) and PEXR4 (SEQ ID NO:64) at 1 µM each amplified the 1 bp - 218 bp fragment. The 402 bp - 605 bp fragment was amplified in a standard PWO-PCR with 10 ng vector-template pIVEX2.1MCS PEX2 and the primers PEXF4 (SEQ ID NO:65) and PEX2rev (SEQ ID NO:66) at 1 µM each. The sequence 196 bp - 432 bp formed overlaps with the DNA fragments 1 bp - 218 bp and 402 bp - 605 bp and was synthesized by template-free PCR with the primers PEXF1 (SEQ ID NO:67) and PEXR1 (SEQ ID NO:68) at 1 µM each and PEXR3 (SEQ ID NO:69), PEXR2 (SEQ ID NO:70) and PEXF2 (SEQ ID NO:71) at 0.25 µM each. The PCR-profile was the same for all three PCRs: TIM (initial melting temperature): 1 min at 94 °C, TM (melting temperature): 20 sec at 94 °C, TA (annealing temperature): 30 sec at 60°C, TE (elongation temperature): 15 sec at 72 °C, 25 cycles, TFE (final elongation temperature): 2 min at 72 °C. The full length randomized PEX2 sequence (588 bp) was obtained when 70 ng of each DNA sequence-fragment was applied to a standard PWO-PCR with the bridging primers T7P_PEX2 (SEQ ID NO:72) and PEX2_RD (SEQ ID NO:73) at 1 µM each. The PCR-profile was: TIM: 1 min at 94 °C, TM: 20 sec at 94 °C, TA: 30 sec at 60 °C, TE: 60 sec at 72 °C, 25 cycles, TFE: 5 min at 72 °C. The bridging primers introduced homologues DNA overlaps for an assembly of the PEX2 gene-library into a ribosome display template by OEL-PCR.

### Example 3

### Cell-free protein in vitro transcription and translation

According to the instructions of the manufacturer, Linear Expression Elements were transcribed and translated in the RTS 100 HY *E.coli* System. Linear DNA template (100 ng - 500 ng) were incubated at 30°C. Optionally 6 µl GroE-supplement (Roche) was added.

### Example 4

### Site-specific biotinylation of fusion proteins

The RTS 100 *E.coli* HY System was modified for the sequence specific, enzymatic biotinylation. Sixty µl RTS mixture were assembled according to the manufacturer's instructions. The mixture was supplemented with 2 µl stock-solution Complete EDTA-Free Protease Inhibitor, 2 µM d-(+)-biotin, 50 ng T7P_BirA_T7T Linear Expression Element (1405 bp), coding for the *E.coli* Biotin Ligase (BirA, EC 6.3.4.15) and 100 ng to 500 ng linear template coding for the substrate fusion-protein. The substrate fusion-protein was N- or C-terminally fused to a Biotin Accepting Peptide sequence (BAP). In all experiments a 15-mer variant of sequence #85 as identified by Schatz (Schatz, P.J., Biotechnology (NY) 11 (1993) 1138-1143; Beckett, D. et al., Protein Sci. 8 (1999) 921-929) was used (Avitag, Avidity Inc., Denver, Colo. USA). Biotin Ligase was co-expressed from the linear template T7Pg10epsilon_birA_T7T.

### Example 5

### a) Ribosome Display Protocol

All buffers were kept on ice. All devices were sterile, Dnase- and Rnase-free. The workbench was cleaned with Rnase-ZAP.

10x Stock washing buffer (Stock WB) Ribosome Display: 0.5 M TRIS (tris(hydroxymethyl)-aminomethan), pH 7.5 (4°C) adjusted by AcOH (acetic acid); 1.5 M NaCl; 0.5 M magnesium acetate, store at -20°C

10x Elution buffer (Stock EB) Ribosome Display: 0.5 M TRIS, pH 7.5 (4°C) adjusted by AcOH; 1.5 M NaCl; 200 mM EDTA, store at -20°C

10 ml Ribosome Display Washing buffer (WB): 1200 µl 10x Stock WB pH 7.5, 0.05% TWEEN 20 (50µL 10 % TWEEN 20), 5 % BSA (5 ml Blocker BSA 10%), 5 µg/ml t-RNA, 670 mM KCl (0.5 g KCl) ad. 10ml with PCR-grade water

10ml Ribosome Display Stopbuffer (SB): 1200µL 10x Stock WB pH 7.5, 0.05% TWEEN 20 (50µL 10 % TWEEN 20), 5 % BSA (5 ml Blocker BSA 10%), 5 µg/ml t-RNA, 670 mM KCl (0.5 g KCl), 4 mM GSSG (oxidized glutathione), 25 µM cAMP (10µl Stock solution), ad. 10ml with PCR-grade water

2 ml Ribosome Display Elution buffer: 200µL 10x Stock EB, 0.25 % BSA (50 µl Blocker BSA 10 %), 5000 A260 units r-RNA 16S-23S ribosomal, 5 µg/ml t-RNA, ad. 2ml with PCR-grade water

Blocking Reagent: 5 % BSA Puffer (2.5 ml Blocker BSA 10 %), 50 % Conjugate Buffer Universal

10x PBS-buffer: 0.1 M NaH₂PO₄; 0.01 M KH₂PO₄ (lOx pH 7.0; 1x pH 7.4); 1.37 M NaCl; 27 mM KCI.

### b) Preparation of the ectodomains erbB2 and erbB3

The human receptor ectodomains erbB2 and erbB3 were obtained from R&D Systems as receptor chimeras. The receptor ectodomains were genetically fused to the human protein IgG1FC (human IgG1 antibody FC fragment). Both molecules revealed a molecular mass of 96 kDa and contained a hexahistidine-peptide at their C-terminus. As a result of glycosylation the molecular weight of the proteins was increased to 130 to 140 kDa. The chimeric proteins were obtained as lyophilized proteins and were resolubilized in PBS buffer containing 0.1 % BSA. The proteins were stored at -80 °C until use.

### c) Coating of micro titer plates

One Reaction Volume (RV) of a micro titer (MT)-plate was washed three times with Conjugate Buffer Universal. Two and a half (2.5) µg ligand was resolved in 100 µl Blocking Reagent. Biotinylated ligands were alternately immobilized in the wells of Streptavidin- and Avidin-coated MT-plates. The erbB2/FC- and erbB3/FC-chimeras were immobilized alternately in the wells of protein A and protein G coated MT-plates. The ligand-solution was incubated for 1 h at room temperature in the MT-plate under 500 rpm shaking on a Biorobot 8000 robotic shaker platform. To determine the background-signal a well was coated with 100 µl Blocking Reagent without ligand. The wells were washed with 3 RV Blocking Reagent. Blocking Reagent (300 µl) was incubated in each well for 1 h at 4 °C and 200 rpm. Before the stopped translation-mixture was applied, the wells were washed with 3 RV ice-cold buffer WB.

### d) Generation of ribosome display templates

For the standard ribosome display procedure a single gene or a gene-library was elongated with specific bridging primers. The elongated DNA-fragments were fused by OEL-PCR to the DNA-modules T7Pg10epsilon (SEQ ID NO:74) and to the ribosome display spacer (SEQ ID NO:62) using the terminal primers T7Pfor (SEQ ID NO:75) and R1A (SEQ ID NO:76) 5'-AAATCGAAAGGCCCAGTTTTTCG-3'. The PCR profile for the PCR assembly was: TIM: 1 min at 94°C, TM: 20 sec at 94 °C, TA: 30 sec at 60°C, TE: 60 sec for 1000 bp at 72 °C, 30 cycles, TFE: 5 min at 72 °C.

Production of the linear expression element (LEE) T7PAviTagFXa-PEX2-T7T: The human PEX2-gene was amplified in a standard PWO-PCR from 10 ng plasmid template pDSPEX2 (Roche) using the bridging primer according to SEQ ID NO:77 and to SEQ ID NO:78. The overlapping gene was fused by an OEL-PCR to the DNA-modules T7PAviTagFXa (SEQ ID NO:79) and T7T (SEQ ID NO:80) using the primers T7Pfor (SEQ ID NO:82) and T7Trev (SEQ ID NO:81).

### e) Preparation of the ribosome display translation mixture

The RTS *E.coli* 100 HY System was prepared according to the manufacture's instructions. One hundred µl of the mixture were supplemented with 40 units (1 µl) Rnasin, 2 µM (2 µl) anti ssrA-oligonucleotide 5'-TTAAGCTGCTAAAGCGTAGTTTTCGTCGTTTGCGACTA-3' (SEQ ID NO:85), 1 µL stock solution of Complete Mini Protease Inhibitor EDTA-free and 500 ng linear ribosome display DNA-template in 20 µl PWO-PCR mixture. The ribosome display DNA-template was transcribed and translated in 1.5 ml reaction tubes at 30 °C for 40 min under shaking at 550 rpm. Complexes consisting of mRNA, ribosome and displayed polypeptide were stabilized when the reaction was immediately stopped with 500 µl ice-cold buffer SB. The mixture was centrifuged at 15.000 g at 2 °C for 10 min. The supernatant was transferred into a fresh, ice-cooled 1.5 ml reaction tube. Two hundred fifty µl of the mixture were transferred into a ligand-coated MT-plate well (signal) and another 250 µl into a non-ligand coated well (background). The mixture was incubated for 1 h at 4 °C and 300 rpm. To remove background protein and weak binding ternary complexes the wells were washed with ice-cold buffer WB. Messenger RNA from the bound ternary complexes was eluted by 100 µl ice-cold buffer EB for 10 min at 4 °C and 750 rpm.

### f) Preparation of Protein G coated magnetic beads

Protein G coated magnetic beads were used to deplete the stopped ribosome display translation mixtures from protein derivatives, which unspecifically recognized IgG1-FC binders. One hundred µl of the magnetic bead suspension was equilibrated in stopping buffer SB by washing the beads five times in 500 µl buffer SB. The beads were incubated for 1 h at 4 °C in 500 µl buffer SB containing 50 µg IgG1-FC protein. The beads were washed three times with buffer SB and were stored on ice in 100 µl buffer SB. Prior to their use the beads were magnetically separated and stored on ice. The stopped ribosome display translation mixture was added to the beads. The mixture was incubated for 30 min at 4 °C at 750 rpm. Prior to use the beads were magnetically separated form the mixture.

### g) Purification of mRNA and removal of remaining DNA

Messenger RNA was purified using the High Pure RNA Isolation Kit (Roche Applied Science, Mannheim, Germany). Remaining DNA-template in the eluate was removed with a modified protocol of the Ambion DNA-free kit (ambion Inc., USA). Fifty µl eluate were supplemented with 5.7 µl DNAse I buffer and 1.3 µl DNAse I containing solution. After incubation of the mixture at 37 °C for 30 min 6.5 µl DNAse I inactivating reagent was added. The slurry was incubated in the digestion-assay for 3 min at room temperature followed by 1 min centrifugation at 11,000 g. The supernatant was used in the reverse transcription

### h) Reverse Transcription and cDNA amplification

For the reverse transcription of the mRNA the *C. therm.* RT Polymerase Kit (Roche Applied Sciences, Mannheim, Germany) was used. Twenty µl reactions were assembled: 4 µl 5x RT buffer, 1 µl DTT (dithiothreitol) solution, 1.6 µl dNTP's, 1 µl DMSO solution, 0.1 µM (1 µl) RT 5'-CAGAGCCTGCACCAGCTCCAGAGCCAGC-3' (SEQ ID NO:86), 40 units (1 µl) Rnasin, 1.5 µl *C. therm.* RNA-Polymerase, 9 µl mRNA containing eluate. Transcription was performed for 35 min at 70°C. Further amplification of the cDNA was performed in 100 µl PWO-PCRs containing 10 µl 10x PWO-PCR buffer with MgSO₄, 200 µM dNTPs, 12 µl transcription mixture, 2.5 units PWO DNA-Polymerase and the primers RT 5'-CAGAGCCTGCACCAGCTCCAGAGCCAGC-3' (SEQ ID NO:86) and F1 5'-GTTTAACTTTAAGAAGGAGATATACATATG-3' (SEQ ID NO:87) at 1 µM each. The PCR profile was TIM: 1 min at 94 °C, TM: 20 sec at 94 °C, TA: 30 sec at 60 °C, TE: 60 sec at 72 °C, 20 cycles, TFE: 5 min at 72 °C. A reamplification by a standard PWO-PCR was performed. Two µl of the PCR mixture were transferred into a second standard PWO-PCR. Gene-specific bridging primers were used wherever possible. The PCR-profiles were according to the physical parameters of the gene-templates and oligonucleotide-primers. Twenty five PCR cycles were performed. The gene-sequences were elongated with DNA overlaps to hybridize with the DNA-modules T7Pg10epsilon and the ribosome display spacer in a further OEL-PCR. The ribosome display DNA-templates were then reused in further ribosome display cycles.

### i) Sub cloning of genes after ribosome display

The PCR-products were sub cloned into vector-systems with techniques know to a person skilled in the art. Library members of PEX2 were sub cloned via the NdeI/EcoRI sites into the vector pUC18 using the primers NdeI-PEX2for (SEQ ID NO:83) and EcoRI-PEX2rev (SEQ ID NO:84).

## Claims

1. Method for identifying a nucleic acid encoding a polypeptide which specifically binds a predetermined target molecule from a DNA-library, **characterized in that** said method comprises the steps of
a) selecting the sequence of SEQ ID NO:02;
b) preparing a DNA-library of the selected sequence in which at least one amino acid position according to table V is altered;
c) screening the prepared DNA-library for encoded polypeptides specifically binding a predetermined target molecule;
d) choosing the nucleic acid encoding one specific binder identified in step c);
e) repeating the steps b) to d) for two to five times; and
f) isolating said nucleic acid encoding a polypeptide specifically binding a predetermined target molecule.

2. Method as claimed in claim 1, **characterized in that** the DNA-library comprises linear expression elements.

3. Method as claimed in claim 1 or 2, **characterized in that** the members of the library of the polypeptide are expressed by display on ribosomes.

4. Method as claimed in claim 1 or 2, **characterized in that** the members of the library of the polypeptide are expressed by display on bacteriophages.

## Patentansprüche

1. Verfahren zur Identifizierung einer Nukleinsäure, die ein Polypeptid kodiert, das ein vorbestimmtes Zielmolekül aus einer DNA-Bibliothek spezifisch bindet, **dadurch gekennzeichnet, dass** das Verfahren die Schritte
a) Auswählen der Sequenz der SEQ ID NO: 02;
b) Herstellen einer DNA-Bibliothek der ausgewählten Sequenz, in der mindestens eine Aminosäureposition gemäß Tabelle V verändert ist;
c) Screenen der hergestellten DNA-Bibliothek auf kodierte Polypeptide, die ein vorbestimmtes Zielmolekül spezifisch binden;
d) Auswählen der Nukleinsäure, die ein in Schritt c) identifiziertes spezifisches Bindemolekül kodiert;
e) Wiederholen der Schritte b) bis d) für zwei bis fünf Mal; und
f) Isolieren der Nukleinsäure, die ein vorbestimmtes Zielmolekül spezifisch bindet,
umfasst.

2. Verfahren wie in Anspruch 1 beansprucht, **dadurch gekennzeichnet, dass** die DNA-Bibliothek lineare Expressionselemente umfasst.

3. Verfahren wie in Anspruch 1 oder 2 beansprucht, **dadurch gekennzeichnet, dass** die Mitglieder der Bibliothek des Polypeptids durch Display auf Ribosomen exprimiert werden.

4. Verfahren wie in Anspruch 1 oder 2 beansprucht, **dadurch gekennzeichnet, dass** die Mitglieder der Bibliothek des Polypeptids durch Display auf Bakteriophagen exprimiert werden.

## Revendications

1. Procédé d'identification d'un acide nucléique codant pour un polypeptide qui se lie de façon spécifique à une molécule cible prédéterminée dans une banque d'ADN, **caractérisé en ce que** ledit procédé comprend les étapes de:
a) sélection de la séquence de SEQ ID NO: 02;
b) préparation d'une banque d'ADN de la séquence sélectionnée dans laquelle au moins une position d'aminoacide selon le tableau V est modifiée;
c) criblage de la banque d'ADN préparée pour la sélection de polypeptides codés se liant de façon spécifique à une molécule cible prédéterminée;
d) choix de l'acide nucléique codant pour un lieur spécifique identifié dans l'étape c);
e) répétition 2 à 5 fois des étapes b) à d); et
f) isolement dudit acide nucléique codant pour un polypeptide se liant de façon spécifique à une molécule cible prédéterminée.

2. Procédé selon la revendication 1, **caractérisé en ce que** la banque d'ADN comprend des éléments d'expression linéaires.

3. Procédé selon la revendication 1 ou 2, **caractérisé en ce que** les membres de la banque du polypeptide sont exprimés par exposition sur ribosome.

4. Procédé selon la revendication 1 ou 2, **caractérisé en ce que** les membres de la banque du polypeptide sont exprimés par exposition à la surface de bactériophages.
